(19) Europäisches Patentamt
European Patent Office
Office européen des brevets

(11) **EP 4 628 512 A1**

(12) **EUROPEAN PATENT APPLICATION**
published in accordance with Art. 153(4) EPC

(43) Date of publication:
**08.10.2025 Bulletin 2025/41**

(21) Application number: **23896941.4**

(22) Date of filing: **01.12.2023**

(51) International Patent Classification (IPC):
*C07K 19/00* (2006.01)     *C07K 16/18* (2006.01)
*C12N 15/62* (2006.01)     *C12N 15/63* (2006.01)
*A61K 39/395* (2006.01)     *A61P 35/00* (2006.01)

(52) Cooperative Patent Classification (CPC):
A61K 39/395; A61P 35/00; C07K 16/18;
C07K 19/00; C12N 15/62; C12N 15/63

(86) International application number:
**PCT/CN2023/135888**

(87) International publication number:
**WO 2024/114799 (06.06.2024 Gazette 2024/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **01.12.2022  CN 202211537857**

(71) Applicant: **Innovent Biologics (Suzhou) Co., Ltd.
Suzhou, Jiangsu 215123 (CN)**

(72) Inventors:
• **LI, Yiming
Suzhou, Jiangsu 215123 (CN)**
• **WANG, Feifei
Suzhou, Jiangsu 215123 (CN)**
• **ZHOU, Shuaixiang
Suzhou, Jiangsu 215123 (CN)**
• **YU, Yanting
Suzhou, Jiangsu 215123 (CN)**

(74) Representative: **Sagittarius IP
Marlow International
Parkway
Marlow SL7 1YL (GB)**

Remarks:
The complete document including Reference
Table(s) and the Sequence Listing(s) can be
downloaded from the EPO website

(54) **TRISPECIFIC FUSION PROTEIN AND USE THEREOF**

(57)     The present invention relates to a multi-specific fusion protein, and more particularly, to a trispecific fusion protein specifically binding to VEGFA, VEGFC and Ang2. The present invention further relates to a polynucleotide encoding the protein, an expression vector and a host cell, a pharmaceutical composition of same, and a method and a use for treating neovascularization-related diseases.

## Description

## Technical Field

The present invention relates to the field of multi-specific fusion proteins, and more particularly, to a trispecific fusion protein specifically binding to VEGFA, VEGFC and Ang2. The present invention further relates to a polynucleotide encoding the protein, an expression vector and a host cell, a pharmaceutical composition of same, and a method and a use for treating neovascularization-related diseases.

## Background

Vascular endothelial growth factor (VEGF) is a major regulator of vascular development and blood and lymphatic function in adults during health and disease. The VEGF family is currently known to consist of five structurally related factors: VEGFA, VEGFB, VEGFC, VEGFD and placental growth factor (PIGF). VEGF family members mainly exist in the form of homodimeric polypeptides, inducing signal transduction and triggering corresponding biological effects by binding to related VEGF receptors.

Vascular endothelial growth factor A (VEGFA) has multiple isoforms, including VEGF121, VEGF145, VEGF165, VEGF183, VEGF189 and VEGF206. These isoforms are generated by alternative splicing, of which VEGF165 is the main isoform. VEGFA participates in the angiogenesis process by interacting with tyrosine kinase receptors VEGFR-1 and VEGFR-2. Blocking the VEGFA pathway has been proposed to improve neovascularization-related diseases. Such VEGFA blockers/antagonists include: neutralizing antibodies targeting VEGFA, and soluble decoy receptors and Trap molecules that prevent VEGFA from binding to its normal receptors. Aflibercept (also known as VEGFA-Trap, trade name Eylea) is a recombinant fusion protein formed by the ligand-binding extracellular domains of human VEGF receptors 1 and 2 fused to the Fc region of human IgG1. It has been approved for the treatment of neovascularization-related retinal diseases such as age-related macular degeneration, and is being used in clinical trials for the treatment of solid tumors.

Vascular endothelial growth factor C (VEGFC) shares approximately 30% sequence homology with VEGF165. VEGFC exerts its effects by binding to the tyrosine kinase receptors VEGFR2 and VEGFR3, and is involved in the neovascularization process and lymphangiogenesis. VEGFR2 is expressed on vascular endothelial cells and it also binds to VEGFA. VEGFR3 is expressed on vascular endothelial cells and lymphatic cells, but does not bind to VEGFA. In a variety of metastatic tumor models, VEGFC/VEGFR3 signaling pathway blockers have been used to inhibit tumor lymphangiogenesis and metastasis. In addition, VEGFC Trap molecules, such as OPT-032 (a VEGFC/D inhibitor constructed from the ligand-binding extracellular domain from VEGFR3) developed by Opthea Ltd., have been proposed for the treatment of neovasculogenesis-related retinopathy.

In addition to the VEGF family, human angiopoietin (ANG) is also believed to be involved in vascular development and postnatal angiogenesis. Angiopoietin 2 (also known as Angiopoietin-2, ANGPT2, or Ang2) is a member of the angiopoietin family. As a secreted glycoprotein, the protein consists of three parts: a secretory signal peptide, an N-terminal coiled-coil domain (CCD), and a C-terminal fibrinogen-like domain (FLD). The FLD domain of the angiopoietin protein is highly conservative and is responsible for binding to the Tie2 receptor. Preclinical studies using acute ocular inflammation models and choroidal neovascularization models (CNV models) have shown that ang-2 and VEGF are synergistically involved in the pathological neovascularization and vascular permeability. Therefore, in addition to anti-VEGF therapy, the Tie2/angiopoietin pathway has also been proposed as a therapeutic target for vascular-related retinopathy. For example, the combination of the anti-Ang2 antibody Nesvacumab (Regeneron, Tarrytown, NY, USA) and aflibercept (anti-VEGFA) is used to treat neovascular age-related macular degeneration (nAMD) and diabetic macular edema (DME). In addition, the anti-VEGFA/ang2 bispecific antibody Faricimab (co-developed by Roche, Basel, Switzerland and Genentech, South San Francisco, CA, USA) has also been proposed for the treatment of nAMD and DME.

It is known that a variety of disease processes involve abnormal vascular permeability and angiogenesis, including, for example, cancers, autoimmunity, and retinopathy. The development of existing drugs that block angiogenesis signaling pathways has brought therapeutic benefits to some patients with diseases. However, the treatment of such diseases still faces challenges in many aspects such as drug accessibility, efficacy, patient compliance, and treatment burden. Therefore, the field is still in urgent need of new drug molecules that can effectively target and improve abnormal angiogenesis processes to meet treatment needs.

## Summary of the Invention

To meet the above needs, the inventors have designed and developed a trispecific fusion protein molecule that can specifically target and bind to VEGFA, VEGFC and Ang2. The trispecific molecule of the present invention exhibits relatively strong binding and blocking effects on each target antigen; and exhibits good anti-neovascularization effects in animal models. On this basis, the inventors established the present invention.

Therefore, in a first aspect, the present invention provides a trispecific fusion protein, comprising:

a polypeptide chain comprising (i) a VEGFC-binding domain; (ii) an Ang2-binding domain and (iii) a VEGFA-binding domain,

wherein the VEGFC-binding domain and the Ang2-binding domain comprise or consist of a VHH domain that specifically binds to VEGFC or Ang2, respectively, and

wherein the VEGFA-binding domain comprises or consists of a ligand capture domain that specifically binds to a VEGFA, wherein the ligand capture domain comprises an extracellular domain from VEGFR,

wherein, preferably, the polypeptide further comprises (iv) a dimerization domain, in particular a dimerization domain selected from a hinge region, a CH3 region and an Fc region.

In some embodiments, the polypeptide comprises a hinge region. In other embodiments, the polypeptide comprises a CH3 region, and preferably the polypeptide comprises a CH3 region having a hinge region at the N-terminus. In further embodiments, the polypeptide comprises an Fc region, and preferably the polypeptide comprises an Fc region having a hinge region at the N-terminus. In some embodiments, the hinge region comprises a CPPC or CPPCPPC amino acid sequence, and preferably the hinge region comprises an amino acid sequence selected from SEQ ID NOs: 12-19 and 45-46, more preferably an amino acid sequence of SEQ ID NO: 12 or 13. In some embodiments, the Fc region is the Fc region of human IgG1, IgG2, IgG3, or IgG4. In some embodiments, the CH3 region is the CH3 region of human IgG1, IgG2, IgG3, or IgG4.

In yet another aspect, the present invention provides a polynucleotide, a vector, and a host cell encoding the trispecific fusion protein of the present invention; and a pharmaceutical composition, a pharmaceutical combination and a kit comprising the molecule of the present invention.

In yet another aspect, the present invention provides a use of the trispecific fusion protein of the present invention for treatment or use as a drug, or the trispecific fusion protein of the present invention for preventing and/or treating diseases or for preparing a drug for preventing and/or treating diseases, such as neovasculogenesis-related diseases, such as tumors and eye diseases.

In yet another aspect, the present invention also provides a diagnostic use of the trispecific fusion protein of the present invention.

In yet another aspect, the present invention also provides a multi-specific fusion protein having a unique configuration, wherein the fusion protein comprises:

a polypeptide chain comprising a first VHH domain that specifically binds to a first antigen, a second VHH domain that specifically binds to a second antigen, and a ligand capture domain that specifically binds to a third antigen, respectively, wherein the polypeptide further comprises a dimerization domain selected from a hinge region, a CH3 region, or an Fc region. In some preferred embodiments, the dimerization domain is a hinge region, and preferably, wherein the hinge region is connected to the C-terminus of the ligand capture domain.

In yet another aspect, the present invention also provides a hinge region comprising or consisting of an amino acid sequence selected from SEQ ID NOs: 12-19 and 45-46, and preferably comprising or consisting of an amino acid sequence of SEQ ID NO: 12 or 13.

## Brief Description of the Drawings

Figure 1A shows an SDS-PAGE purity test of anti-VEGFA/VEGFC/Ang2 multi-specific fusion protein after one-step purification with protein A. NR represents protein samples treated in non-reducing loading buffer; R represents protein samples treated in reducing loading buffer.

Figure 1B shows an SEC-HPLC purity test of anti-VEGFA/VEGFC/Ang2 multi-specific fusion protein after one-step purification with protein A.

Figure 2 shows an experiment in which anti-VEGFA/VEGFC/Ang2 multi-specific fusion protein blocks VEGFA-induced HEK 293 KDR reporter activation.

Figure 3 shows an experiment in which anti-VEGFA/VEGFC/Ang2 multi-specific fusion protein blocks VEGFC-induced HEK 293 KDR reporter activation.

Figure 4 shows an experiment in which anti-VEGFA/VEGFC/Ang2 multi-specific fusion protein blocks VEGFC-induced Baf3-FLT4 proliferation.

Figure 5 shows an ELISA blocking experiment in which anti-VEGFA/VEGFC/Ang2 multi-specific fusion protein blocks the binding of Ang2 to Tie2.

Figure 6 shows a blocking experiment in which anti-VEGFA/VEGFC/Ang2 multi-specific fusion protein blocks the binding of Ang2 to Tie2-expressing cells.

Figure 7 shows an experiment in which anti-VEGFA/VEGFC/Ang2 multi-specific fusion protein inhibits Ang2-induced Tie2 phosphorylation.

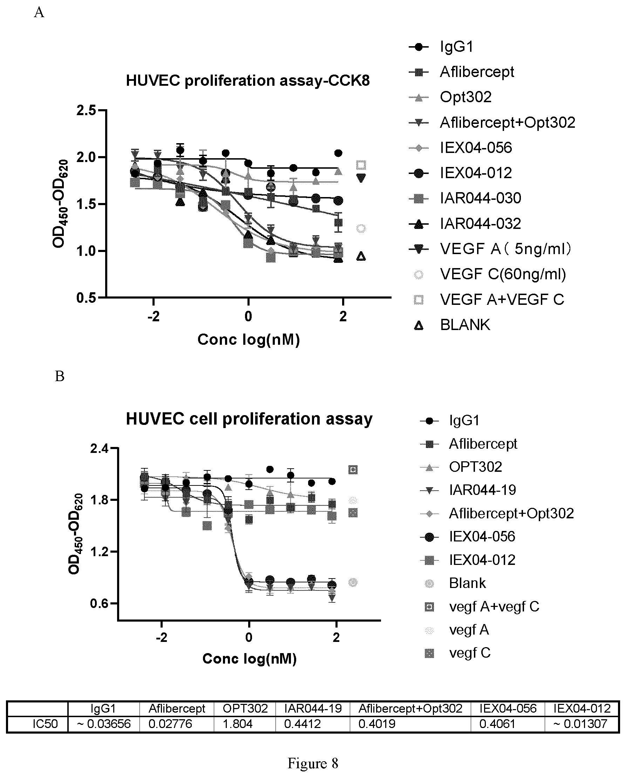

Figure 8 shows an experiment in which anti-VEGFA/VEGFC/Ang2 multi-specific fusion protein inhibits VEGFA+C-induced HUVEC proliferation.

Figure 9 shows the amount of change in A375 tumor volume over time under different modes of administration in a neovascularization experiment in an A375 subcutaneous tumor model.

Figure 10 shows fundus angiography images showing that anti-VEGFA/VEGFC/Ang2 multi-specific fusion protein inhibits laser-induced choroidal neovascularization.

Figure 11 shows the improvement rate (%) of leakage area measured by FFA on day 5 of administration in a laser-induced CNV model.

Figure 12 shows that anti-VEGFA/VEGFC/Ang2 multi-specific fusion protein inhibits DL-AAA-induced retinal neovascularization (RNV).

Figure 13 shows that anti-VEGFA/VEGFC/Ang2 multi-specific fusion protein inhibits vascular leakage.

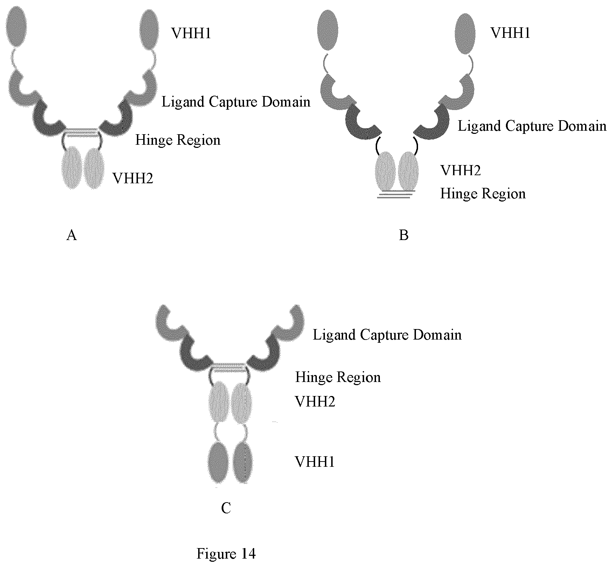

Figure 14 shows exemplary configurations of the anti-VEGFA/VEGFC/Ang2 multi-specific fusion protein according to the present invention, wherein VHH1 and VHH2 independently represent a VEGFC-binding domain or an Ang2-binding domain, respectively, and the ligand capture domain represents a VEGFA ligand capture domain from a VEGF receptor.

## Detailed Description of the Invention

Unless defined otherwise, all technical and scientific terms used herein have the same meaning as those typically understood by those of ordinary skill in the art to which the present invention belongs. All publications, patent applications, patents, and other references mentioned herein are incorporated by reference in their entirety. In addition, the materials, methods, and examples described herein are illustrative only and are not intended to be limiting. Other features, objectives and advantages of the present invention will be apparent from this Description and the accompanying drawings and from the appended Claims.

Definition

The term "about" when used in conjunction with a numerical value is meant to encompass numerical values within the range of the lower limit that is 5% less than the specified numerical value and the upper limit that is 5% greater than the specified numerical value.

As used herein, the term "comprising" or "including" is meant to include the stated elements, integers, or steps, but does not exclude any other elements, integers, or steps. For example, when referring to an antigen-binding domain "comprising" a particular sequence, it is also intended to cover an antigen-binding domain consisting of that particular sequence.

As used herein, the term "fusion protein" refers to a protein comprising at least one polypeptide chain formed by the fusion of at least two heterologous polypeptide sequences, optionally via a linker peptide. The fusion protein may be produced by recombinant expression and may be in the form of a monomer or polymer (e.g., dimer) comprising one or more of the polypeptide chains. In one embodiment, the fusion protein of the present invention is a dimeric protein formed by two identical polypeptide chains.

The term "heterologous" when referring to a polypeptide chain constituting a fusion protein means that the polypeptide chain comprises two or more subsequences that do not occur in the same protein or polypeptide in nature.

The term "recombinant" herein refers to the production of a polypeptide or protein by a biological host. The host may be selected from, including but not limited to, a mammalian expression system, an insect cell expression system, a yeast expression system, and a bacterial expression system. In one embodiment, the polypeptide/protein of the present invention is a recombinant polypeptide/protein expressed in a prokaryotic organism (e.g., an Escherichia coli cell) or a eukaryotic host cell (e.g., a mammalian host cell).

As used herein, the term "monospecific" refers to a polypeptide/protein molecule that has one or more antigen-binding sites, each of which binds to the same epitope of the same antigen.

As used herein, the term "multi-specific" refers to a polypeptide/protein molecule having at least two antigen-binding sites that bind to different epitopes (different epitopes on the same antigen or different epitopes on different antigens). In some embodiments, the present invention provides a trispecific fusion protein comprising at least one polypeptide chain comprising antigen-binding sites having binding specificity for three different antigens VEGFA, VEGFC and Ang2.

As used herein, the terms "antigen-binding site" and "antigen-binding domain" may be used interchangeably and refer to the region of a molecule that actually binds to the target antigen. Examples of antigen-binding sites include, for example, but are not limited to, variable domains of antibodies, extracellular ligand-binding domains of receptors, and the like. Preferably, the VEGFC antigen-binding site used in the trispecific fusion protein of the present invention is provided by the variable domain of an anti-VEGFC heavy chain antibody (i.e., "VHH"); the Ang2 antigen-binding site used in the trispecific fusion protein of the present invention is provided by the variable domain of an anti-Ang2 heavy chain antibody (i.e., "VHH"); the VEGFA antigen-binding site used in the trispecific fusion protein of the present invention is provided by a VEGFA ligand capture domain (i.e., a VEGF receptor extracellular domain polypeptide fragment that specifically binds to VEGFA) or by an artificial ligand "mini-Trap" domain.

The term "VHH domain" is used herein to refer to a heavy chain variable domain derived from a heavy chain antibody lacking a light chain, also referred to as a single variable domain fragment or a nanobody fragment. The VHH domain differs from the conventional VH domain of a four-chain immunoglobulin in that it does not require pairing with a light chain variable domain to form an antigen-binding site. Such a VHH domain may be derived from a heavy chain antibody produced in camelidae species (e.g., camel, alpaca, dromedary, llama, and guanaco). Other species besides camelidae may also produce heavy chain antibodies that naturally lack light chains, and the VHH domains contained in such heavy chain antibodies are also within the scope of consideration of the present invention. The variable domain of the heavy chain antibody (i.e., VHH domain) is the same as the heavy chain variable region (VH) in a conventional four-chain IgG antibody, which is the domain responsible for the binding of the antibody to the antigen, and is also composed of three complementary determining regions (CDRs) and four conservative framework regions (FRs) in structure, arranged in the following order from the amino terminus to the carboxyl terminus: FR1, CDR1, FR2, CDR2, FR3, CDR3, FR4. In some cases, for the therapeutic application of the VHH domain, it is desirable to reduce its immunogenicity. Therefore, preferably, in one embodiment, the VHH domain used in the present invention is a humanized VHH domain or a further sequence-optimized form thereof (e.g., an affinity-matured form to increase binding affinity).

As used herein, the term ligand "Trap" molecule refers to a fusion protein comprising the extracellular domain of a receptor protein that interacts with the ligand and an immunoglobulin Fc region fused thereto. Currently, a variety of trap molecules that "capture" VEGF ligands, including VEGFA and VEGFC, have been developed from VEGF receptors. These Trap molecules may be used to bind to the corresponding ligands in the extracellular environment and reduce their concentration.

Herein, the terms "ligand capture domain" and ligand "mini-Trap" domain are used interchangeably, referring to a polypeptide fragment that does not contain an immunoglobulin Fc region, which is different from a Trap molecule and consists of or is mainly composed of a ligand-interacting extracellular domain from (at least one) receptor protein. It should be understood that the receptor extracellular domain that interacts with the ligand contained in the ligand capture domain and the mini-Trap domain may be in the form of a natural sequence or a modified sequence, such as a natural extracellular domain sequence from a receptor, or a hybrid extracellular domain sequence composed of extracellular domains from different receptors, or a variant sequence in which amino acid mutations are introduced into the natural extracellular domain or the hybrid extracellular binding domain. In one embodiment, the VEGFA-binding domain of the present invention comprises or consists of a mini-trap domain that "captures" VEGFA. The mini-trap domain can compete with the natural VEGFA cell receptor for binding to VEGFA, thereby inhibiting VEGFA-induced signal transduction. In one embodiment, the mini-trap domain that binds to VEGFA has a length of no more than 350 amino acids, preferably no more than 250 amino acids. In one embodiment, the mini-trap domain that binds to VEGFA is formed by fusing the extracellular ligand binding domains from VEGF receptors 1 and 2, and preferably has amino acids from humans to minimize the immunogenicity of the molecule in humans.

Herein, the term "linker peptide", "flexible linker peptide" or "linker" refers to a short amino acid sequence consisting of amino acids with a length of no more than 50 amino acid residues, such as glycine (G) and/or serine (S) and/or threonine residues (T) used alone or in combination, and/or a hinge region sequence from the natural hinge region of an immunoglobulin or its truncated and/or modified form. In one embodiment, the linker peptide has a length of 5-50 amino acids, for example, a length of 10, 15, 20, 25, or 30 amino acids. In one embodiment, the linker peptide comprises the amino acid sequence $(G_4S)n$ (SEQ ID NO: 55), wherein n is an integer equal to or greater than 1, for example, n is an integer of 2, 3, 4, 5, 6, or 7. In one embodiment, the linker peptide comprises the amino acid sequence TS(G$_4$S)$_n$ (SEQ ID NO: 56), wherein n is an integer equal to or greater than 1, for example, n is an integer of 2, 3, 4, 5, 6, or 7. In one embodiment, the linker peptide comprises the amino acid sequence G(G$_4$S)n (SEQ ID NO: 57), wherein n is an integer equal to or greater than 1, for example, n is an integer of 2, 3, 4, 5, 6, or 7. In one embodiment, the linker peptide comprises the amino acid sequence (GGGGG)n (SEQ ID NO: 58) or (GRPGS)n (SEQ ID NO: 59), wherein n is an integer of 2, 3, 4, 5, 6, or 7, for example, n is an integer of 2 or 3. In yet another embodiment, the linker peptide comprises a hinge region, for example, a hinge region having the amino acid sequence CPPC (SEQ ID NO: 60) or CPPCPPC (SEQ ID NO: 61). In some further embodiments, the linker peptide further comprises a short linker with a length of 5-15, e.g., 10 amino acids that is located at the C-terminus of the hinge region, in particular a short linker composed of glycine (G) and/or serine (S) and/or threonine residues (T), thereby conferring the ability of the polypeptide comprising the linker peptide to pair and dimerize in the hinge region, and conferring the segment flexibility required for the normal function of the antigen-binding domains at both ends of the linker peptide.

Herein, the term "hinge region" refers to a natural hinge region from an immunoglobulin IgG, or a truncated form thereof that retains at least a core hinge segment, or a variant sequence thereof, provided that the hinge region comprises at least 2, for example 2-4 or preferably 3 cysteine residues capable of forming an interchain disulfide bond. These cysteine residues in the hinge region may be residues originally present in the natural hinge region, or residues introduced by amino acid substitution and/or addition. The hinge region of the natural immunoglobulin heavy chain is well known in the art and is composed of a segment between the Fab and Fc portions. According to crystallographic data, the natural IgG hinge region may be divided into an upper hinge region, an intermediate (core) hinge region, and a lower hinge region. The upper hinge region is a segment from the end of the Fab to the first inter-heavy chain disulfide bridge; the core hinge region is a polyproline core segment from the first inter-heavy chain disulfide bridge to the last inter-heavy chain disulfide bridge; the lower hinge region is a segment from the last inter-heavy chain disulfide bridge to the Fc starting residue. According to the EU numbering system, the hinge region of human heavy chain IgG1 extends from heavy chain Glu216 to Pro230 or to Gly236, wherein the polyproline segment from Cys226 to Cys229 is the core segment, which divides the entire hinge region into an upper hinge region and a lower hinge region. For a review of immunoglobulin hinge regions, see General Characteristics of Immunoglobulin Molecules, ROALD NEZLIN, in The Immunoglobulins, 1998, https://doi.org/10.1016/B978-012517970-6/50001-1, which is incorporated herein by reference. The hinge region sequences of human immunoglobulin IgG isotypes IgG1, IgG2, IgG3, and IgG4 are disclosed at https://www.imgt.org/IMGTScientificChart/Numbering/ Hu IGHGnber.html. Those skilled in the art can easily determine the hinge region sequence corresponding to IgG1 residues 216-230 or residues 216-236 in other immunoglobulins by comparing with the IgG1 sequence. Herein, the term "hinge region" does not include an immunoglobulin Fc region sequence or a variant sequence thereof. In some embodiments according to the present invention, the hinge region has a length of 4 to 25 amino acids, such as 8-22, especially 10-20 amino acids, such as 11, 12, 13, 14, 15, 16, 17, or 18 amino acids. In some preferred embodiments, the hinge region for the fusion protein polypeptide chain of the present invention comprises a CPPC or preferably CPPCPPC amino acid sequence, whereby two polypeptide chains may be paired and form a stable dimer through the hinge region. In some more preferred embodiments, the hinge region comprises an amino acid sequence selected from SEQ ID NOs: 12-19 and 45-46.

Herein, the terms "Fc region" and "Fc domain" may be used interchangeably herein to define the C-terminal region of an immunoglobulin heavy chain containing at least a portion of a constant region. The term includes natural sequence Fc regions and variant Fc regions. A natural immunoglobulin "Fc region" comprises two or three constant domains, i.e., a CH2 domain, a CH3 domain, and an optional CH4 domain. The Fc region that may be used in the fusion protein of the present invention includes, but is not limited to, an Fc region of IgG1, IgG2, IgG3, or IgG4 that has a natural sequence or a variant sequence. Unless otherwise specified herein, the numbering of the amino acid residues in the hinge region and the Fc region and each constant region of the heavy chain is numbered according to the EU numbering system (also referred to as the EU index) as described in Kabat et al., Sequences of Proteins of Immunological Interes, 5th edition, Public Health Service, National Institutes of Health, Bethesda, MD, 1991. Herein, the term "Fc region" or "Fc domain" does not comprise the heavy chain variable region VH and light chain variable region VL, as well as the heavy chain constant region CH1 and light chain constant region CL of an immunoglobulin, but may or may not comprise all or part of the hinge region. In the case where the Fc region does not have a natural N-terminal hinge region sequence; in some embodiments of the present invention, the Fc region may be artificially connected to a hinge region as defined herein at its N-terminus, for example, a hinge region comprising an amino acid sequence selected from SEQ ID NOs: 12-19 and 45-46.

Herein, the term "natural sequence Fc region" covers various naturally occurring immunoglobulin Fc region sequences, such as the Fc region sequences of various Ig subtypes and their allotypes (Gestur Vidarsson et al., IgG subclasses and allotypes: from structure to effector functions, 20 October 2014, doi: 10.3389/fimmu.2014.00520). In some embodiments, the human IgG heavy chain Fc region comprises an amino acid sequence extending from Cys226 or from Pro230 to the carboxy-terminus of the heavy chain. However, the C-terminal lysine (Lys447) in the Fc region may or may not exist. In yet some embodiments, the human IgG heavy chain Fc region carries a hinge sequence or partial hinge sequence of a natural immunoglobulin at the N-terminus, such as a sequence from E216 to T225 or a sequence from D221 to T225, according to EU numbering. Herein, the term "Fc region of a variant sequence" refers to an Fc region polypeptide comprising a modification relative to a natural sequence Fc region polypeptide. The modification may be an addition, deletion, or substitution of an amino acid residue. Substitutions may include naturally occurring amino acids and non-naturally occurring amino acids. The purpose of the modification may be to alter the binding of the Fc region to its receptor and the effector function thereby induced.

Herein, the terms "CH3 region" and "CH3 domain" may be used interchangeably herein to define the CH3 constant region of an immunoglobulin heavy chain. The term includes natural sequence CH3 regions and variant CH3 regions. The Fc region that may be used in the fusion protein of the present invention includes, but is not limited to, a CH3 region of IgG1, IgG2, IgG3, or IgG4 that has a natural sequence or a variant sequence.

Herein, the "natural sequence CH3 region" covers various naturally occurring immunoglobulin Fc region sequences, e.g., the CH3 region sequence of various Ig subtypes and their allotypes (Gestur Vidarsson et al., IgG subclasses and allotypes: from structure to effector functions, 20 October 2014, doi: 10.3389/fimmu.2014.00520). In some embodiments, the human IgG heavy chain CH3 region comprises an amino acid sequence extending from Gly341 to the carboxy-terminus of the heavy chain, wherein the C-terminal lysine (Lys447) of the CH3 region may or may not exist. Herein, the term "CH3 region of a variant sequence" refers to a CH3 region polypeptide comprising a modification relative to a natural sequence CH3 region polypeptide. The modification may be an addition, deletion, or substitution of an amino acid residue. Substitutions may include naturally occurring amino acids and non-naturally occurring amino acids. The purpose of the modification may be to alter the binding of the CH3 region to its receptor and the effector function thereby induced.

Herein, the term "complementarity-determining region" or "CDR region" or "CDR" or "hypervariable region" refers to a region in an antibody variable domain (VH/VL or VHH) that is highly variable in sequence and forms a structurally determined loop ("hypervariable loop") and/or contains antigen contacting residues ("antigen contact points"). CDRs are primarily responsible for binding to antigen epitopes. CDRs are numbered sequentially from the N-terminus and are typically referred to as CDR1, CDR2, and CDR3. CDRs located in the heavy chain antibody variable region (VHH domain) are sometimes also referred to as HCDR1, HCDR2, and HCDR3. In a given amino acid sequence of a heavy chain antibody variable region, its CDR sequence may be determined using a variety of schemes known in the art, such as: CDR defined by Chothia based on antibody three-dimensional structure and CDR loop topology, Kabat (Kabat et al., Sequences of Proteins of Immunological Interest, 4th Edition, U.S. Department of Health and Human Services, National Institutes of Health (1987)), AbM (University of Bath), Contact (University College London), international ImMunoGeneTics database (IMGT) (International Immunogenetics Information System, World Wide Web imgt.cines.fr/) based on antibody sequence variability, and North (North et al., "A New Clustering of Antibody CDR Loop Conformations", Journal of Molecular Biology, 406, 228-256 (2011)) based on affinity propagation clustering using a large number of crystal structures.

The following are the regional ranges of CDRs defined using the Kabat, AbM, Chothia, Contact, and IMGT schemes.

| CDR | Kabat Scheme | AbM Scheme | Chothia Scheme | Contact Scheme | IMGT Scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L51 |
| LCDR3 (Kabat and Chothia numbering systems) | L89-L97 | L89-L97 | L91-L96 | L89-L96 | L89-L97 |
| HCDR1 (Kabat numbering system) | H31-H35B | H26-H35B | H26-H32 | H30-H35B | H26-H35B |
| HCDR1 (Chothia numbering system) | H31-H35 | H26-H35 | H26-H32 | H30-H35 | H26-H35 |
| HCDR2 (Kabat and Chothia numbering systems) | H50-H65 | H50-H58 | H53-H55 | H47-H58 | H51-H56 |

(continued)

| CDR | Kabat Scheme | AbM Scheme | Chothia Scheme | Contact Scheme | IMGT Scheme |
|---|---|---|---|---|---|
| HCDR3 (Kabat and Chothia numbering systems) | H95-H102 | H95-H102 | H96-H101 | H93-H101 | H93-H102 |

Unless otherwise specified, the term "CDR" or "CDR sequence" in the present invention covers CDR sequences identified by any of the methods described above.

In addition, CDRs may also be identified based on reference CDR sequences with the same Kabat number position. In the present invention, unless otherwise specified, when the position of residues in an antibody variable region (including heavy chain variable region residues and light chain variable region residues) is mentioned, it refers to the number position according to the Kabat numbering system (Kabat et al., Sequences of Proteins of Immunological Interest, 5th Ed. Public Health Service, National Institutes of Health, Bethesda, Md. (1991)).

Herein, an "isolated" protein or polypeptide (e.g., the fusion protein of the present invention) is a protein or polypeptide that has been isolated from components of its natural environment. In some embodiments, the protein or polypeptide is purified to more than 95% or 99% purity, as determined by, for example, electrophoresis (e.g., SDS-PAGE, isoelectric focusing (IEF), capillary electrophoresis), or chromatography (e.g., ion exchange or reversed-phase HPLC).

As used herein, the term "VEGFA" or "VEGFA" refers to vascular endothelial growth factor A (e.g., the human VEGFA protein under Accession No. UniProt P15692). VEGFA binds to the receptors VEGFR1 (also known as FLT1) and VEGFR2 (also known as KDR). Herein, "antigen-binding specificity for VEGFA" refers to a binding site or binding domain in a molecule that specifically binds to human VEGFA.

Herein, the term "VEGFC" or "VEGFC" refers to vascular endothelial growth factor C (e.g., the human VEGFC protein under Accession No. UniProt P49767). VEGFC binds to the receptors VEGFR2 (also known as KDR) and VEGFR3 (also known as FLT4). Herein, "antigen-binding specificity for VEGFC" refers to a binding site or binding domain in a molecule that specifically binds to human VEGFC.

Herein, the term "Ang2" (also known as ANGPT2 or ANGP2) refers to angiopoietin 2 (e.g., the human Ang2 protein under Accession No. UniProt 015123). Ang2 binds to the receptor Tie2 (also known as TEK) and induces tyrosine phosphorylation of TEK/TIE2. Ang2 can synergize with VEGF to promote endothelial cell migration and proliferation. Herein, "antigen-binding specificity for Ang2" refers to a binding site or binding domain in a molecule that specifically binds to human Ang2.

As used herein, the term "bind" or "specifically bind" means that binding is selective for an antigen and may be distinguished from unwanted or non-specific interactions. The ability of an antigen-binding site to bind to a particular antigen may be determined by an enzyme-linked immunosorbent assay (ELISA) or conventional binding assays known in the art.

"Affinity" or "binding affinity" refers to the intrinsic binding affinity that reflects interactions between members of a binding pair. The affinity of a molecule X for its partner Y may typically be represented by a dissociation constant (KD), and the dissociation constant is a ratio of the dissociation rate constant to the association rate constant (kdis and kon, respectively). Affinity may be measured by common methods known in the art. One specific method for measuring affinity is the ForteBio kinetic binding assay herein.

"Identity percentage (%)" of an amino acid sequence refers to the percentage of amino acid residues in a candidate sequence that are identical to the amino acid residues of the specific amino acid sequence shown in this Description, after aligning the candidate sequence with the specific amino acid sequence shown in the this Description, and introducing gaps if necessary to achieve the maximize the percentage of sequence identity, and not considering any conservative substitutions as part of the sequence identity. In some embodiments, the present invention contemplates variants of the antibody molecule of the present invention, wherein the variant has a considerable degree of identity, e.g., identity of at least 80%, 85%, 90%, 95%, 97%, 98%, or 99% or higher, relative to the antibody molecules specifically disclosed herein and the sequences thereof. The variant may comprise conservative modifications.

For a polypeptide sequence, a "conservative modification" includes a substitution, deletion, or addition of a polypeptide sequence, but does not substantially change the desired functional activity of the polypeptide sequence. For example, conservative substitutions often result in the substitution of an amino acid for a chemically similar amino acid. Conservative substitution tables that provide functionally similar amino acids are well known in the art. Eight sets of amino acids containing conservative substitutions are listed below: 1) alanine (A), glycine (G); 2) aspartate (D), glutamic acid (E); 3) asparagine (N), glutamine (Q); 4) arginine (R), lysine (K); 5) isoleucine (I), leucine (L), methionine (M), valine (V); 6) phenylalanine (F), tyrosine (Y), tryptophan (W); 7) serine (S), threonine (T); and 8) cysteine (C), methionine (M). In some embodiments, the term "conservative sequence modification" is used to refer to an amino acid modification that does not significantly affect or change the target antigen-binding features of the antibody molecule or binding protein molecule of the present invention containing the amino acid sequence. For example, a conservatively modified variant maintains a binding affinity for a target antigen of at least 80%, 85%, 90%, 95%, 98%, 99% or higher, e.g., 100-110% or higher, relative to a parental antibody or a binding protein.

The term "host cell" refers to a cell into which an exogenous polynucleotide has been introduced, including progeny of such cells. Host cells include "transformants" and "transformed cells", which include primary transformed cells and progeny derived therefrom. Host cells are any type of cell system that may be used to produce the trispecific fusion protein of the present invention, including eukaryotic cells, e.g., mammalian cells, insect cells, yeast cells; and prokaryotic cells, e.g., Escherichia coli cells. Host cells include cultured cells, and also include cells within transgenic animals, transgenic plants, or cultured plant tissues or animal tissues.

The term "expression vector" refers to a vector comprising a recombinant polynucleotide, comprising an expression control sequence that effectively links the nucleotide sequence to be expressed. The expression vector comprises sufficient cis-acting elements for expression; other elements for expression may be provided by the host cell or in an in vitro expression system. Expression vectors include all those known in the art, including cosmids, plasmids (e.g., naked plasmids or plasmids packaged in liposomes) and viruses (e.g., lentiviruses, retroviruses, adenoviruses, and adeno-associated viruses) incorporated into the recombinant polynucleotide.

The terms "individual" and "subject" may be used interchangeably to refer to a mammal. Mammals include, but are not limited to, domesticated animals (e.g., dairy cattle, sheep, cats, dogs, and horses), primates (e.g., humans and non-human primates such as monkeys), rabbits, and rodents (e.g., mice and rats). In particular, individuals refer to humans.

The term "treatment" refers to a clinical intervention intended to alter the natural course of a disease in an individual being treated. A desired therapeutic effect includes, but is not limited to, preventing the onset or recurrence of a disease, alleviating symptoms, reducing any direct or indirect pathological consequences of a disease, preventing metastasis, reducing the rate of disease progression, improving or alleviating the disease state, and relieving or improving prognosis. In some embodiments, the antibody molecule of the present invention is used to delay disease development or to slow disease progression.

The term "anti-tumor effect" or "tumor inhibitory effect" refers to a biological effect that may be demonstrated by various means, including, but not limited to, e.g., reduction in tumor volume, reduction in number of tumor cells, reduction in tumor cell proliferation, or reduction in tumor cell survival. The terms "tumor" and "cancer" are used interchangeably herein to cover a variety of solid tumors.

Herein, the term "neovasculogenesis-related disease" or the term "neovascularization-related disease" refers to a disease, disorder, and/or condition in which the onset, development, and/or progression of a disease involves neovasculogenesis (including neovascularization, lymphangiogenesis, and/or both). Such a disease, disorder or condition would benefit from the blocking of the biological activity of VEGFC, VEGFA, Ang2, or any of two or three of these.

## The Fusion Protein of the Present Invention

Various IgG and IgG-like antibody molecules targeting VEGF and angiopoietin signaling pathways have been previously proposed, e.g., anti-VEGFA, anti-VEGFC, and anti-Ang2 antibodies, and bispecific antibodies against VEGF and Ang2. However, there have been no relevant reports on trispecific molecules that simultaneously block VEGFA, VEGFC and Ang2.

The inventors constructed a trispecific fusion protein molecule with simultaneously high binding affinity and specificity for the three target antigens, and confirmed that the targeted blockade of the three main signaling pathways involved in abnormal angiogenesis, namely VEGFA and VEGFC signaling pathways and angiopoietin 2 signaling pathway, by a single molecule, may help to curb the development of neovascularization-associated diseases, thereby further benefiting patients in clinical practice. Moreover, compared with combination dosing that requires multiple injections, a single dose of the trispecific molecule is capable of simultaneously achieving anti-VEGFA, anti-VEGFC and anti-Ang2 effects, which also advantageously reduces the dosing frequency. At the same time, the relatively strong blocking activity of the trispecific molecule of the present invention on the three target antigens also helps to prolong the dosing cycle.

In addition, in some preferred embodiments, the deliberately designed molecular form also gives the trispecific molecule of the present invention the characteristics of having good druggability and a relatively small molecular weight, which would be advantageous for production and therapeutic administration. Especially in the treatment of ocular diseases, drug molecules are often required to be administered intravitreally as injection dosage forms. Molecules with relatively small molecular weight may have relatively higher molar concentrations at the same dosing concentration by mass, thereby contributing to a reduced dosing frequency, improved patient compliance, and reduced treatment burden.

Therefore, the present invention provides, in one aspect, a trispecific fusion protein comprising (i) a VEGFA-binding domain, (ii) a VEGFC-binding domain and (iii) an Ang2-binding domain fused in one polypeptide chain, wherein:

the VEGFC-binding domain and the Ang2-binding domain comprise or consist of a VHH domain; the VEGFA-binding domain comprises or consists of or primarily consists of an extracellular binding domain from a VEGF receptor that specifically binds to VEGFA (also referred to herein as a VEGFA ligand capture domain or a mini-Trap domain). In some preferred embodiments, the polypeptide chain further comprises (iv) a dimerization domain, in particular a dimerization domain selected from a hinge region, a CH3 region, or an Fc region. In some embodiments of the fusion protein of the present invention, the dimerization domain is a hinge region with a length of 10-20 amino acids, preferably the hinge region comprises the core hinge region sequence CPPC (SEQ ID NO: 60) from an immunoglobulin IgG, or its modified sequence CPPCPPC (SEQ ID NO: 61), so that the two polypeptide chains may be paired through the hinge region and form a stable dimeric fusion protein. In other embodiments, as an alternative to the hinge region, the polypeptide chain comprises an Fc region or a CH3 region, or in other embodiments, the polypeptide chain comprises an Fc region or a CH3 region having a hinge region at the N-terminus. In some embodiments, the hinge region, CH3 region or Fc region is present at the C-terminus of the polypeptide chain; in other embodiments, the hinge region, CH3 region or Fc region is located between the two binding domains of the polypeptide chain, for example, in some embodiments, the VEGFA-binding domain is connected to the VEGFC-binding domain or the Ang2-binding domain or both at the C-terminus through the hinge region or through the CH3 region or Fc region. In some preferred embodiments, the polypeptide chain of the fusion protein of the present invention does not contain an immunoglobulin Fc region or a modified form thereof. In some further preferred embodiments, the fusion protein of the present invention is formed from a binding domain of a linker peptide with a length of 5-30 amino acids and a hinge region linking (i) to (iii).

In yet another aspect, the present invention also provides a multi-specific fusion protein having a unique configuration, wherein the fusion protein comprises:

a polypeptide chain comprising a first VHH domain that specifically binds to a first antigen, a second VHH domain that specifically binds to a second antigen, and a ligand capture domain that specifically binds to a third antigen, respectively, wherein the polypeptide further comprises a dimerization domain selected from a hinge region, a CH3 region, or an Fc region. Preferably, the dimerization domain is a hinge region, and preferably the hinge region is linked at the C-terminus of the ligand capture domain.

The hinge region used in the multi-specific fusion protein may be any of the hinge regions described herein. In some preferred aspects, the hinge region comprises or consists of an amino acid sequence selected from SEQ ID NOs: 12-19 and 45-46, more preferably comprises or consists of an amino acid sequence of SEQ ID NO: 12 or 13.

In addition to the hinge region, CH3 region, or Fc region, in some aspects, the multi-specific fusion protein of the present invention preferably comprises one or two additional linker peptides to ensure that each domain comprised in the polypeptide chain can perform its normal function. There are no particular limitations on the sequence of the linker peptide, e.g., the linker peptide may be any linker peptide sequence described herein. In some embodiments, the linker peptide may have a length of 5-12 amino acids, preferably comprising the amino acid sequence (GRPGS)n (SEQ ID NO: 59) or (GGGGG)n (SEQ ID NO: 58), wherein n=2 or 3.

Therefore, in some embodiments, the present invention also provides such a multi-specific fusion protein and the fusion protein comprises a polypeptide chain having the following structure, from the N-terminus to the C-terminus:

(i) a first VHH domain, a first linker peptide, a ligand capture domain, a hinge region, a second linker peptide, and a second VHH domain,

(ii) a ligand capture domain, a hinge region, a first linker peptide, a first VHH domain, a second linker peptide, and a second VHH domain, or

(iii) a first VHH domain, a first linker peptide, a second VHH domain, a second linker peptide, a ligand capture domain, and a hinge region.

In some embodiments, the present invention also provides such a multi-specific fusion protein, and the fusion protein comprises a polypeptide chain having the following structure, from the N-terminus to the C-terminus:

(i) a first VHH domain, a first linker peptide, a ligand capture domain, an Fc region or a CH3 region, a second linker peptide, and a second VHH domain,

(ii) a ligand capture domain, an Fc region or a CH3 region, a first linker peptide, a first VHH domain, a second linker peptide, and a second VHH domain, or

(iii) a first VHH domain, a first linker peptide, a second VHH domain, a second linker peptide, a ligand capture domain, and an Fc region or a CH3 region,

Optionally, the Fc region or CH3 region has a hinge region at the N-terminus.

It may be understood that in the foregoing embodiments, the first, second, and third antigens may be different from one another, and thus the multi-specific fusion protein is a trispecific fusion protein; but in some cases, the first antigen and second antigen may be the same, and whereby the multi-specific fusion protein is a bispecific fusion protein.

The first and second VHH domains used in the multi-specific fusion protein of the present invention may be the same or different and may be for the same or different target antigens. Those skilled in the art may choose to use the first VHH domain and second VHH domain in combination, as desired for specific applications.

The ligand capture domain used in the multi-specific fusion protein of the present invention may be a receptorligand interaction domain from a natural or artificial receptor that binds to the third antigen. For example, in some preferred aspects, the ligand capture domain is a VEGFA-binding domain from the extracellular region of VEGFR1/2. In some more preferred aspects, the multi-specific fusion protein is an anti-VEGFA/Ang2/VEGFC trispecific fusion protein according to the present invention.

The components constituting the fusion protein of the present invention, as well as exemplary fusion protein molecules, are described in detail below, using the anti-VEGFA/Ang2/VEGFC trispecific fusion protein of the present invention as an example. Those skilled in the art may understand that any combination of any technical features of these components is within the scope of consideration of the present invention, unless the context clearly indicates to the contrary. Moreover, those skilled in the art may understand that the fusion protein of the present invention may comprise any such combinations of the features, unless the context clearly indicates to the contrary.

## VEGFA Binding Domain

In the present invention, the VEGFA-binding domain of the fusion protein of the present invention is provided by a ligand capture domain or mini-Trap domain from a VEGF receptor. In some embodiments, the capture domain or mini-Trap domain comprises a natural sequence or artificial sequence extracellular domain from a VEGF receptor (particularly, VEGFR1 and/or VEGFR2) that specifically binds to VEGFA.

The VEGF receptor (VEGFR) is a tyrosine kinase receptor with an extracellular region composed of seven immunoglobulin (Ig)-like domains. Herein, the Ig-like domains are numbered sequentially from the N-terminus to the C-terminus of the VEGFR protein. For example, human VEGFR-1 comprises seven Ig-like domains numbered 1, 2, 3, 4, 5, 6, and 7, with Ig-like domain 1 at the N-terminus of the extracellular domain and Ig-like domain 7 at the C-terminus of the extracellular domain. VEGFRs interact with ligands through Ig-like domains. VEGFR-1 (Flt-1) binds to VEGFA, VEGFB, and PIGF and may act as a decoy receptor for VEGF or a regulator of VEGFR-2. VEGFR-2 (KDR/Flk-1) binds to all VEGF isotypes, and is a major mediator of VEGF-induced angiogenesis signal transduction. VEGFR-3 (Flt-4) binds to VEGFC and VEGFD, but does not bind to VEGFA, and acts as a mediator for lymphangiogenesis. It has been confirmed that a part of an Ig-like domain from the extracellular region of receptor VEGFR1 or VEGFR2, or a hybrid form thereof, is sufficient to act as a VEGFA-capturing ligand and compete with natural VEGFRs for binding to VEGFA. More detailed descriptions of such Ig-like domains are found in, e.g., U.S. Patent No. 7,531173, Yu, D. et al., (2012). Mol. Ther. 20(3): 938-947 and Holash, J. et al., (2002). PNAS. 99(17): 11,393-11,398, and hereby introduced by reference in their entirety.

In one embodiment, therefore, the VEGFA-binding domain according to the present invention comprises at least one Ig-like domain, e.g., at least two or three Ig-like domains, from VEGFR1 and/or VEGFR2. VEGFRs that may be used to provide the VEGFA-binding domain of the present invention may be from a mammal, such as a human, a baboon, a chimpanzee, a mouse or a rat, preferably from a human. In one embodiment, the VEGFA-binding domain according to the present invention comprises Ig-like domains 1-3 of VEGFR-1 (e.g., human VEGFR-1), Ig-like domains 2-3 of VEGFR-1 (e.g., human VEGFR-1), Ig-like domains 1-3 of VEGFR-2 (e.g., human VEGFR-2), or Ig-like domain 2 of VEGFR-1 (e.g., human VEGFR-1) and Ig-like domain 3 of VEGFR-2 (e.g., human VEGFR-2), or Ig-like domain 2 of VEGFR-1 (e.g., human VEGFR-1) and Ig-like domains 3-4 of VEGFR-2 (e.g., human VEGFR-2). In one preferred embodiment, the VEGFA-binding domain according to the present invention comprises Ig-like domain 2 of VEGFR-1 (e.g., human VEGFR-1) and Ig-like domain 3 of VEGFR-2 (e.g., human VEGFR-2). In addition, the present invention also contemplates the presence of a mutated VEGFA-binding domain in the natural sequence Ig-like domain from the VEGFRs described above, including a chemically modified form, or a variant form with 1-10 amino acid substitutions, deletions or additions.

In one preferred embodiment, the VEGFA-binding domain comprises Ig-like domain 3 of VEGFR2 (e.g., human VEGFR2) that is genetically fused to the C-terminus of Ig-like domain 2 of VEGFR1 (e.g., human VEGFR1). Preferably, the VEGFA-binding domain comprises the amino acid sequence of SEQ ID NO: 9, or an amino acid sequence having at least 85%, 90%, 95% or 99% identity thereto, or an amino acid sequence having one or more (preferably 1-10, more preferably 1-5) amino acid additions, deletions, and/or substitutions compared to SEQ ID NO: 9. Most preferably, the VEGFA-binding domain comprises the amino acid sequence shown in SEQ ID NO: 9, or is composed of the amino acid sequence shown in SEQ ID NO: 9.

## VEGFC Binding Domain

In the trispecific fusion protein according to the present invention, the VEGFC-binding domain is provided by a VHH domain. Therefore, in some embodiments, the VEGFC-binding domain according to the present invention comprises a VHH domain that specifically binds to VEGFC. Preferably, the VHH comprises CDR1, CDR2 and CDR3 sequences having the variable region shown in SEQ ID NO: 4. The CDR sequence ranges of the SEQ ID NO: 4 amino acid sequence in the variable region may be defined according to Kabat, AbM, Chothia, Contact, or IMGT schemes, or may be defined according to any two, or more, or all, of these definition schemes. In one embodiment, the VEGFC-binding domain according to the present invention comprises a CDR1 sequence, a CDR2 sequence and a CDR3 sequence defined according to Kabat or Chothia; or a CDR1 sequence defined according to a combination of Kabat and Chothia, and CDR2 and CDR3 sequences defined according to Kabat, in a variable region having SEQ ID NO: 4.

In one embodiment, the VEGFC-binding domain according to the present invention comprises

(i) CDR1 comprising SEQ ID NO: 1 or consisting of SEQ ID NO: 1;

(ii) CDR2 comprising SEQ ID NO: 2 or consisting of SEQ ID NO: 2; and

(iii) CDR3 comprising SEQ ID NO: 3 or consisting of SEQ ID NO: 3.

In one embodiment, the VEGFC-binding domain according to the present invention comprises the amino acid sequence shown in SEQ ID NO: 4. In yet another embodiment, the VEGFC-binding domain comprises an amino acid sequence having at least 80%, 85%, 90%, 95% or 99% identity to SEQ ID NO: 4, and retaining the ability to specifically bind to VEGFC. In yet another preferred embodiment, the VEGFC-binding domain comprises an amino acid sequence having one or more (preferably 1-10, more preferably 1-5) amino acid additions, deletions, and/or substitutions (e.g., conservative substitutions) compared to SEQ ID NO: 4, and retaining the ability to specifically bind to VEGFC. Preferably, the amino acid addition, deletion, and/or substitution does not occur in the CDR. Most preferably, in the trispecific fusion protein according to the present invention, the VEGFC-binding domain according to the present invention comprises the amino acid sequence shown in SEQ ID NO: 4, or consists of the amino acid sequence shown in SEQ ID NO: 4.

**Ang2 Binding Domain**

In the trispecific fusion protein according to the present invention, the Ang2-binding domain is provided by a VHH domain. Therefore, in some embodiments, the Ang2-binding domain according to the present invention comprises a VHH domain that specifically binds to Ang2. Preferably, the VHH comprises CDR1, CDR2 and CDR3 sequences having the variable region shown in SEQ ID NO: 8. The CDR sequence ranges of the SEQ ID NO: 8 amino acid sequence in the variable region may be defined according to Kabat, AbM, Chothia, Contact, or IMGT schemes, or may be defined according to any two, or more, or all, of these definition schemes. In one embodiment, the Ang2-binding domain according to the present invention comprises a CDR1 sequence, a CDR2 sequence and a CDR3 sequence defined according to Kabat or Chothia; or a CDR1 sequence defined according to a combination of Kabat and Chothia, and CDR2 and CDR3 sequences defined according to Kabat, in a variable region having SEQ ID NO: 8.

In one embodiment, the Ang2-binding domain according to the present invention comprises

(i) CDR1 comprising SEQ ID NO: 5 or consisting of SEQ ID NO: 5;

(ii) CDR2 comprising SEQ ID NO: 6 or consisting of SEQ ID NO: 6; and

(iii) CDR3 comprising SEQ ID NO: 7 or consisting of SEQ ID NO: 7.

In one embodiment, the Ang2-binding domain according to the present invention comprises the amino acid sequence shown in SEQ ID NO: 8. In yet another embodiment, the Ang2-binding domain comprises an amino acid sequence having at least 80%, 85%, 90%, 95% or 99% identity to SEQ ID NO: 8, and retaining the ability to specifically bind to Ang2. In yet another preferred embodiment, the Ang2-binding domain comprises an amino acid sequence having one or more (preferably 1-10, more preferably 1-5) amino acid additions, deletions, and/or substitutions (e.g., conservative substitutions) compared to SEQ ID NO: 8, and retaining the ability to specifically bind to Ang2. Preferably, the amino acid addition, deletion, and/or substitution does not occur in the CDR. Most preferably, in the trispecific fusion protein according to the present invention, the Ang2-binding domain according to the present invention comprises the amino acid sequence shown in SEQ ID NO: 8, or consists of the amino acid sequence shown in SEQ ID NO: 8.

**Dimerization Domain**

Hinge Region

In addition to the three antigen-binding domains, in some embodiments, the polypeptide chain of the fusion protein according to the present invention preferably also comprises a hinge region capable of driving the dimerization of the polypeptide chain. In some embodiments, the hinge region comprises at least two or preferably three cysteine residues. In some other embodiments, the hinge region comprises the C-terminus of the VEGFA-binding domain or is present at the C-terminus of the polypeptide chain.

In one embodiment, the hinge region comprises the CPPC (SEQ ID NO: 60) or CPPCPPC (SEQ ID NO: 61) amino acid sequence. In one embodiment, the hinge region comprises the CPPC (SEQ ID NO: 60) or CPPCPPC (SEQ ID NO: 61) amino acid sequence from the upper or partial upper hinge region of an (e.g., human) IgG isotype (IgG1, IgG2, IgG3 or IgG4) or a mutant sequence thereof, fused to the N-terminus, or is composed of the sequence. In one embodiment, the hinge region has a length of no more than 20 amino acid sequences, e.g., a length of 10, 11, 12, 13, 14, 15, 16, 17, 18, 19, or 20 amino acids. In another embodiment, the hinge region has a length of 10-15 amino acid sequences.

In one embodiment, the hinge region comprises an amino acid sequence selected from SEQ ID NOs: 12-19 and 45-46, or is composed thereof.

In some more preferred embodiments, the hinge region comprises the amino acid sequence of SEQ ID NO: 12, or is composed thereof; or comprises the amino acid sequence of SEQ ID NO: 13, or is composed thereof.

Fc Region

In addition to the three antigen-binding domains, the polypeptide chain of the fusion protein according to the present invention in some embodiments also comprises an Fc region capable of driving the dimerization of the polypeptide chain.

In the trispecific fusion protein of the present invention, a suitable Fc region may be an Fc region or a CH3 region from any natural immunoglobulin molecule, or a variant form thereof. For example, the Fc region may comprise two or three constant region domains, i.e., the CH2 domain, the CH3 domain, and optionally the CH4 domain. Preferably, the Fc region comprises, from the N-terminus to the C-terminus: CH2-CH3, more preferably, from the N-terminus to the C-terminus: hinge region-CH2-CH3. In some embodiments, the Fc region used in the molecule of the present invention is the Fc region from an IgG (particularly a human IgG), e.g., the Fc region of IgG1, IgG2 or IgG4, preferably the Fc region from human IgG1. Preferably, the Fc region comprises SEQ ID NO: 35 or an amino acid sequence having at least 90%, 95%, 96%, 97%, 98%, 99% or higher identity thereto.

CH3 Region

In addition to the three antigen-binding domains, some embodiments of the polypeptide chain of the fusion protein according to the present invention also comprise a CH3 region capable of driving the dimerization of the polypeptide chain.

In the trispecific fusion protein of the present invention, a suitable CH3 region may be the CH3 region from any natural immunoglobulin molecule, or a variant form thereof. Preferably, the CH3 region has a hinge region or a flexible linker peptide at the N-terminus. In some embodiments, the CH3 region used in the molecule of the present invention is the CH3 region from an IgG (particularly a human IgG), e.g., the CH3 region of IgG1, IgG2 or IgG4, preferably the CH3 region from human IgG1. Preferably, the CH3 region comprises SEQ ID NO: 47 or an amino acid sequence having at least 90%, 95%, 96%, 97%, 98%, 99% or higher identity thereto.

**Linker Peptide**

In the trispecific fusion protein according to the present invention, the antigen-binding domains may be linked by a linker peptide. There are no specific limitations on the linker peptide that may be used in the fusion protein of the present invention. Those skilled in the art are capable of readily determining linker peptide sequences that may be used based on the components to be linked and the location of the link after reading the present Description.

In some embodiments, the various antigen-binding domains of the present invention, and optionally a dimerization domain, are joined by a linker peptide. In one embodiment, the linker peptide is a flexible linker peptide with 5-50 amino acids, preferably comprising a linker peptide of glycine (G) and/or serine (S) and/or threonine residues (T). In one embodiment, the linker peptide has a length of 5-50 amino acids, e.g., a length of 8, 10, 15, 20, 25, or 30 amino acids, or a length of amino acids that falls between any two integers. In one embodiment, the linker peptide comprises the amino acid sequence (GRPGS)n (SEQ ID NO: 59) or (GGGGG)n (SEQ ID NO: 58), wherein n is an integer equal to or greater than 1, e.g., n is an integer of 2, 3, 4, 5, 6, or 7, preferably, n = 2 or 3. In some embodiments, the linker peptide comprises a hinge region. In some embodiments, the linker peptide does not comprise a hinge region.

In some embodiments, the fusion protein polypeptide chain of the present invention comprises at least one (and preferably one) linker peptide comprising a hinge region. The linker peptide may comprise a length of 20-50 amino acids, particularly a length of 20-30 amino acids. The hinge region may have a length of no more than 20 amino acid sequences, e.g., a length of 10-15 amino acid sequences. In some embodiments, the linker peptide comprises or is composed of the hinge region and a short linker of 5-20 amino acids at the C-terminus thereof. In some embodiments, the short linker is a short linker comprising or consisting of amino acid residues selected from glycine (G) and/or serine (S) and/or threonine residues (T), e.g., having a length of 5, 8, 10, 12, 15, 20 amino acids, or having a length of amino acids that falls between any two integers. In some other embodiments, the short linker comprises or consists of the amino acid sequence of SEQ ID NO: 10 or 11. Preferably, the linker peptide comprises the sequence of SEQ ID NO: 20 or 21. In some preferred embodiments, the linker peptide comprising a hinge region is linked to the C-terminus of the VEGFA-binding domain, and the VEGFA-binding domain is linked to the Ang2-binding domain or the VEGFC-binding domain or both at the C-terminus by the linker peptide.

In some other embodiments, the fusion protein polypeptide chain of the present invention links the antigen-binding domains together by a linker peptide that does not contain a hinge region. Where the polypeptide chain comprises a hinge region dimerization domain, the hinge region dimerization domain is preferably located at the C-terminus of the polypeptide chain and is directly linked to the binding domain at the C-terminus of the polypeptide chain. Where the polypeptide chain comprises an Fc region or a CH3 region dimerization domain, the Fc region or CH3 region may be directly linked to the C-terminus of any antigen-binding domain (e.g., when the Fc region or CH3 region has a hinge region at the N-terminus), or by a linker peptide. In these embodiments, the linker peptide may comprise a length of 5-20 amino acids, more preferably a length of 5-15 amino acids. In some embodiments, the linker peptide is a short linker comprising or consisting of amino acid residues selected from glycine (G) and/or serine (S) and/or threonine residues (T), e.g., having a length of 5, 8, 10, 12, 15, 20 amino acids, or having a length of amino acids that falls between any two integers. In some other embodiments, the linker peptide comprises the amino acid sequence of SEQ ID NO: 10 or 11.

## Exemplary Trispecific Fusion Protein

In the trispecific fusion protein according to the present invention, there are no particular limitations on the linking sequence between the antigen-binding domains and optionally the dimerization domain. Those skilled in the art are capable of readily determining linking sequences that may be used, as desired, after reading the present Description.
In some embodiments, the present invention provides a trispecific fusion protein, wherein the VEGFC-binding domain and the Ang2-binding domain are linked to the VEGFA-binding domain in separate form or in tandem form.
In some embodiments, the present invention provides a trispecific fusion protein, wherein the VEGFC-binding domain and the Ang2-binding domain are linked at the N-terminus or C-terminus of the VEGFA binding domain, respectively, preferably the Ang2-binding domain is bound to the N-terminus of the VEGFA-binding domain and the VEGFC-binding domain is bound to the C-terminus of the VEGFA-binding domain.
In some other embodiments, the present invention provides a trispecific fusion protein, wherein the VEGFC-binding domain and the Ang2-binding domain are linked in tandem and further linked at the C-terminus of the VEGFA-binding domain. In some embodiments, the VEGFC-binding domain is linked at the N-terminus of the Ang2-binding domain. In some other embodiments, the VEGFC-binding domain is linked at the C-terminus of the Ang2-binding domain.
In some embodiments, the fusion protein also comprises a hinge region, and preferably the hinge region is located at the C-terminus of the VEGFA-binding domain, or at the C-terminus of the polypeptide chain.
In some other embodiments, the fusion protein also comprises an Fc region, and preferably the Fc region is located at the C-terminus of the VEGFA-binding domain, or the C-terminus of the polypeptide chain.
In some other embodiments, the fusion protein also comprises a CH3 region, and preferably the CH3 region is located at the C-terminus of the VEGFA-binding domain, or at the C-terminus of the polypeptide chain.
In any of the embodiments described above, the linking may be direct linking, or performed by a linker peptide according to the Description herein.
In some preferred embodiments, the trispecific fusion protein according to the present invention comprises a hinge region. Preferably, the trispecific fusion protein comprises at least one polypeptide chain, and the polypeptide chain comprises, from the N-terminus to the C-terminus:

(i) an Ang2-binding domain, a first linker peptide, a VEGFA-binding domain, a hinge region, a second linker peptide, and a VEGFC-binding domain,
(ii) a VEGFC-binding domain, a first linker peptide, a VEGFA-binding domain, a hinge region, a second linker peptide, and an Ang2-binding domain,
(iii) a VEGFA-binding domain, a hinge region, a first linker peptide, an Ang2-binding domain, a second linker peptide, and a VEGFC-binding domain, or
(iv) a VEGFA-binding domain, a hinge region, a first linker peptide, a VEGFC-binding domain, a second linker peptide, and an Ang2-binding domain,
(v) an Ang2-binding domain, a first linker peptide, a VEGFC-binding domain, a second linker peptide, a VEGFA-binding domain, and a hinge region, or

(vi) a VEGFC-binding domain, a first linker peptide, an Ang2-binding domain, a second linker peptide, a VEGFA-binding domain, and a hinge region.

In one embodiment, the first linker peptide and the second linker peptide may be the same or different. In some embodiments, the first and second linker peptide is a short linker comprising or consisting of amino acid residues selected from glycine (G) and/or serine (S) and/or threonine residues (T), e.g., having a length of 5, 8, 10, 12, 15, 20 amino acids, or having a length of amino acids that falls between any two integers. In some embodiments, the first linker peptide and/or second linker peptide comprises the amino acid sequence $(G_4S)n$ (SEQ ID NO: 55), wherein n is an integer equal to or greater than 1, e.g., n is an integer of 2, 3, or 4. In one embodiment, the first linker peptide and/or second linker peptide consists of the amino acid sequence $(G_4S)_2$ (SEQ ID NO: 62) or $(G_4S)_3$ (SEQ ID NO: 63). In another embodiment, the first linker peptide and/or second linker peptide comprises the amino acid sequence $TS(G_4S)_n$ (SEQ ID NO: 56), wherein n is an integer equal to or greater than 1, e.g., n is an integer of 2, 3, or 4. In another embodiment, the first linker peptide and/or second linker peptide comprises the amino acid sequence $(GGGGG)n$ (SEQ ID NO: 58), wherein n is an integer equal to or greater than 2, e.g., n is an integer of 2 or 3. In another embodiment, the first linker peptide and/or second linker peptide comprises the amino acid sequence $(GGGGG)n$ (SEQ ID NO: 58) or $(GRPGS)n$ (SEQ ID NO: 59), wherein n is an integer equal to or greater than 2, e.g., n is an integer of 2 or 3. In one preferred embodiment, the first and second linker peptides each comprise the amino acid sequence of SEQ ID NO: 10. In another preferred embodiment, the first and second linker peptides each comprise the amino acid sequence of SEQ ID NO: 11.

In some embodiments, the hinge region comprises the CPPC (SEQ ID NO: 60) or CPPCPPC (SEQ ID NO: 61) amino acid sequence. In one embodiment, the hinge region comprises an amino acid sequence selected from SEQ ID NOs: 12-19 and 45-46, or is composed thereof. In one preferred embodiment, the hinge region comprises the amino acid sequence of SEQ ID NO: 12, or is composed thereof. In another preferred embodiment, the hinge region comprises the amino acid sequence of SEQ ID NO: 13, or is composed thereof. In one preferred embodiment, the hinge region comprises the amino acid sequence of SEQ ID NO: 15, or is composed thereof. In another preferred embodiment, the hinge region comprises the amino acid sequence of SEQ ID NO: 16, or is composed thereof. In one preferred embodiment, the hinge region comprises the amino acid sequence of SEQ ID NO: 17, or is composed thereof. In another preferred embodiment, the hinge region comprises the amino acid sequence of SEQ ID NO: 18, or is composed thereof. In one preferred embodiment, the hinge region comprises the amino acid sequence of SEQ ID NO: 45, or is composed thereof. It has been found that in the fusion protein of the present invention that comprises a hinge region as the dimerization domain, the hinge region that comprises a sequence, e.g., SEQ ID NO: 12, 13, 15, 16, 17, 18, or 45, is more conducive to improving the stability of the fusion protein dimer and thereby enhancing the production performance and druggability thereof.

In some preferred embodiments, the present invention provides a trispecific fusion protein comprising at least one polypeptide chain, and the polypeptide chain comprises, from the N-terminus to the C-terminus: an Ang2-binding domain, a first linker peptide, a VEGFA-binding domain, a hinge region, a second linker peptide, and a VEGFC-binding domain. Preferably, the polypeptide chain comprises

(i) an amino acid sequence selected from SEQ ID NOs: 23-28, 48 and 50; or
(ii) an amino acid sequence having at least 80%, 85%, 90%, 91%, 92%, 93%, 94%, 95%, 96%, 97%, 98%, 99% or higher identity to the amino acid sequence of (i); or
(iii) an amino acid sequence having at least 1-30, or 1-20, or 1-15, or 1-10, or 1-5 amino acid changes (e.g., substitutions, deletions and/or insertions, preferably substitutions, more preferably conservative substitutions) relative to the amino acid sequence of (i).

In one preferred embodiment, the present invention provides a trispecific fusion protein comprising the following polypeptide chain, wherein the polypeptide chain comprises:

(i) the amino acid sequence of SEQ ID NO: 24; or
(ii) an amino acid sequence having at least 80%, 85%, 90%, 91%, 92%, 93%, 94%, 95%, 96%, 97%, 98%, 99% or higher identity to the amino acid sequence of (i); or
(iii) an amino acid sequence having at least 1-30, or 1-20, or 1-15, or 1-10, or 1-5 amino acid changes (e.g., substitutions, deletions and/or insertions, preferably substitutions, more preferably conservative substitutions) relative to the amino acid sequence of (i).

Most preferably, the present invention provides a trispecific fusion protein comprising the following polypeptide chain, wherein the polypeptide chain comprises the amino acid sequence of SEQ ID NO: 24, or is composed thereof. Preferably, the fusion protein is a dimer composed of two of the polypeptide chains.

In one preferred embodiment, the present invention provides a trispecific fusion protein comprising the following polypeptide chain, wherein the polypeptide chain comprises:

(i) the amino acid sequence of SEQ ID NO: 25; or

(ii) an amino acid sequence having at least 80%, 85%, 90%, 91%, 92%, 93%, 94%, 95%, 96%, 97%, 98%, 99% or higher identity to the amino acid sequence of (i); or

(iii) an amino acid sequence having at least 1-30, or 1-20, or 1-15, or 1-10, or 1-5 amino acid changes (e.g., substitutions, deletions and/or insertions, preferably substitutions, more preferably conservative substitutions) relative to the amino acid sequence of (i).

Most preferably, the present invention provides a trispecific fusion protein comprising the following polypeptide chain, wherein the polypeptide chain comprises the amino acid sequence of SEQ ID NO: 25, or is composed thereof. Preferably, the fusion protein is a dimer composed of two of the polypeptide chains.

In some other preferred embodiments, the present invention provides a trispecific fusion protein comprising at least one polypeptide chain, and the polypeptide chain comprises, from the N-terminus to the C-terminus: a VEGFA-binding domain, a hinge region, a first linker peptide, an Ang2-binding domain, a second linker peptide, and a VEGFC-binding domain. Preferably, the polypeptide chain comprises

(i) an amino acid sequence selected from SEQ ID NOs: 29-32; or

(ii) an amino acid sequence having at least 80%, 85%, 90%, 91%, 92%, 93%, 94%, 95%, 96%, 97%, 98%, 99% or higher identity to the amino acid sequence of (i); or

(iii) an amino acid sequence having at least 1-30, or 1-20, or 1-15, or 1-10, or 1-5 amino acid changes (e.g., substitutions, deletions and/or insertions, preferably substitutions, more preferably conservative substitutions) relative to the amino acid sequence of (i).

Preferably, the present invention provides a trispecific fusion protein comprising the following polypeptide chain, wherein the polypeptide chain comprises the amino acid sequence of SEQ ID NO: 29, 30, or 31, or is composed thereof. Preferably, the fusion protein is a dimer composed of two of the polypeptide chains.

In some other preferred embodiments, the present invention provides a trispecific fusion protein comprising at least one polypeptide chain, and the polypeptide chain comprises, from the N-terminus to the C-terminus: an Ang2-binding domain, a first linker peptide, a VEGFA-binding domain, a second linker peptide, a VEGFC-binding domain, and a hinge region. Preferably, the polypeptide chain comprises

(i) an amino acid sequence selected from SEQ ID NOs: 33-34; or

(ii) an amino acid sequence having at least 80%, 85%, 90%, 91%, 92%, 93%, 94%, 95%, 96%, 97%, 98%, 99% or higher identity to the amino acid sequence of (i); or

(iii) an amino acid sequence having at least 1-30, or 1-20, or 1-15, or 1-10, or 1-5 amino acid changes (e.g., substitutions, deletions and/or insertions, preferably substitutions, more preferably conservative substitutions) relative to the amino acid sequence of (i).

Preferably, the present invention provides a trispecific fusion protein comprising the following polypeptide chain, wherein the polypeptide chain comprises the amino acid sequence of SEQ ID NO: 33 or 34, or is composed thereof. Preferably, the fusion protein is a dimer composed of two of the polypeptide chains.

In yet some embodiments, the present invention also provides a fusion protein comprising the following polypeptide chain, wherein the polypeptide chain comprises, from the N-terminus to the C-terminus:

(i) an Ang2-binding domain, a first linker peptide, a VEGFA-binding domain, an Fc region or CH3 region, a second linker peptide, and a VEGFC-binding domain,

(ii) a VEGFC-binding domain, a first linker peptide, a VEGFA-binding domain, an Fc region or CH3 region, a second linker peptide, and an Ang2-binding domain,

(iii) a VEGFA-binding domain, an Fc region or a CH3 region, a first linker peptide, an Ang2-binding domain, a second linker peptide, and a VEGFC-binding domain, or

(iv) a VEGFA-binding domain, an Fc region or a CH3 region, a first linker peptide, a VEGFC-binding domain, a second linker peptide, and an Ang2-binding domain,

(v) an Ang2-binding domain, a first linker peptide, a VEGFC-binding domain, a second linker peptide, a VEGFA-binding domain, and an Fc region or CH3 region, or

(vi) a VEGFC-binding domain, a first linker peptide, an Ang2-binding domain, a second linker peptide, a VEGFA-binding domain, and an Fc region or CH3 region.

In one embodiment, the first linker peptide and the second linker peptide may be the same or different. In some embodiments, the first and second linker peptide is a short linker comprising or consisting of amino acid residues selected from glycine (G) and/or serine (S) and/or threonine residues (T), e.g., having a length of 5, 8, 10, 12, 15, 20 amino acids, or having a length of amino acids that falls between any two integers. In some embodiments, the first linker peptide and/or second linker peptide comprises the amino acid sequence $(G_4S)n$ (SEQ ID NO: 55), wherein n is an integer equal to or greater than 1, e.g., n is an integer of 2, 3, or 4. In one embodiment, the first linker peptide and/or second linker peptide consists of the amino acid sequence $(G_4S)_2$ (SEQ ID NO: 62) or $(G_4S)_3$ (SEQ ID NO: 63). In another embodiment, the first linker peptide and/or second linker peptide comprises the amino acid sequence $TS(G_4S)_n$ (SEQ ID NO: 56), wherein n is an integer equal to or greater than 1, e.g., n is an integer of 2, 3, or 4. In another embodiment, the first linker peptide and/or second linker peptide comprises the amino acid sequence (GGGGG)n (SEQ ID NO: 58), wherein n is an integer equal to or greater than 2, e.g., n is an integer of 2 or 3. In another embodiment, the first linker peptide and/or second linker peptide comprises the amino acid sequence (GGGGG)n (SEQ ID NO: 58) or (GRPGS)n (SEQ ID NO: 59), wherein n is an integer equal to or greater than 2, e.g., n is an integer of 2 or 3. In one preferred embodiment, the first and second linker peptides each comprise the amino acid sequence of SEQ ID NO: 10. In another preferred embodiment, the first and second linker peptides each comprise the amino acid sequence of SEQ ID NO: 11.

In some embodiments, the Fc region comprises a natural hinge region sequence or a variant hinge region sequence, preferably the Fc region is the Fc region from a human IgG1, more preferably the Fc region comprises the amino acid sequence of SEQ ID NO: 35 or an amino acid sequence having at least 85%, 90%, 95% or 99% identity thereto.

In some embodiments, the CH3 region is the Fc region from a human IgG1, more preferably the CH3 region comprises the amino acid sequence of SEQ ID NO: 47 or an amino acid sequence having at least 85%, 90%, 95% or 99% identity thereto. Preferably, the CH3 region has a hinge region at the N-terminus.

In some embodiments, the present invention provides a trispecific fusion protein comprising the following polypeptide chain, wherein the polypeptide chain comprises:

(i) the amino acid sequence of SEQ ID NO: 22 or 49; or
(ii) an amino acid sequence having at least 80%, 85%, 90%, 91%, 92%, 93%, 94%, 95%, 96%, 97%, 98%, 99% or higher identity to the amino acid sequence of (i); or
(iii) an amino acid sequence having at least 1-30, or 1-20, or 1-15, or 1-10, or 1-5 amino acid changes (e.g., substitutions, deletions and/or insertions, preferably substitutions, more preferably conservative substitutions) relative to the amino acid sequence of (i).

Most preferably, the present invention provides a trispecific fusion protein comprising the following polypeptide chain, wherein the polypeptide chain comprises the amino acid sequence of SEQ ID NO: 22 or 49, or is composed thereof. Preferably, the fusion protein is a dimer composed of two of the polypeptide chains.

In some embodiments, the trispecific fusion protein of the present invention has biological activity selected from any or a plurality of the following:

(i) high affinity for binding to VEGFA, VEGFC, and Ang2, preferably, with an affinity constant KD value of 1-10 nM or less for bivalent binding to VEGFA; and an affinity constant KD value of 0.1-1 nM for bivalent binding to VEGFC and Ang2, as determined by bio-layer interferometry, e.g., the assay method shown in embodiment 3;
(ii) blocking of VEGFA and/or VEGFC-induced VEGFR2 signaling pathway activation;
(iii) blocking of VEGFC-induced VEGFR3 signaling pathway activation;
(iv) blocking of binding of Ang2 to Tie2-expressing cells;
(v) blocking of Ang2-induced Tie2 signaling pathway activation;
(vi) inhibition of VEGFC and VEGFA co-induced endothelial cell survival and proliferation;
(vii) inhibition of neovascularization of a tumor (e.g., a solid tumor, such as melanoma);
(viii) inhibition of the growth of a tumor (e.g., a solid tumor, such as melanoma);
(ix) suppression of the onset and/or development of neovascularization-related ocular diseases, e.g., by reducing the level of neovascularization, reducing vascular leakage, inhibiting fundus retinal edema and/or thickening caused by neovascularization.

Therefore, in some embodiments, the present invention provides an anti-VEGFA/VEGFC/Ang2 trispecific fusion protein that may be used in the treatment of neovasculogenesis-related diseases, such as cancers (e.g., solid tumors) and ocular diseases.

## Polynucleotides, Vectors, and Hosts

The present invention provides a nucleic acid encoding any of the above trispecific fusion proteins of the present invention. Also provided is a vector comprising the nucleic acid. In one embodiment, the vector is an expression vector. Also provided is a host cell comprising the nucleic acid or the vector. In one embodiment, the host cell is eukaryotic. In another embodiment, the host cell is selected from yeast cells, mammalian cells (e.g., CHO cells or 293 cells). In another embodiment, the host cell is prokaryotic.

A nucleic acid sequence encoding the molecule of the present invention may be generated by methods well known in the art, e.g., by de novo solid-phase DNA synthesis, or by PCR amplification. As is clear to those skilled in the art, each polypeptide amino acid sequence may be encoded by various nucleic acid sequences due to codon degeneracy. In addition, for ease of production and purification, the nucleic acid encoding the trispecific fusion protein of the present invention may also comprise a secretory signal peptide fused at the N-terminus of the fusion protein and/or a tag peptide fused at the C-terminus of the fusion protein and that is conducive to purification, such as a hexa-histidine tag.

The encoding nucleic acid encoding the trispecific fusion protein of the present invention may be introduced into a vector for amplification or expression. In one embodiment, the vector is an expression vector, e.g., a prokaryotic expression vector or a eukaryotic expression vector. The vector includes but is not limited to viruses, plasmids, cosmids, lambda phages and yeast artificial chromosomes (YACs).

The present invention also provides a host cell comprising the expression vector of the present invention. Host cells suitable for replicating and supporting the expression of the fusion protein of the present invention are well known in the art. Such cells may be transfected or transduced with a particular expression vector, as desired, and a large number of vector-containing cells may be incubated for inoculating a large-scale fermenter to obtain sufficient amounts of the molecule of the present invention for clinical applications. Suitable host cells include prokaryotic microbes, such as Escherichia coli, eukaryotic microbes such as filamentous fungi or yeast, or various eukaryotic cells such as Chinese hamster ovary cells (CHO), insect cells, and the like. Mammalian cell lines suitable for suspension culture may be used. Examples of useful mammalian host cell lines include SV40-transformed monkey kidney CV1 line (COS-7); human embryonic kidney line (HEK 293 or 293F cells), baby hamster kidney cells (BHK), monkey kidney cells (CV1), African green monkey kidney cells (VERO-76), human cervical cancer cells (HELA), canine kidney cells (MDCK), Buffalo rat liver cells (BRL 3A), human lung cells (W138), human liver cells (Hep G2), CHO cells, NSO cells, myeloma cell lines, such as YO, NS0, P3X63, Sp2/0, and the like. A review of mammalian host cell lines suitable for protein production is found in, e.g., Yazaki and Wu, Methods in Molecular Biology, Vol. 248 (B.K.C. Lo, Humana Press, Totowa, NJ), pages 255-268 (2003). In one preferred embodiment, the host cell is a CHO, HEK293 or NSO cell.

**Production and Purification of the Fusion Protein of the Present Invention**

In yet another aspect, the present invention provides a method for producing the trispecific fusion protein of the present invention, the method comprising: culturing a host cell comprising a [polynucleotide] encoding the polypeptide chain under conditions suitable for expressing the polypeptide chain of the fusion protein, thereby producing the fusion protein. For the purpose of this recombinant production, an expression vector comprising a polynucleotide encoding the polypeptide chain of the fusion protein of the present invention may be constructed using methods well known to those skilled in the art. The expression vector may be transfected or introduced into an appropriate host cell using various techniques, including, e.g., protoplast fusion, calcium phosphate precipitation, electroporation, retrovirus transduction, viral transfection, gene gun, liposome-based transfection, or other conventional techniques. After the fusion protein is expressed from the host cell, it may be recovered and/or purified by technical means known in the art.

Methods that may be used to purify the fusion protein of the present invention include, but are not limited to, high-performance liquid chromatography, ion exchange chromatography, gel electrophoresis, affinity chromatography, size exclusion chromatography, and the like. The actual conditions used to purify a particular protein also depend on factors such as net charge, hydrophobicity, hydrophilicity, and the like, and these are apparent to those skilled in the art. In one embodiment, after expression, the trispecific fusion protein of the present invention is isolated and purified by a protein A column.

The purity of the antibody molecule of the present invention may be determined by any one of various well-known analytical methods, and the well-known analytical methods include size exclusion chromatography, gel electrophoresis, high-performance liquid chromatography, and the like. The physical/chemical properties and/or biological activity of the fusion protein provided herein may be identified, screened, or characterized by various assays known in the art.

**Assays**

The physical/chemical properties and/or biological activity of the fusion protein provided herein may be identified, screened, or characterized by various assays known in the art.

The binding of the fusion protein of the present invention to human VEGFA and/or human VEGFC and/or human Ang2 may be determined by methods known in the art, such as ELISA, Western blotting, flow cytometry, and the like, or exemplary methods disclosed in the embodiments herein. For example, binding kinetics (e.g., $K_D$ values) of the molecule may be determined in a bio-layer interferometry assay using recombinant VEGFA and/or human VEGFC and/or human Ang2 protein. In some embodiments, the binding equilibrium dissociation constant, e.g., bivalent binding affinity, of the molecule to human VEGFA and/or human VEGFC and/or human Ang2 is determined at a temperature of 30°C using bio-layer interferometry assay (e.g., ForteBio affinity measurement), e.g., the method shown in embodiment 3. The binding activity of the fusion protein of the present invention to these cells may also be detected by flow cytometry using cells naturally or recombinantly expressing VEGFA and/or human VEGFC and/or human Ang2 protein. For example, the Ang2 cell binding experiment as in embodiment 7.

The VEGFA and/or VEGFC and/or Ang2 antagonistic/inhibitory activity of the fusion protein of the present invention may be determined by methods known in the art, such as ELISA blocking test, receptor fluorescent reporter molecule activation test, cell-based receptor phosphorylation test, cell proliferation test, or exemplary methods disclosed in the embodiments herein. For example, a cell-based receptor reporter molecule determination test, such as the method described in embodiment 4, may be employed to determine the molecular blockade of VEGFR2 signaling pathway activation induced by hVEGFA alone or hVEGFC alone or a combination thereof using, e.g., NFAT-RE-luc2P/KDR HEK293 cells. The inhibition of cell survival and/or proliferation induced by the molecule in vitro by hVEGFA alone or hVEGFC alone or a combination thereof may also be determined using a cell proliferation determination test, such as CCK-8 test, e.g., the methods described in embodiment 5 or 9, using lymphocytes or endothelial cells.

The anti-neovascularization effects of the molecule of the present invention may be measured by methods known in the art, such as in vitro tests and/or in vivo animal experiments. For example, VEGFA and VEGFC may be used to induce lumen formation in human umbilical vein endothelial cells (HUVECs) using an in vitro endothelial cell tube formation experiment, such as the method shown in embodiment 9, and to detect the inhibitory effect of the molecule on lumen formation induced by VEGFA and VEGFC in primary cells. The anti-neovascularization and/or anti-tumor effects of the molecule may also be detected, e.g., in a tumor-bearing mouse model, e.g., according to the method shown in embodiment 10. Or alternatively, an animal model of laser-induced choroidal neovascularization or an animal model of DL-AA-induced retinal neovascularization may be employed to observe the inhibition of choroidal neovascularization or retinal neovascularization by, e.g., color fundus photography, fundus fluorescein angiography, and optical coherence tomography, after administration of the molecule.

**Pharmaceutical Composition, Pharmaceutical Combination and Kit**

In one aspect, the present invention provides a composition, e.g., a pharmaceutical composition, and the composition comprises a trispecific fusion protein of the present invention formulated together with a pharmaceutically acceptable carrier. As used herein, "pharmaceutical carrier" includes any or all physiologically compatible solvents, dispersing media, isotonic agents, absorption-delaying agents, and the like. The pharmaceutical composition of the present invention is suitable for intravenous, intramuscular, subcutaneous, parenteral, rectal, spinal or epidermal administration (e.g., by injection or infusion). In one embodiment, the fusion protein of the present invention is formulated as a pharmaceutical composition suitable for injection administration. In some embodiments, the fusion protein of the present invention is formulated as a pharmaceutical composition suitable for intraperitoneal injection. In yet some embodiments, the fusion protein of the present invention is formulated as a pharmaceutical composition suitable for ocular injection administration (e.g., intravitreal injection). In some embodiments, the fusion protein of the present invention is the only active ingredient in the pharmaceutical composition. In some other embodiments, the pharmaceutical composition may comprise the fusion protein molecule described herein and more than one other therapeutic agent.

In another aspect, the present invention also provides a pharmaceutical combination comprising the fusion protein of the present invention and more than one therapeutic agent.

Therapeutic agents suitable for use in the pharmaceutical composition and pharmaceutical combination of the present invention may be therapeutic agents selected from any one of the following categories: (i) anti-neovascularization drugs; (ii) immunosuppressive drugs; (iii) anti-fibrotic drugs; (iv) neuroprotective drugs; and (v) drugs with tumor inhibitory effect.

The composition of the present invention may be in various forms. Such forms, e.g., include liquid, semi-solid, and solid dosage forms, such as liquid solutions (e.g., injectable solutions and infusible solutions), dispersions or suspensions, liposomal agents and suppositories. The preferred form depends on the expected mode of administration and therapeutic use. Common preferred compositions are in the form of injectable solutions or infusible solutions. Preferred modes of administration are parenteral (e.g., intravenous, subcutaneous, intraperitoneal (i.p.), intramuscular) injection. In one preferred embodiment, the antibody molecule is administered by intravenous infusion or injection. In another preferred embodiment, the antibody molecule is administered by intramuscular, intraperitoneal, or subcutaneous injection.

As used herein, the phrases "parenteral administration" and "parenteral mode of administration" mean modes of administration other than enteral and topical administration, typically administered by injection, and include, but are not limited to, intravenous, intramuscular, intra-arterial, intradermal, intraperitoneal, transtracheal, subcutaneous, and infusion.

Therapeutic compositions should generally be sterile and stable under the manufacturing and storage conditions. The composition may be formulated as a solution, microemulsion, dispersion, liposome, or lyophilized form. Sterile injectable solutions may be prepared by adding the active compound in the required amount to an appropriate solvent that is then disinfected by filtration. Typically, dispersions are prepared by incorporating the active compound into a sterile vehicle, and the sterile vehicle contains the basic dispersion medium and other components. Coating agents such as lecithin and the like may be used. In the case of dispersions, a suitable liquidity of the solution may be maintained by the use of surfactants. Prolonged absorption of the injectable composition may be caused by the inclusion of substances that delay absorption in the composition, such as monostearate and gelatin.

The pharmaceutical composition of the present invention may comprise a "therapeutically effective amount" or a "prophylactically effective amount" of the molecule of the present invention. A "therapeutically effective amount" refers to an amount effective to achieve a desired therapeutic result at a desired dose and for a desired period of time. The therapeutically effective amount may vary based on various factors such as disease state, age, sex, weight, and the like of the individual. A therapeutically effective amount is the amount at which any toxic or harmful effects are less than the therapeutically beneficial effects. A "therapeutically effective amount" preferably inhibits a measurable parameter (e.g., tumor growth rate) by at least approximately 20%, more preferably at least approximately 40%, even more preferably at least approximately 60%, and still more preferably at least approximately 80%, relative to an untreated subject. The ability of the molecule of the present invention to inhibit a measurable parameter (e.g., tumor volume) may be evaluated in an animal model system that predicts efficacy in human tumors.

A "prophylactically effective amount" refers to an amount effective to achieve a desired prophylactic result at a desired dose and for a desired period of time. Typically, since a prophylactic dose is used before or at a relatively early stage of the disease in a subject, the prophylactically effective amount is less than the therapeutically effective amount.

A kit comprising the antibody molecule described herein is also within the scope of the present invention. The kit may comprise one or more other elements, e.g., including: instructions for use; other reagents, such as markers or reagents for conjugation; a pharmaceutically acceptable carrier; and a device or other materials for administration to a subject.

## Use and Method

In one aspect, the present invention provides an in vivo and in vitro use and a method of application of the fusion protein of the present invention.

In some embodiments, the use and method of the present invention relate to applying the fusion protein of the present invention in vivo and/or in vitro to:

- binding to a VEGFA antigen and/or a VEGFC antigen and/or an Ang2 antigen;
- blocking the binding of VEGFA and/or VEGFC and/or Ang2 to a related receptor thereof, e.g., VEGFR2 and/or VEGFR3 and/or Tie2;
- inhibition of VEGFA and/or VEGFC and/or Ang2-induced VEGFR2 and/or VEGFR3 and/or Tie2 cell signal transduction activation;
- inhibition of VEGFA and/or VEGFC and/or Ang2-induced vascular endothelial cell survival, proliferation, and/or migration;
- inhibition of VEGFC and/or Ang2-induced lymphocyte survival and/or proliferation;
- inhibition of VEGFC and/or Ang2-induced lymphangiogenesis and lymphatic endothelial cell growth and migration;
- inhibition of VEGFA and/or VEGFC and/or Ang2-induced neovascularization and/or vascular leakage;
- inhibition of neovascularization and/or growth and/or metastasis of a tumor.

## Therapeutic Application

In one aspect, the present invention provides a use of the trispecific molecule of the present invention for prevention/treatment of a neovascularization-related disease and a corresponding treatment method. In some embodiments, the disease or disorder is a disease or disorder that would benefit from inhibition of neovascularization in the diseased tissue, including, but not limited to, solid tumors and ophthalmic diseases. In some embodiments, the disease is a solid tumor, preferably melanoma, wherein administration of the bispecific binding protein inhibits neovascularization within the tumor and/or growth of the tumor. In some other embodiments, the disease is an ocular disease.

In the treatment method according to the present invention, the antibody of the present invention may be used alone or in combination with other treatment methods or therapeutic agents, as appropriate. Other treatment methods/therapeutic agents that may be used in combination with the antibody of the present invention include, but are not limited to, other anti-neovascularization drugs, e.g., integrins, plasma kallikrein, and the like, immunosuppressive drugs, e.g., anti-complement HTRA1, anti-fibrotic drugs, e.g., anti-PDGF, anti-CTGF, and the like, and neuroprotective drugs, e.g., NGF and the like.

In some embodiments, the fusion protein of the present invention or a pharmaceutical composition comprising the fusion protein of the present invention is used as medicine for the treatment and/or prevention of a disease in a subject, preferably the subject is a mammal, more preferably a human.

In some embodiments, the present invention provides a method of treating a neovascularization-related disease, comprising administering to a subject the fusion protein of the present invention or a pharmaceutical composition thereof. In some embodiments, the disease is a solid tumor, wherein administration of the trispecific fusion protein inhibits neovascularization within the tumor and/or growth of the tumor. In some other embodiments, the disease is an ocular disease.

In some embodiments, the present invention provides a use of the fusion protein of the present invention in the preparation of a medicine for the treatment and/or prevention of a disease in a subject, wherein the disease is preferably a neovascularization-related disease, e.g., a solid tumor and an ocular disease. In some embodiments, the ocular disease is a choroidal neovascularization disease or a retinal neovascularization disease.

In any of the embodiments described above, one or more other active agents, e.g., chemotherapeutic agents and/or cancer therapeutic agents or anti-neovascular therapies, may be further administered in combination with the trispecific fusion protein of the present invention. Combination administration may be in the form of simultaneous administration, concurrent administration, or sequential administration in any order.

Detection and Diagnostic Applications

In yet another aspect, the present invention also provides a diagnostic method for detecting the presence of a related antigen in a biological sample, e.g., a serum, semen, or urine or tissue biopsy sample (e.g., from a hyperproliferative or cancerous lesion) in vitro or in vivo. This diagnostic method includes: (i) contacting a sample (and optionally, a control sample) with the (labeled or unlabeled) fusion protein of the present invention or administering the fusion protein to a subject under conditions that allow the interaction to occur, and (ii) detecting the formation of a complex between the fusion protein and the sample (and optionally, a control sample). Formation of the complex indicates the presence of the related antigen and, in some cases, may show applicability or need for treatment and/or prevention as described herein. In some embodiments, the present invention also provides a use of the fusion protein of the present invention for the preparation of a diagnostic tool for the preparation of diagnosing a disease, wherein the disease is preferably a neovascularization-related disease, e.g., a solid tumor and ocular disease.

**Embodiments**

**Embodiment 1.Production and Purification of Multi-specific Fusion Protein**

Expi293F cells (purchased from Thermo Fisher Scientific) were passaged into Expi293F cell culture medium (purchased from Thermo Fisher Scientific). The cell density was detected the day before transfection, and adjusted to $2 \times 10^6$ cells/mL with fresh Expi293 cell culture medium for continued culture. The cell density was adjusted to $3 \times 10^6$ cells/mL on the day of transfection.

1/10 of the final volume of the transfected Expi293F cells in Opti-MEM medium (purchased from Gibco) was used as the transfection buffer. 10 $\mu$g of the corresponding recombinant plasmid was added to each mL of transfection buffer, mixed well, and 30 $\mu$g of polyethylenimine (PEI) (Polysciences, CAT#: 23966) was added to each mL of the transfection buffer, mixed well, incubated at room temperature for 20 min, then the PEI/DNA mixture was gently poured into the Expi293F cell suspension, mixed well, and placed on a shaker for culture at 8% $CO_2$, 36.5°C, 120 rpm.

After 16-18 h of culture, 1/50 of the transfected culture volume of FEED (100 g/L Phytone Peptone + 100 g/L Difco Select Phytone) with a concentration of 200 g/L, glucose solution with a final concentration of 4 g/L, and VPA (valproic acid) with a final concentration of 2 mM/L were supplemented to the culture flask, gently mixed, and placed on a shaker at 8% $CO_2$, 36.5°C, 120 rpm for continued culture. Culture was continued for six days, the culture was collected, centrifuged at 4,000 rpm for 30 min, the cell supernatant was collected, filtered with 0.45 $\mu$M filter membrane, and purified by affinity chromatography and gel chromatography.

The specific operating steps of affinity chromatography purification are: Amsphere™ A3 (JSR Life Sciences, CAT#: 10000327-C05) protein A affinity chromatography column was selected, endotoxins were removed from the affinity chromatography column and tubing with 0.1 M NaOH for 2 h before purification, and then the tubing and column were washed with distilled water. The packed column was equilibrated with 5-fold column volume of 1×PBS (Gibco, CAT#: 10010023); the supernatant of the collected feed liquid was passed through the column, and the packed column was washed with 10-fold column volume of 1×PBS to remove non-specific binding protein; the packing was flushed with 5-fold column volume of elution buffer (100 mM glycine, pH 2.7), the eluent was collected, and the protein pH was adjusted to 6.0 with 2 M Tris for further gel chromatography.

The specific operating steps of gel exchange chromatography purification are: Superdex 200 Increase 10/300 GL (GE Healthcare, CAT#: 28-9909-44) gel chromatography column was selected and placed in the AKTApure system. Endotoxins in AKTApure system equipped with Superdex 200 Increase 10/300 GL gel chromatography column was removed with 0.1 M NaOH for 2 h, then the system and column were washed with distilled water; the column was equilibrated with 2-5-fold column volumes of 1×PBS until conductivity and pH were stable; the protein obtained by affinity chromatography was loaded, continued to be eluted with 1×PBS, impurities such as polymers were removed according to the ultraviolet absorption peak, and high purity samples were collected. The samples were filtered through a 0.22 μm membrane to determine the protein concentration.

The amino acid sequences and sequence numbers of the IAR044 multi-specific fusion protein of the present invention that is expressed and purified based on the procedures described above are provided in the Overview of Sequence Listing section appended to the back.

**Embodiment 2.Purity Identification of Multi-specific Fusion Protein Dimer**

SDS-PAGE analysis: A protein sample was taken, reducing loading buffer (Yeasen, CAT#: 20315ES05) or non-reducing loading buffer (Beijing Baiao Laibo, CAT#: WE0289) was added, heated at 70°C for 10 min, an appropriate amount of the sample was taken, precast gel 4-20% Mini-PROTEAN® TGX™ Precast Gel (Bio-Rad CAT#: 4561095) was added, the protein marker (Bio-Rad, CAT#: 1610375) was simultaneously added as a control, 150 V electrophoresis was performed for 50 min, stained with a protein staining system (GenScript, L00760C), and then photographed with a gel imager (Shanghai Tanon, Tanon-1600).

SEC-HPLC analysis: A protein sample was taken and the purity was detected using a size exclusion chromatography column, wherein the chromatographic column was a TSKgel G3000Swxl (7.8 × 300 mm, 5 μm) analytical column, the guard column was TSKgel guard column Swxl (6.0 × 40 mm, 7 μm), the mobile phase was 20 mmol/L phosphate buffer + 400 mmol/L NaClO4, pH 6.8, the sample tray temperature was 10°C, the column temperature was 25°C, the injection volume was 50 μL, the flow rate was 0.5 mL/min, the acquisition time was 30 min, and the FLD fluorescence detector was used, the PMT value was 7, the excitation wavelength was 285 nm, and the emission wavelength was 340 nm. The test sample was diluted with mobile phase to 1.0 mg/mL, and used as the test solution. The formulation buffer was diluted with the same method of treatment as described above and used as the blank solution. 50 μL each of the blank solution and test solution were injected into the liquid chromatography instrument to start the detection.

CE-SDS (capillary gel electrophoresis) analysis: A protein sample was taken and purity was detected by capillary gel electrophoresis. The capillary was an uncoated capillary with an inner diameter of 50 μm, a total length of 30.2 cm, and an effective length of 20.2 cm. Before electrophoresis, the capillary column was flushed with 0.1 mol/L sodium hydroxide, 0.1 mol/L hydrochloric acid, ultrapure water, and electrophoresis gel at 70 psi. The test sample was diluted with an appropriate amount of ultrapure water to 1.0 mg/mL, 50 μL of the above diluted sample was taken, placed in a 1.5 mL centrifuge tube, 45 μL of pH 6.5 sample buffer was added (0.32 g of citric acid monohydrate and 2.45 g of disodium hydrogen phosphate dodecahydrate were weighed, dissolved in 45 mL of ultrapure water, and made up to a constant volume of 50 mL to obtain citric acid-phosphate buffer, 200 μL of this buffer was accurately measured, 80 μL of 10% (w/v) sodium lauryl sulfate solution was added, water was added to 1 mL, and mixed well), respectively, 5 μL of 250 mmol/L NEM solution was added (62 mg of N-ethylmaleimide was weighed and dissolved in 2 mL of ultrapure water), mixed well and heated at 70 ± 2°C for 10 ± 2 min, cooled to room temperature and transferred to a sample bottle for use as the test solution. The same volume of formulation buffer as the test article was taken and the same operations as the method described above were performed to prepare the blank solution. Sample injection conditions: - 5 kV for 20 seconds; isolation voltage: - 10 kV for 35 min. The capillary column temperature was controlled at 25°C, and the detection wavelength was 220 nm.

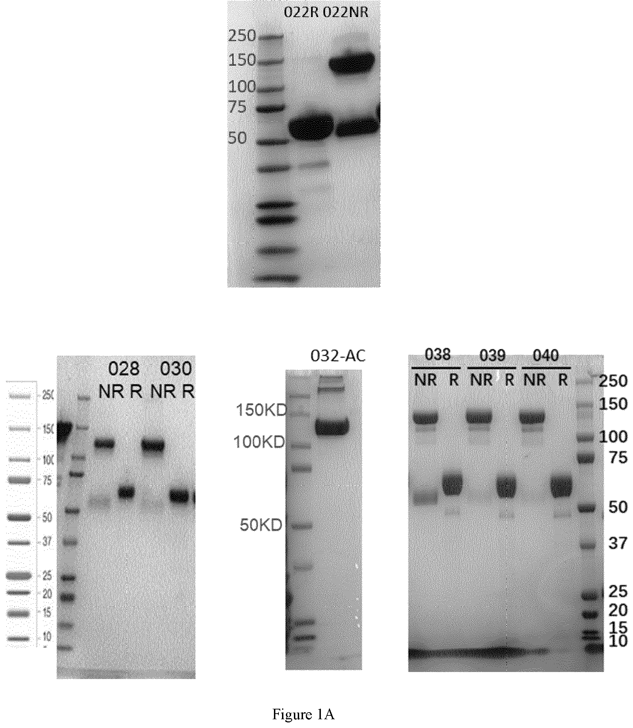

Figure 1A shows SDS-PAGE images of a representative multi-specific fusion protein after protein A one-step purification. As shown in the figure, all of the multi-specific fusion proteins tested formed dimers of the correct size; IAR044-028, 030, 032, and 038, 039, and 040 all formed a relatively high proportion of dimers relative to the IAR044-022 molecule having an unmodified hinge region.

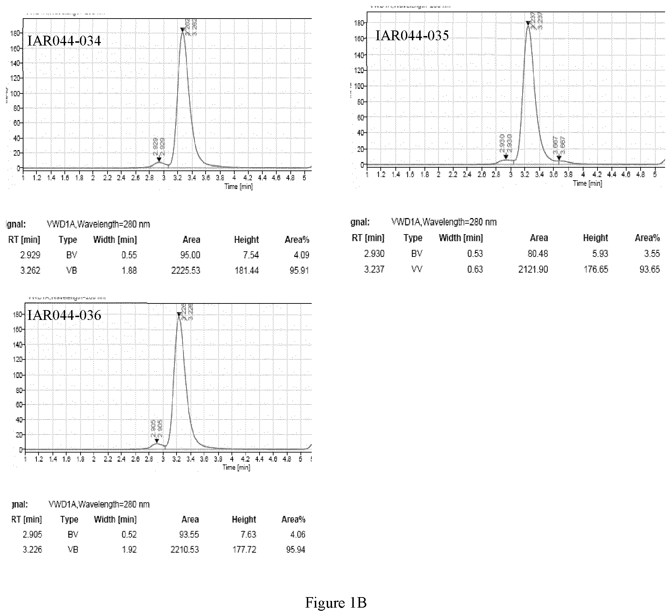

Figure 1B shows the results of SEC-HPLC purity analysis for molecules IAR044-034, 035, and 036.

Table 1 below shows the expression yield, SEC-HPLC purity, and CE-SDS purity of various different fusion proteins employing different first and second linker peptides and optionally the C-terminal hinge region.

Table 1. Expression Yield and Purity of Fusion Proteins

| Molecule Number | Molecule Configuration | First Linker Peptide | Second Linker Peptide | C-terminus Hinge Region | Yield (mg/L) | SEC Purity | CE-SDS Purity |
|---|---|---|---|---|---|---|---|
| IAR0 44-026 | Ang2_L1_Va_L2_Vc | (GGGGG) 2 (SEQ ID NO: 10) | EPKSSPPCPPC PAPCLLG (GGGGG) 2 (SEQ ID NO: 64) | | 165 | 93.28 | |
| IAR0 44-027 | Ang2_L1_Va_L2_Vc | (GGGGG) 2 (SEQ ID NO: 10) | DKTHTCPPCP APELLG-CH3 (SEQ ID NO: 65) | | 312 | 51.61 | 78.1 |
| IAR0 44-028 | Ang2_L1_Va_L2_Vc | (GGGGG) 2 (SEQ ID NO: 10) | EPKSSPPCPPC PAPC (GGGGG) 2 (SEQ ID NO: 66) | | 272 | 90.6 | 89 |
| IAR0 44-029 | Ang2_L1_Va_L1_Vc-hinge | (GGGGG) 2 (SEQ ID NO: 10) | (GGGGG) 2 (SEQ ID NO: 10) | DKTHTCP PCPPC (SEQ ID NO: 16) | 210 | 98.35 | 98.7 |
| IAR0 44-030 | Ang2_L1_Va_L2_Vc | (GGGGG) 2 (SEQ ID NO: 10) | ESKYGPPCPP CPPC (GGGGG) 2 (SEQ ID NO: 20) | | 340 | 94.6 | 96.8 |
| IAR0 44-031 | Ang2_L1_Va_L2_Vc | (GGGGG) 2 (SEQ ID NO: 10) | DKTHTCPPCP APCLLG (GGGGG) 2 (SEQ ID NO: 67) | | 220 | 88.7 | 84.4 |
| IAR0 44-032 | Ang2_L1_Va_L2_Vc | (GGGGG) 2 (SEQ ID NO: 10) | EKTHTCPPCP PC (GGGGG) 2 (SEQ ID NO: 21) | | 250 | 97.5 | 98.7 |
| IAR0 44-033 | Ang2_L1_Va_L1_Vc-hinge | (GRPGS)2 (SEQ ID NO: 11) | (GRPGS)2 (SEQ ID NO: 11) | DKTHTCP PCPPC (SEQ ID NO: 16) | 150 | 90.8 | 90.4 |
| IAR0 44-034 | Ang2_L1_Va_L2_Vc | (GGGGG) 2 (SEQ ID NO: 10) | DTTHTCPPCP PC (GGGGG) 2 (SEQ ID NO: 68) | | 350 | 95.9 | |
| IAR0 44-035 | Ang2_L1_Va_L2_Vc | (GGGGG) 2 (SEQ ID NO: 10) | EPKSCDTPPP CPPCP (GGGGG) 2 (SEQ ID NO: 69) | | 280 | 93.6 | |
| IAR0 44-036 | Ang2_L1_Va_L2_Vc | (GGGGG) 2 (SEQ ID NO: 10) | EPKSSDTPPPC PPCPPC (GGGGG) 2 (SEQ ID NO: 70) | | 320 | 95.9 | |

**Embodiment 3. Determination of Binding Kinetics of the Multi-specific Fusion Protein of the Present Invention to Antigen by Bio-layer Interferometry (BLI) and Surface Plasmon Resonance (SPR)**

The equilibrium dissociation constant (KD) of the binding of the multi-specific fusion protein of the present invention to the target antigen was determined using bio-layer interferometry determination technique (ForteBio). ForteBio affinity determination was performed using an existing method (Estep, P et al., High throughput solution Based measurement of antibody-antigen affinity and epitope binning. MAbs, 2013.5(2): pages 270-8).

Half an hour before the start of the experiment, according to the number of samples, a suitable number of AMQ (Pall, 1506091) (for sample detection) or AHQ (Pall, 1502051) (for positive control detection) sensors were soaked in SD buffer (PBS1×, BSA 0.1%, Tween-20 0.05%). 100 µL of SD buffer, multi-specific fusion protein, antigen (including human Ang2 (Beijing Sino Biological), human VEGF165 (R&D) and human VEGFC (R&D)) were taken and was added to a 96-well black polystyrene half area microplate (Greiner, 675076), respectively. The sensor position was selected based on the sample position and layout. The instrument setting parameters are as follows: Run steps: Baseline, Loading ~1 nm, Baseline, Association and Dissociation; the run time of each step depended on the sample binding and dissociation rate, and the rotational speed was 400 rpm and temperature was 30°C. ForteBio analysis software was used to analysis the $K_D$ value.

In the experiments described in the above assays, the affinities of representative multi-specific fusion proteins are shown in Table 2-4:

Table 2. ForteBio Detection of Affinity Constant (Equilibrium Dissociation Constant) of Bivalent Binding to VEGFAAntigen

| Multivalent Fusion Protein | Antigen | $K_D$ (M) | $K_{on}$ (1/Ms) | $k_{dis}$ (1/s) |
|---|---|---|---|---|
| IAR044-019 | | | | |
| IAR044-028 | | | | |
| IAR044-030 | | 5.15E-10 | 3.89E+05 | 2.00E-04* |
| IAR044-032 | VEGFA | 5.17E-10 | 3.87E+05 | 2.00E-04* |
| IAR044-038 | | 6.68E-10 | 4.50E+05 | 3.00E-04 |
| IAR044-039 | | 5.82E-10 | 4.68E+05 | 2.72E-04 |
| IAR044-040 | | 6.09E-10 | 4.81E+05 | 2.93E-04 |

Table 3. ForteBio Detection of Affinity Constant (Equilibrium Dissociation Constant) of Bivalent Binding to Antigen VEGFC

| Multivalent Fusion Protein | Antigen | $K_D$ (M) | $K_{on}$ (1/Ms) | $k_{dis}$ (1/s) |
|---|---|---|---|---|
| IAR044-019 | | | | |
| IAR044-028 | | | | |
| IAR044-030 | | 1.04E-09 | 1.93E+05 | 2.00E-04* |
| IAR044-032 | VEGFC | 1.01E-09 | 1.97E+05 | 2.00E-04* |
| IAR044-038 | | 9.20E-10 | 2.17E+05 | 2.00E-04 * |
| IAR044-039 | | 1.01E-09 | 2.25E+05 | 2.27E-04 |
| IAR044-040 | | 8.85E-10 | 2.36E+05 | 2.09E-04 |

Table 4. ForteBio Detection of Affinity Constant (Equilibrium Dissociation Constant) of Bivalent Binding to Antigen Ang2

| Multivalent Fusion Protein | Antigen | $K_D$ (M) | $K_{on}$ (1/Ms) | $k_{dis}$ (1/s) |
|---|---|---|---|---|
| IAR044-019 | | | | |
| IAR044-028 | | | | |
| IAR044-030 | | 2.06E-10 | 9.73E+05 | 2.00E-04* |
| IAR044-032 | | 2.20E-10 | 9.08E+05 | 2.00E-04 * |
| IAR044-038 | Ang2 | 4.75E-10 | 5.97E+05 | 2.83E-04 |
| IAR044-039 | | 7.05E-10 | 6.06E+05 | 4.27E-04 |
| IAR044-040 | | 7.83E-10 | 6.52E+05 | 5.10E-04 |

*Represents the dissociation constant exceeding the limit of detection of ForteBio

In the above tests, the affinity KD values of bivalent binding of antibodies IAR044-030 and IAR044-032 to human VEGFA were 5.15E-10 M and 5.17E-10 M, respectively; the affinity KD values of bivalent binding of antibodies IAR044-030 and IAR044-032 to human VEGFC were 1.04E-9 M and 1.01E-9 M, respectively; and the affinity KD values of bivalent binding of antibodies IAR044-030 and IAR044-032 to human Ang2 were 2.06E-10 M and 2.20E-10 M, respectively.

The equilibrium dissociation constants (KD) of the binding of each antibody expressed and purified in embodiment 1 to human VEGFA, VEGFC, and Ang2 were determined using surface plasmon resonance (SPR).

The KD of the antibody was determined by Biacore (GE Healthcare, T200), wherein after the antibody was captured on a chip by anti-human Fc antibody, the affinity and kinetic constants were obtained by detecting the binding and dissociation between the antigen human TIGIT and the captured antibody. The specific method is as follows: the anti-human Fc antibody (R&D CAT# MAB110) or antigen was conjugated to a CM5 chip (29-1496-03, GE Healthcare) using an amino conjugation kit (BR-1006-33, GE Healthcare) at a conjugation height of approximately 6,000 RU. 1 M ethanolamine was injected and the remaining active sites were blocked. The diluted antibody was captured at a flow rate of 10 μL/min in the fourth channel of the CM5 chip, with a capturing time of 60 s, and the third channel was the blank control channel. Then, the gradient-diluted antigens (including human Ang2 (Beijing Sino Biological), human VEGF165 (R&D) and human VEGFC (R&D)) were injected into both channels of the chip in the order from low concentration to high concentration, with an association time of 180 s and a dissociation time of 600 s. Finally, the chip was regenerated with Glycine pH 1.5 (BR-1003-54, GE Healthcare). The data results were kinetically analyzed using Biacore T200 analysis software with a 1:1 binding model. The results are shown in Tables 5-7 below.

Table 5. SPR Detection of Affinity Constant (Equilibrium Dissociation Constant) of Antigen-Antibody Binding

| Multivalent Fusion Protein | Antigen | $K_D$ (M) | $K_{on}$ (1/Ms) | $k_{dis}$ (1/s) |
|---|---|---|---|---|
| IAR044-019 | | 9.25E-13 | 5.34E+07 | 4.94E-05 |
| IAR044-028 | | | | |
| IAR044-030 | | 1.51E-10 | 3.99E+05 | 6.01E-05 |
| IAR044-032 | VEGFA | 2.03E-10 | 3.82E+05 | 7.75E-05 |
| IAR044-038 | | | | |
| IAR044-039 | | | | |
| IAR044-040 | | | | |

Table 6. SPR Detection of Affinity Constant (Equilibrium Dissociation Constant) of Antigen-Antibody Bivalent Binding

| Multivalent Fusion Protein | Antigen | $K_D$ (M) | $K_{on}$ (1/Ms) | $k_{dis}$ (1/s) |
|---|---|---|---|---|
| IAR044-019 | | <1.78E-11 | 5.62E+05 | <1.00E-05 |
| IAR044-028 | | | | |
| IAR044-030 | | <2.34E-10 | 4.28E+04 | <1.00E-05 |
| IAR044-032 | VEGFC | <2.76E-10 | 3.62E+04 | <1.00E-05 |
| IAR044-038 | | | | |
| IAR044-039 | | | | |
| IAR044-040 | | | | |

Table 7. SPR Detection of Affinity Constant (Equilibrium Dissociation Constant) of Antigen-Antibody Bivalent Binding

| Multivalent Fusion Protein | Antigen | $K_D$ (M) | $K_{on}$ (1/Ms) | $k_{dis}$ (1/s) |
|---|---|---|---|---|
| IAR044-019 | Ang2 | 5.56E-11 | 5.94E+05 | 3.30E-05 |
| IAR044-028 | | | | |
| IAR044-030 | | 9.32E-12 | 1.89E+06 | 1.76E-05 |
| IAR044-032 | | 1.20E-11 | 1.63E+06 | 1.92E-05 |
| IAR044-038 | | | | |
| IAR044-039 | | | | |
| IAR044-040 | | | | |

In the above tests, the affinity KD values of bivalent binding of antibodies IAR044-030 and IAR044-032 to human VEGFA were 1.51E-10 M and 2.03E-10 M, respectively; the affinity KD values of bivalent binding of antibodies IAR044-030 and IAR044-032 to human VEGFC were <2.34E-10 M and <2.76E-10 M, respectively; and the affinity KD values of bivalent binding of antibodies IAR044-030 and IAR044-032 to human Ang2 were 9.32E-12 M and 1.20E-11 M, respectively.

## Embodiment 4. Blocking of VEGFA or C-induced HEK293 KDR Activation by Anti-VEGFA/VEGFC/Ang2 Multi-specific Fusion Proteins

VEGFA or VEGFC is capable of binding to the related receptor VEGFR2 (also known as KDR), activating the VEGFR2 signaling pathway, and inducing vascular endothelial cell survival, proliferation, migration, and the like. In this study, the KDR reporter experimental system was used, and NFAT-RE-luc2P/KDR HEK293 cells (Promega Cat CS181401) were used to detect the blocking effect of gradient-diluted fusion protein molecules on VEGFA or VEGFC activation-related receptor signaling pathway. The experimental method was carried out according to the supplier's instructions.

The NFAT-RE-luc2P/KDR HEK293 cells that were changed to the experimental culture medium (DMEM medium containing 10% FBS) three days in advance were taken out, the old culture medium was aspirated, and washed once with PBS. Then the cells were digested with 1 mL of Accutase solution until the cells became round and detached from the wall, and the reaction was terminated with 5 mL of diluted culture medium (DMEM medium containing 10% FBS). The cells were aspirated into centrifuge tubes, centrifuged at 1,000 rpm for 5 min, and the culture medium was discarded. 10 mL of the diluted culture medium was added to resuspend the cells, mixed well and counted. The cell viability should be above 90%. The cell density was adjusted to $0.8 \times 10^6$ cells/mL with the dilution culture medium, and the cells were added to the 96-well white cell culture plate at 50 $\mu$L/well according to the experimental layout.

A mixture with VEGFA (R&D) at a concentration of 120 ng/mL or VEGFC (R&D) at a concentration of 200 ng/mL and a gradient-diluted fusion protein to be tested was formulated. After leaving the mixture to stand for 30 min, the mixture was added to a 96-well white cell culture plate containing cells at 50 $\mu$L/well, and incubated in a 37°C, 5% carbon dioxide incubator for 6 h. A blank control, as well as a control comprising only VEGFA or VEGFC molecules, and a control with an IgG isotype control antibody or anti-VEGFA molecule Eylea or anti-VEGFC molecule OPT302, added as an alternative to the fusion protein to be tested, were also set.

The 96-well white cell culture plate that was incubated for 6 h was taken out from the carbon dioxide incubator and equilibrated for 10-15 min to room temperature. The Bio-Glo Luciferase Assay System (Promega) that was equilibrated to room temperature in advance was added to a 96-well white cell culture plate at 100 $\mu$L/well according to the experimental layout, and incubated at room temperature in the dark for 5 min.

Fluorescence reading was performed using a multi-mode microplate reader. The chemiluminescence mode was selected for the plate reading mode, the endpoint method was selected for the plate reading type, and the wavelength was set to full wavelength. The fluorescence was collected column by column, and the collection time for each column was 1,000 ms.

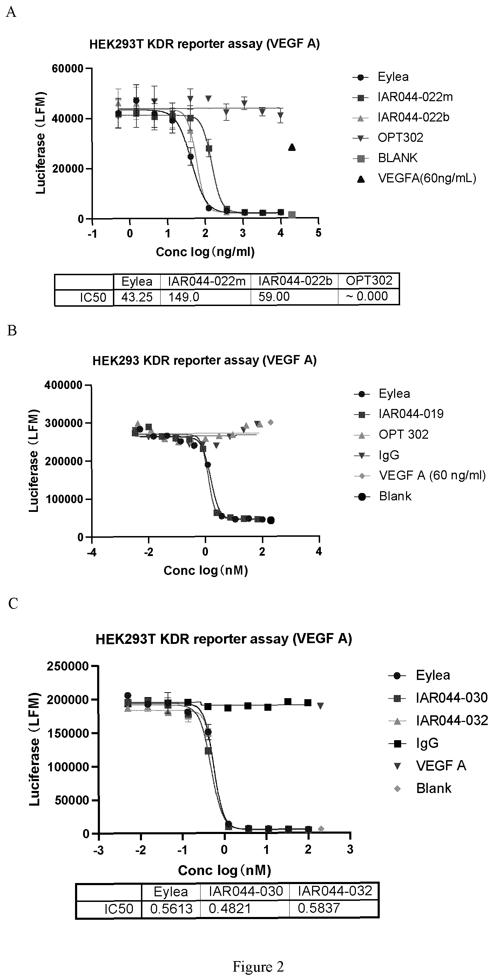

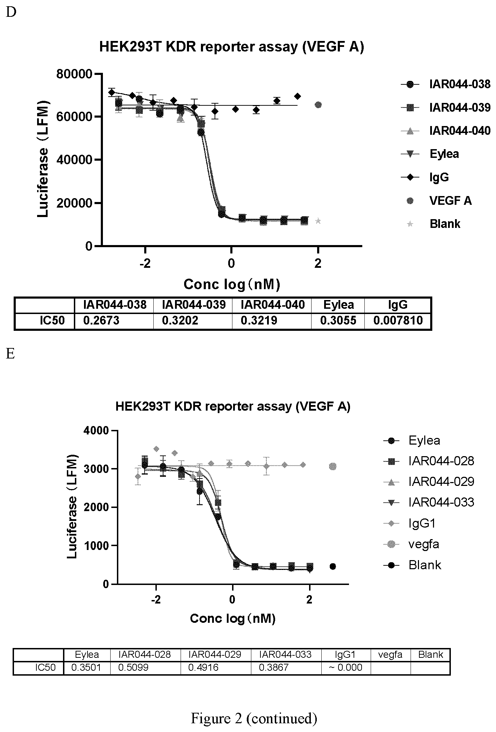

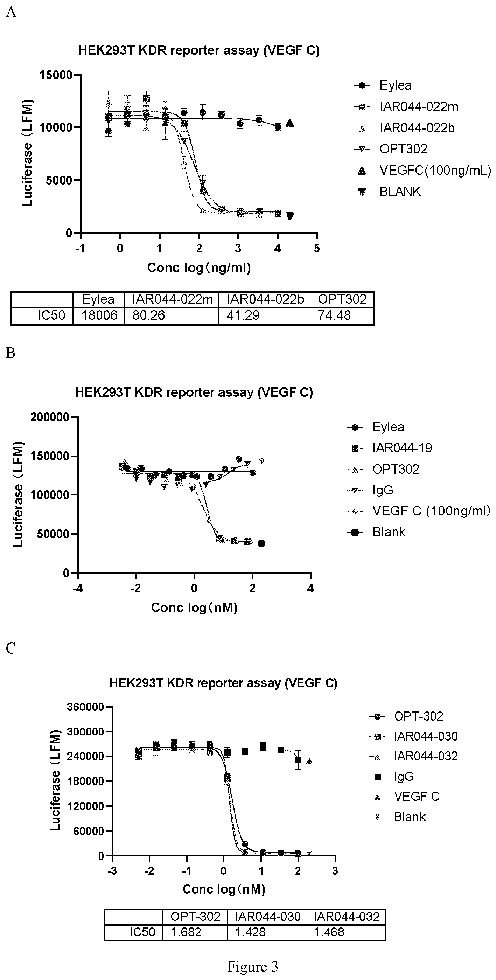

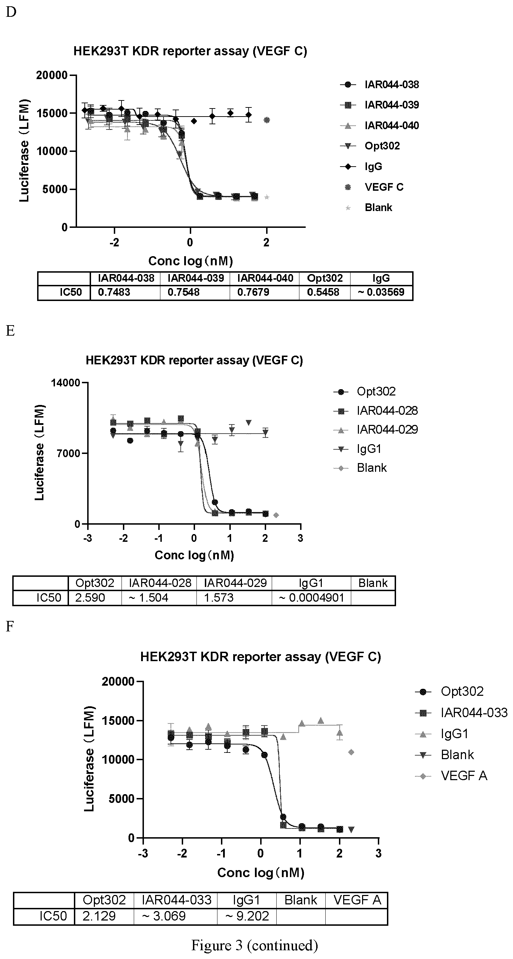

In the experiment described in the above assay, the detection results are shown in Figures. 2 and 3, and the trispecific fusion proteins of the present invention tested are capable of blocking VEGFA or VEGFC-induced KDR signaling pathways activation.

## Embodiment 5. Blocking of VEGFC-Induced BaF3-FLT4 Proliferation by Anti-VEGFA/VEGFC/Ang2 Multi-specific Fusion Proteins

In this study, the multi-specific fusion proteins of the present invention and recombinant human VEGFC protein were co-incubated with BaF3-FLT4 cells (BaF3 cells overexpressing FLT4 (VEGFR3), Institute of Basic Medical Sciences, China Academy of Chinese Medical Sciences). The number of viable cells was detected by the CCK-8 kit (Dojindo) to reflect the inhibitory effect of different fusion proteins on VEGFC-induced BaF3-FLT4 proliferation.

BaF3 cells overexpressing FLT4, BaF3-FLT4, were obtained by infecting BaF3 cells using a lentivirus carrying the FLT4 gene.

The cell proliferation inhibition test was performed according to the instructions for use of the CCK-8 kit. The experimental culture medium was formulated using 1640 medium containing 10% FBS. The maximum final concentration of the test antibody was 10 nM, and it was serially diluted in 1:3 ratio. At the same time, a blank control group (blank) and a VEGFC experimental group with only VEGFC added were set up. In the test system, the final concentration of hVEGFC (R&D) was 20 ng/mL, and the final concentration of BaF3-FLT4 cells was $2 \times 10^5$ cells/mL. The cells were incubated in 96-well plates in the 100 uL/well system in a 37°C $CO_2$ incubator for 72 h. Then, 15 $\mu$L of CCK-8 was added to each well and incubated in a 37°C $CO_2$ incubator for 4 h. The absorbance value was determined using dual wavelength, the detection wavelength was 450 nm, and the reference wavelength was 620 nm. The OD450-OD620 value was calculated.

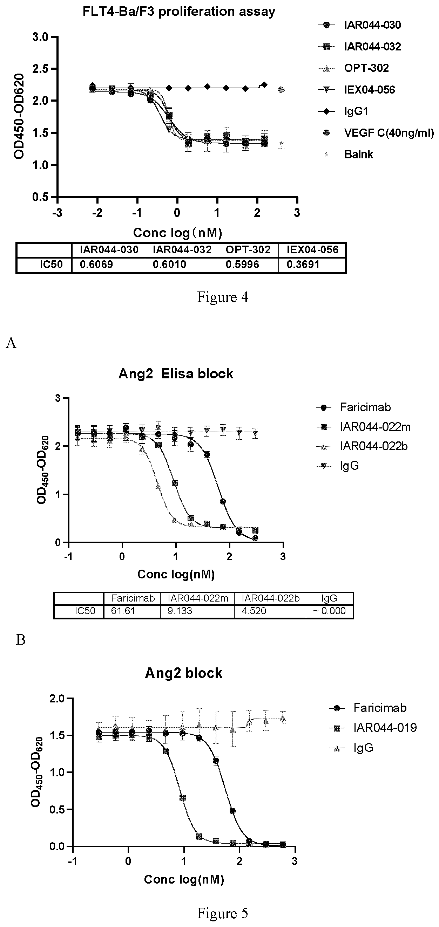

The experimental results are shown in Figure 4. The trispecific fusion proteins of the present invention are capable of effectively inhibiting hVEGFC-induced BaF3-FLT4 survival and proliferation in vitro.

## Embodiment 6. Blocking of Ang2 Binding to Tie2 by Anti-VEGFA/VEGFC/Ang2 Multi-specific Fusion Proteins

The ability of multi-specific fusion proteins of the present invention, as well as the control antibody, Faricimab, to block the binding of human Ang2 to hTie2 was detected by ELISA.

The hTie2 protein (Beijing Sino Biological) was resuspended with PBS and formulated to a concentration of 2 ug/mL for coating the microplate overnight. The plate was blocked with 5% BSA for 1 h. The biotin antigen Recombinant Biotinylated hAngiopoietin-2 protein (R&D) was diluted to 600 ng/mL, and added to the microplate at 50 $\mu$L/well. The multi-specific fusion proteins prepared as described above were gradient-diluted from the maximum concentration to a total of 8 or 12 dilution gradients. The gradient-diluted fusion protein was added to the microplate at 50 $\mu$L/well, and incubated on ice with PBS for 30 min, wherein the final antigen concentration was 300 ng/mL. The microplate containing the antigen and fusion protein mixture was incubated for 90 min, washed with PBS three times, the supernatant was discarded, Avidin-HRP (Invitrogen) diluted 1:10,000 was added at 100 $\mu$L/well, incubated at room temperature for 30 min, and washed with PBS six times. The color was developed using TMB chromogenic solution (Solarbio) at 100 uL/well for 1 min and terminated with stop solution (Solarbio) at 100 uL/well. The $OD_{450}$ and $OD_{620}$ absorbance values were read using a microplate reader.

The experimental results showed that the multi-specific fusion proteins of the present invention and the control antibody, Faricimab, achieved complete blocking effect, and the multi-specific fusion proteins of the present invention are significantly superior to the control antibody, Faricimab, in terms of IC50 values. (Refer to Figure 5)

## Embodiment 7. Blocking of Human Ang2-Fc Protein Binding to 293 Cells Overexpressing Tie2 by Anti-VEGFA/VEGFC/Ang2 Multi-specific Fusion Proteins

The ability of the multi-specific fusion proteins of the present invention (IAR044-030 and IAR044-032) and control antibody to block human Ang2-hFc binding to Tie2 on the cell surface was detected by flow cytometry (FACS).

Expi-293 cells (293-tie2 cells) overexpressing human Tie2 were generated by transfecting pCHO1.0 vector (Invitrogen) carrying the human Tie2 gene cloned into MCS.

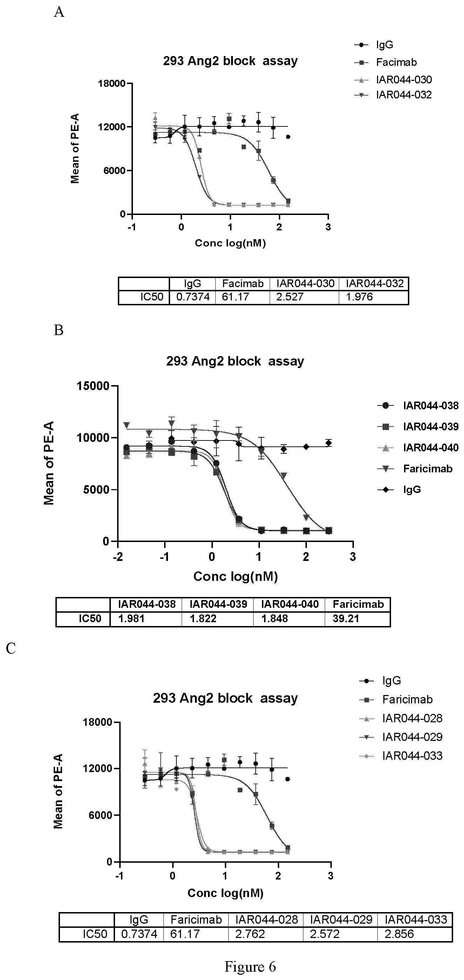

The antigen hAng2-Fc protein (Beijing Sino Biological) was diluted to 4 ug/mL, and added to the plate wells at 50 $\mu$L/well. The anti-Ang2 molecules (IAR044-030, IAR044-032 and control antibody Faricimab) were diluted by a 2-fold gradient starting from the maximum concentration of 800 nM to a total of 12 dilution gradients, added to the plate wells at 50 $\mu$L/well and incubated on ice with PBS for 30 min, wherein the final antigen concentration was 2 ug/mL and the maximum final concentration of the anti-Ang2 molecules was 400 nM. The 293-tie2 cells were adjusted to $2 \times 10^5$ cells/well, at 100 $\mu$L/well. The cells were centrifuged at 300 g for 5 min, the supernatant was discarded, resuspended in the antigen/anti-Ang2 molecular mixture, and incubated on ice for 30 min. PBS was added at 100 $\mu$L/well, centrifuged at 300 g for 5 min, and washed with PBS once. 1:200-diluted goat anti-human IgG-PE (SouthernBiotech) was added at 100 $\mu$L/well, and placed in an ice bath for 20 min. PBS was added at 100 $\mu$L/well, centrifuged at 300 g for 5 min, and washed with PBS once. The cells were resuspended with 100 $\mu$L of PBS, and the fluorescence signal value of the cells was detected by a cell flow cytometer (BD Biosciences). Based on the MFI thereof, a concentration-dependent curve was fit with GraphPad. The results are shown in Figure 6.

The experimental results showed that IAR044-030 and IAR044-032, as well as the control antibody, Faricimab, achieved complete blocking, and IAR044-030 and IAR044-032 are significantly superior to the control antibody in terms of IC50. (Refer to Figure 6)

## Embodiment 8. Inhibition of Human Ang2-Fc Protein-induced Tie2 Phosphorylation in 293-tie2 Cells by Anti-VEGFA/VEGFC/Ang2 Multi-specific Fusion Proteins

The hAng2-induced phosphorylation experiment was used to detect the inhibitory effect of the fusion proteins of the present invention on hAng2-Fc-induced tie2 phosphorylation.

In this experiment, the fusion proteins of the present invention and recombinant hAng2-fc protein were incubated with expi293 cells (293-tie2 cells) overexpressing tie2, and the inhibitory effect of different fusion proteins on hAng2-fc-induced Tie2 phosphorylation was reflected by detecting the content of phosphorylated Tie2 in the system.

293-tie2 cells overexpressing Tie2 were taken, diluted to $2 \times 10^6$ cells/mL, added to a 96-well plate at 100 $\mu$L/well, centrifuged at 400 g for 5 min, and the supernatant was removed.

The experimental culture medium was formulated using Expi293 medium, wherein the maximum final concentration of the test fusion protein was 60 ug/mL, and serially diluted in 1:2 ratio in sequence; the final concentration of hAng2-fc was 2.5 ug/mL. The anti-VEGFA/Ang2 bispecific antibody, Faricimab, as well as the bispecific fusion protein (IEX04-56) having a similar anti-VEGFC VHH domain to the fusion protein of the present invention to be tested, or the bispecific antibody (IEX04-012) with the same anti-Ang2 VHH domain were used as controls.

The cells were resuspended with experimental culture medium at 100 uL/well, and incubated at 37°C for 15 min. The cells were centrifuged to remove the culture medium, 100 $\mu$L of NP-40 lysis buffer containing 1% protease and phosphatase inhibitors was added, and placed on ice for 30 min. The cells were centrifuged at 2,000 g, the protein supernatant was collected, and stored in a -80°C refrigerator.

pTie2 concentration was detected according to the phosphorylation Tie2 ELISA kit (R&D, CAT#: DYC2720E) scheme. In other words, capture antibody was coated onto the microplate at a concentration of 4 ug/mL at 4°C overnight. The microplate was washed with PBS three times, and blocked with 5% BSA for 1 h. 100 uL of the test sample (may be diluted 2-3-fold) and the control pTie2 (used to prepare the standard curve) were added, and incubated at room temperature for 2 h. The test sample was washed with PBST three times, 100 $\mu$L of HRP-conjugated anti-pTyr antibody was added, and incubated at room temperature for 2 h. The solution was washed with PBST six times, 100 $\mu$L of TMB was

added for color development, and 100 $\mu$L of stop buffer was added after 15 min to terminate the reaction. The $OD_{450}$-$OD_{620}$ values were determined.

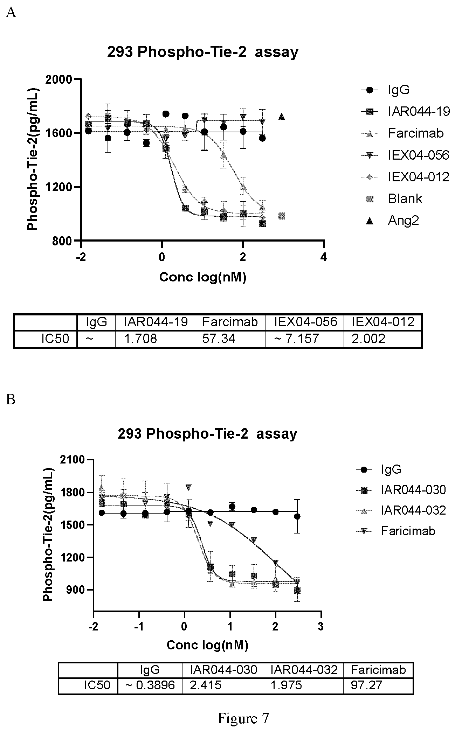

The experimental results are shown in Figure 7. The multi-specific fusion proteins of the present invention are capable of effectively inhibiting hAng2-Fc-induced Tie2 phosphorylation in 293 cells in vitro, and the inhibitory activity is superior to that of the positive control antibody, Faricimab.

### Embodiment 9. Inhibition of VEGFA + VEGFC-Induced HUVEC Proliferation by Anti-VEGFA/VEGFC/Ang2 Multi-specific Fusion Proteins

VEGFA and VEGFC may act on VEGFR and other associated receptors in vascular endothelial cells to promote the survival, proliferation and migration of vascular endothelial cells, thereby inducing neovascularization. In the present experiment, VEGFA and VEGFC were used to jointly induce the survival and proliferation of human umbilical vein endothelial cells (HUVECs), and the inhibitory effect of the multi-specific fusion proteins of the present invention on VEGFA and VEGFC-induced primary cell survival and proliferation was detected.

In the present embodiment, HUVEC survival and proliferation were determined by CCK-8, and the specific method is as follows: The cells were treated one day in advance, placed in a 96-well culture plate at 2,000 cells/well and incubated for 24 h in a 37°C, 5% carbon dioxide incubator. After the cells adhered to the wall, the experimental culture medium containing a final concentration of 5 ng/mL VEGFA and 60 ng/mL VEGFC and the gradient-diluted fusion protein were prepared, the endothelial cell culture medium in the 96-well plate was replaced, and incubated in a 37°C, 5% carbon dioxide incubator for 72 h. The anti-VEGFA molecule, Aflibercept, and the anti-VEGFC molecule, OPT302, and the 1:1 molar ratio mixture thereof, as well as the anti-VEGFC/VEGFA bispecific fusion protein (IEX04-056) and the anti-Ang2/VEGFA bispecific antibody (IEX04-012) were used as controls.

After the cells were incubated with the experimental culture medium, the CCK-8 detection solution (Dojindo) was added at 10 $\mu$L/well, and placed in a 37°C, 5% carbon dioxide incubator for incubation for 12-24 h. The absorbance $OD_{450}$-$OD_{620}$ was read using a multi-mode microplate reader.

In the experiment described in the above assay, the detection results are shown in Fligure 8. The trispecific fusion proteins of the present invention are capable of completely inhibiting VEGFA + VEGFC-induced HUVEC proliferation and survival.

### Embodiment 10. Experiment of Inhibition of A375 Tumor Neovascularization by Anti-VEGFA/VEGFC/Ang2 Multi-specific Fusion Proteins

The present embodiment determined the anti-neovascularization and anti-tumor effects of the multi-specific fusion proteins of the present invention in nude mice by inoculating A375 human malignant melanoma cells at 3 x $10^6$ cells per mouse.

Human nude mice:

Female nude mice with BALB/c background were purchased from Beijing Vital River Laboratory Animal Technology Co., Ltd., and the grade was SPF grade. The mice were acclimatized for seven days upon arrival and then the study began.

Cells:

Human A375 cells were purchased from ATCC (CAT#: CRL-1619), and routine passaging was carried out in strict accordance with the instructions for use for subsequent in vivo experiments. The cells were centrifuged and collected, and A375 cells were dispersed with PBS and Matrigel in a ratio of 1:1 to prepare a cell suspension with a cell concentration of 1.5 x $10^7$ cells/mL. The mice were subcutaneously injected with A375 cell suspension at 0.2 mL/mouse on Day 0, i.e., the inoculation amount was 3 x $10^6$ cells/mouse.

Drug administration:

The mice were randomized (six mice per group). The dose and mode of administration are shown in Table 8. PBS (purchased from Gibco) was used as a negative control, and the doses were administered on Days 6, 8, 10, 12, 14, 16, 18 and 20 after inoculation, respectively. The tumor volume and body weight of the mice were monitored twice a week. Body weight and tumor volume were determined prior to each dose. The relative tumor growth inhibition rate (TGI%) was calculated on Day 21 after inoculation. The calculation formula is as follows: TGI% = 100% * (tumor volume of control group - tumor volume of treatment group) / (tumor volume of control group - tumor volume of control group before drug administration). Tumor volume determination: The largest long axis (L) and the largest wide axis (W) of the tumor were determined using a vernier caliper, and the tumor volume was calculated as follows: V = L × W$^2$ / 2. Body weight was determined by electronic balance.

Table 8. Experimental Design

| Group | Dose | Number of Doses | Mode of Administration |
|---|---|---|---|
| IgG | 25 mg/kg | Q2DX8 | Intraperitoneal injection |
| Aflibercept | 20 mg/kg | Q2DX8 | Intraperitoneal injection |
| IEX04-010* | 25 mg/kg | Q2DX8 | Intraperitoneal injection |
| IEX04-056* | 25 mg/kg | Q2DX8 | Intraperitoneal injection |
| IAR044-030 | 20 mg/kg | Q2DX8 | Intraperitoneal injection |

*: The structure of IEX04-010 and IEX04-056 molecules that were used as controls are shown in SEQ ID NOs: 43 and 44.

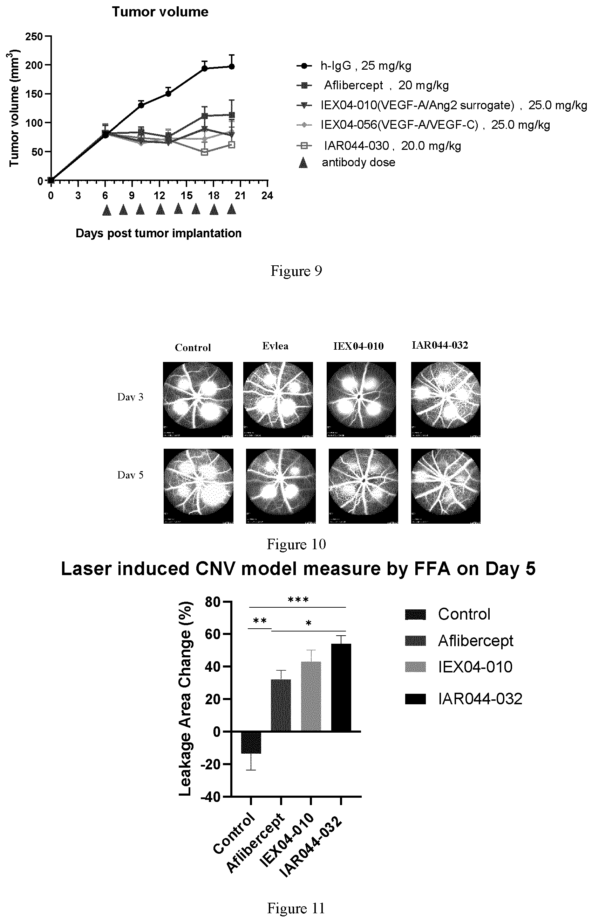

The tumor growth inhibition rate results (as shown in Figure 9 and Table 9) showed: the anti-VEGFA/VEGFC/Ang2 trispecific molecules of the present invention have significant tumor inhibitory effects.

Table 9. Tumor Inhibition Rate on Day 21

| Group | Tumor Volume (mm$^3$) | TGI (%) |
|---|---|---|
| IgG | 209 | / |
| Aflibercept | 114 | 46 |
| IEX04-010 | 78 | 63 |
| IEX04-056 | 85 | 59 |
| IAR044-030 | 62 | 70 |

**Embodiment 11. Pharmacodynamic Test of Laser-induced Choroidal Neovascularization**

In the present experiment, the mouse laser-induced choroidal neovascularization model was used to determine the anti-neovascularization effect of the trispecific fusion proteins of the present invention. The experimental animals were SPF-grade C57 mice, with a body weight of 18-30 g.

In this study, foveal-sparing laser photocoagulation in mice fundus was used to induce fundus choroidal neovascularization and establish an animal model similar to that of human choroidal neovascularization. The mice were grouped into a total of six groups 24 h before photocoagulation, namely the model control group, the Aflibercept group, the IEX04-010 group, the IEX04-056 group, the IAR044-030 group, and the IAR044-032 group, with five mice in each group. Within 24 h after photocoagulation, the doses in Table 10 were administered, the test article was administered via intravitreal injection in both eyes, and the model control group received an equal volume of 0.9% sodium chloride injection. After photocoagulation, color fundus photography and fundus fluorescein angiography were performed on Day 3, Day 5 and Day 10, respectively, to observe the inhibition of choroidal neovascularization by the test article. Both eyes were collected for HE staining histology after euthanasia 10 days after drug administration.

Table 10. Experimental Design Table

| Group | Dose | Dose Volume | Concentration | Mode of Administration |
|---|---|---|---|---|
| Control | / | / | X mg/mL | Intravitreal administration |
| Aflibercept | 40 ug/eye | 1 uL/eye | 40 mg/mL | Intravitreal administration |
| IEX04-010 | 40 ug/eye | 1 uL/eye | 40 mg/mL | Intravitreal administration |
| IAR044-032 | 40 ug/eye | 1 uL/eye | 40 mg/mL | Intravitreal administration |

Color Fundus Photography and Fluorescein Angiography

Evaluation indicators:

(1) Fluorescent spot rating

Grading criteria for spots in fluorescein angiography after modeling:

Grade 1: Spots do not show hyperfluorescence;
Grade 2: Spots are hyperfluorescent but without fluorescein leakage;
Grade 3: Spots are hyperfluorescent, with mild fluorescein leakage, but leakage does not exceed the spot edges;
Grade 4: Spots are hyperfluorescent, with significant fluorescein leakage and leakage beyond the spot edges.

Spots of Grade 1-4 were counted, and the grade of fundus laser spots was recorded for each examination.

(2) Fluorescein leakage area improvement rate

Fluorescein leakage area improvement rate (%) = (fluorescein leakage area before drug administration - fluorescein leakage area after drug administration) /fluorescein leakage area before dug administration * 100%

The results are shown in Figures 10-11. Despite the limited number of test samples, it can be seen that the multi-specific fusion protein of the present invention, IAR044-032, showed a significant trend towards anti-neovascularization after five days of drug administration.

**Embodiment 12. DL-AAA-induced Retinal Neovascularization Test**

In the present experiment, the Dutch rabbit retinal neovascularization model was used to detect the inhibitory effect of the molecules on retinal neovascularization.

*Laboratory Animal Description*

Species and strain: Dutch rabbit
Animal grade: Normal grade
Source of laboratory animal: Pizhou Dongfang Breeding Co., Ltd., Laboratory Animal Production License No.: SCXK (Su) 2017-0002, Laboratory Animal Quality Certificate No.: NO. 202227644, NO. 202259452.
Age at start of drug administration (D1): 8-15 months
Body weight before start of drug administration (D1): 2.46-3.44 kg.
Number of animals: 29 qualified modeled animals (all females, modeled via intravitreal injection of DL-$\alpha$-AAA (DL-$\alpha$-aminoadipic acid) were used, and 36 eyes were enrolled.

*Experimental Method*

Animal grouping and test design

Before drug administration, the animals were evenly divided into groups according to the fluorescein leakage area of the RNV model eye. See Table 11 below for details. Drug administration started on D1.

Table 11. Animal Grouping and Test Design

| Group Number | Test/Control Article | Dose (mg/eye) | Concentration (mg/mL) | Volume (μL/eye) | Number of Eyes | Mode of Administration |
|---|---|---|---|---|---|---|
| 1 | PBS | 0 | 0 | 50 | 6 | Intravitreal administration |
| 2 | Eylea® | 0.1 | 2 | 50 | 6 | Intravitreal administration |
| 3 | IEX04-012 | 0.1 | 2 | 50 | 6 | Intravitreal administration |

(continued)

| Group Number | Test/Control Article | Dose (mg/eye) | Concentration (mg/mL) | Volume (μL/eye) | Number of Eyes | Mode of Administration |
|---|---|---|---|---|---|---|
| 4 | IEX04-056 | 0.1 | 2 | 50 | 6 | Intravitreal administration |
| 5 | Low-dose IAR044-030 | 0.03 | 0.6 | 50 | 6 | Intravitreal administration |
| 6 | High-dose IAR044-030 | 0.1 | 2 | 50 | 6 | Intravitreal administration |
| #: indicates that there are no limitations on the enrolled eye of successfully-modeled animals. | | | | | | |

Red-free image of the fundus

The red-free images of the fundus of the animals were acquired at screening (12 weeks after modeling), before enrollment, on D8, D15, D29, D43, D47 (only animal #2318791), D57, and D71. The examined areas were the temporal, central, and nasal nerve fiber layers, and RNV morphology was observed.

Fundus Fluorescein Angiography (FFA)

FFA examination was performed for all animals at screening (12 weeks after modeling), before enrollment, on D8, D15, D29, D43, D47 (only animal #2318791), D57, and D71. Before the FFA examination, the animals were given 10% fluorescein sodium injection (at a dose of 10 mg/kg and a concentration of 100 mg/mL) via intravenous injection. Early and late FFA images were acquired, with early images within approximately 1.5 min and late images at approximately 1.5-3 min. Whether there was RNV generation and leakage were determined based on whether there was fluorescein leakage in the fundus of animals, the fluorescein leakage area was determined, the amount of reduction in fluorescein leakage area and the fluorescein leakage area improvement rate were calculated:

$$\text{Amount of reduction in fluorescein leakage area} = \text{fluorescein leakage area before drug administration} - \text{fluorescein leakage area after drug administration};$$

$$\text{Fluorescence leakage area improvement rate} = \text{amount of reduction in fluorescein leakage area} / \text{fluorescein leakage area before drug administration} \times 100\%.$$

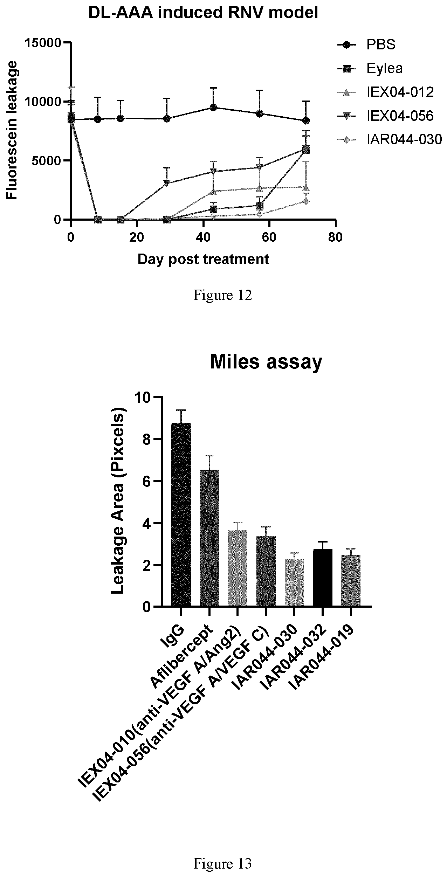

The results showed that (Figure 12) during the test period, Eylea®, IEX04-012, IEX04-056, IAR044-030 administered at 0.1 mg/eye and IAR044-030 administered at 0.03 mg/eye could effectively inhibit fluorescein leakage, the inhibitory effect of Eylea® could last until D57, the inhibitory effect of IEX04-056 could last until D57, and the inhibitory effect of IEX04-012 and low- and high-dose IAR044-030 could last until D71, wherein the efficacy of IEX04-012 and low-dose IAR044-030 was better than that of Eylea®, and the efficacy of low-dose IAR044-030 was better than that of IEX04-012.

**Embodiment 13. Evans Blue Staining of Vascular Leakage in Mice**

Neovascularization may be induced in vivo by subcutaneous injection of VEGFA, Ang2 and VEGFC, promoting leakage. In the present embodiment, the mice were given intravenous injection of evans blue in advance, and 30 min later, the mice were subcutaneously injected with 50 ng of VEGFA + 500 ng of Ang2 + 500 ng of VEGFC to induce the model, wherein there was one point at each locality, three points on each side, and a total of six points in each mouse. At the same time, the following drugs were injected into different groups as shown in Table 12 below, and photographs were taken and the leakage area was calculated 20 min after drug administration.

Table 12. Animal Groups and Test Design

| Group Number | Dose/Point | Mode of Administration |
|---|---|---|
| IgG | 50 $\mu$g | Single, subcutaneous |
| Aflibercept | 40 $\mu$g | Single, subcutaneous |
| IAR044-032 | 40 $\mu$g | Single, subcutaneous |
| IEX04-010 | 50 $\mu$g | Single, subcutaneous |
| IEX04-056 | 50 $\mu$g | Single, subcutaneous |
| IAR044-019 | 60 $\mu$g | Single, subcutaneous |
| IAR044-030 | 40 $\mu$g | Single, subcutaneous |

The results are shown in Figure 13. As shown in the figure, the multi-specific fusion protein molecules (IAR044-030, IAR044-032, and IAR044-019) are capable of significantly inhibiting vascular leakage, with better effects than clinical drugs and bispecific drugs.

**Overview of Sequence Listing**

A sequence listing comprising many nucleic acid and amino acid sequences is appended to the present application. The following table provides an overview of the comprised sequences.

| SEQ ID NO: | Sequence Description | Sequence Information |
|---|---|---|
| | | Anti-VEGFC antibody sequence |
| 1 | Anti-VEGFC HCDR1 | GSSFSPYAMG |
| 2 | Anti-VEGFC HCDR2 | ATSEGGFTLYADSVKG |
| 3 | Anti-VEGFC HCDR3 | HWRGSDPENY |
| 4 | Anti-VEGFC VHH | EVQLVESGGGLVQPGGSLRLSCAASGSSFSPYAMGWYRQAPGKQRELVSATSEGGFTLYADSVKGRFTISRDNSKNTVYLQMNSLRAEDTAVYYCYMHWRGSDPENYWGQGTQVTVSS |
| | | Anti-Ang2 antibody sequence |
| 5 | Anti-Ang2 HCDR1 | GFALDYYAIG |
| 6 | Anti-Ang2 HCDR2 | CISSGEGSTYYADSVKG |
| 7 | Anti-Ang2 HCDR3 | DSRGDDVACEGLRRNEYDY |
| 8 | Anti-Ang2 VHH | EVQLLESGGGLVQPGGSLRLSCAASGFALDYYAIGWFRQAPGKGLEGVSCISSGEGSTYYADSVKGRFTISRDNSKNTVYLQMNSLRAEDTAVYYCATDSRGDDVACEGLRRNEYDYWGQGTLVTVSS |
| | | Anti-VEGFA mini-Trap, human VEGFR1 and VEGFR2 sequences from UniProt P17948 and UniProtP35968 |
| 9 | Mini-Trap containing VEGFR1 domain 2 (aa129-aa230) and VEGR2 domain 3 (aa225-327) | SDTGRPFVEMYSEIPEIIHMTEGRELVIPCRVTSPNITVTLKKFPLDTLIPDGKRIIWDSRKGFIISNATYKEIGLLTCEATVNGHLYKTNYLTHRQTNTIIDVVLSPSHGIELSVGEKLVLNCTARTELNVGIDFNWEYPSSKHQHKKLVNRDLKTQSGSEMKKFLSTLTIDGVTRSDQGLYTCAASSGLMTKKNSTFVRVHEK |
| | | Short linker sequence |
| 10 | Short linker 1 | GGGGGGGGGG |
| 11 | Short linker 2 | GRPGSGRPGS |
| | | Hinge sequence |
| 12 | Hinge_1 | ESKYGPPCPPCPPC |
| 13 | Hinge_2 | EKTHTCPPCPPC |
| 14 | Hinge_3 | EPKSSPPCPPCPAPC |
| 15 | Hinge_4 | DTTHTCPPCPPC |

33

| SEQ ID NO: | Sequence Description | Sequence Information |
|---|---|---|
| | | Anti-VEGFC antibody sequence |
| 16 | Hinge_5 | DKTHTCPPCPPC |
| 17 | Hinge_6 | EPKSCDTPPPCPPCP |
| 18 | Hinge_7 | EPKSSDTPPPCPPCPPC |
| 19 | Hinge_8 | DKTHTCPPCPAPELLG |
| 20 | Linker peptide containing Hinge2 | ESKYGPPCPPCPPCGGGGGGGGGG |
| 21 | Linker peptide containing Hinge3 | EKTHTCPPCPPCGGGGGGGGGG |
| | | Trispecific molecules of the present disclosure |
| 22 | IAR044-019 Ang2_L1_Va_Fc_ L1_Vc | EVQLLESGGGLVQPGGSLRLSCAASGFALDYYAIGWFRQAPGKGLEGVSCISSGEGSTYYA DSVKGRFTISRDNSKNTVYLQMNSLRAEDTAVYYCATDSRGDDVACEGLRRNEYDYWGQ GTLVTVSSGRPGSGRPGSSDTGRPFVEMYSEIPEIIHMTEGRELVIPCRVTSPNITVTLKKFPL DTLIPDGKRIIWDSRKGFIISNATYKEIGLLTCEATVNGHLYKTNYLTHRQTNTIIDVVLSPSH GIELSVGEKLVLNCTARTELNVGIDFNWEYPSSKHQHKKLVNRDLKTQSGSEMKKFLSTLTI DGVTRSDQGLYTCAASSGLMTKKNSTFVRVHEKDKTHTCPPCPAPELLGGPSVFLFPPKPK DTLMISRTPEVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQQGNVFSCSVMHE ALHNHYTQKSLSLSPGGRPGSGRPGSEVQLVESGGGLVQPGGSLRLSCAASGSSFSPYAMG WYRQAPGKQRELVSATSEGGFTLYADSVKGRFTISRDNSKNTVYLQMNSLRAEDTAVYYC YMHWRGSDPENYWGQGTQVTVSS

EVQLLESGGGLVQPGGSLRLSCAASGFALDYYAIGWFRQAPGKGLEGVSCISSGEGSTYYA DSVKGRFTISRDNSKNTVYLQMNSLRAEDTAVYYCATDSRGDDVACEGLRRNEYDYWGQ GTLVTVSSGGGGGGGGGGSDTGRPFVEMYSEIPEIIHMTEGRELVIPCRVTSPNITVTLKKFP LDTLIPDGKRIIWDSRKGFIISNATYKEIGLLTCEATVNGHLYKTNYLTHRQTNTIIDVVLSPS HGIELSVGEKLVLNCTARTELNVGIDFNWEYPSSKHQHKKLVNRDLKTQSGSEMKKFLSTL TIDGVTRSDQGLYTCAASSGLMTKKNSTFVRVHEKEPKSSPPCPPCPAPCGGGGGGGGGGE VQLVESGGGLVQPGGSLRLSCAASGSSFSPYAMGWYRQAPGKQRELVSATSEGGFTLYADS VKGRFTISRDNSKNTVYLQMNSLRAEDTAVYYCYMHWRGSDPENYWGQGTQVTVSS |

EP 4 628 512 A1

EP 4 628 512 A1

| SEQ ID NO: | Sequence Description | Sequence Information |
|---|---|---|
| | | Anti-VEGFC antibody sequence |
| 23 | IAR044-028 Ang2_L1_Va_L2_ Vc | EVQLLESGGGGLVQPGGGSLRLSCAASGFALDYYAIGWFRQAPGKGLEGVSCISSGEGSTYYADSVKGRFTISRDNSKNTVYLQMNSLRAEDTAVYYCATDSRGDDVACEGLRRNEYDYWGQGTLVTVSSGGGGGGGGGGGSDTGRPFVEMYSEIPEIIHMTEGRELVIPCRVTSPNITVTLKKFPLDTLIPDGKRIIWDSRKGFIISNATYKEIGLLTCEATVNGHLYKTNYLTHRQTNTIIDVVLSPSHGIELSVGEKLVLNCTARTELNVGIDFNWEYPSSKHQHKKLVNRDLKTQSGSEMKKFLSTLTIDGVTRSDQGLYTCAASSGLMTKKNSTFVRVHEKEPKSSPPCPPCPAPCGGGGGGGGGGEVQLVESGGGGLVQPGGGSLRLSCAASGSSFSPYAMGWYRQAPGKQRELVSATSEGGFTLYADSVKGRFTISRDNSKNTVYLQMNSLRAEDTAVYYCYMHWRGSDPENYWGQGTQVTVSS |
| 24 | IAR044-030 Ang2_L1_Va_L2_ Vc | EVQLLESGGGGLVQPGGGSLRLSCAASGFALDYYAIGWFRQAPGKGLEGVSCISSGEGSTYYADSVKGRFTISRDNSKNTVYLQMNSLRAEDTAVYYCATDSRGDDVACEGLRRNEYDYWGQGTLVTVSSGGGGGGGGGGGSDTGRPFVEMYSEIPEIIHMTEGRELVIPCRVTSPNITVTLKKFPLDTLIPDGKRIIWDSRKGFIISNATYKEIGLLTCEATVNGHLYKTNYLTHRQTNTIIDVVLSPSHGIELSVGEKLVLNCTARTELNVGIDFNWEYPSSKHQHKKLVNRDLKTQSGSEMKKFLSTLTIDGVTRSDQGLYTCAASSGLMTKKNSTFVRVHEKESKYGPPCPPCPPCGGGGGGGGGGGEVQLVESGGGGLVQPGGGSLRLSCAASGSSFSPYAMGWYRQAPGKQRELVSATSEGGFTLYADSVKGRFTISRDNSKNTVYLQMNSLRAEDTAVYYCYMHWRGSDPENYWGQGTQVTVSS |
| 25 | IAR044-032 Ang2_L1_Va_L2_ Vc | EVQLLESGGGGLVQPGGGSLRLSCAASGFALDYYAIGWFRQAPGKGLEGVSCISSGEGSTYYADSVKGRFTISRDNSKNTVYLQMNSLRAEDTAVYYCATDSRGDDVACEGLRRNEYDYWGQGTLVTVSSGGGGGGGGGGGSDTGRPFVEMYSEIPEIIHMTEGRELVIPCRVTSPNITVTLKKFPLDTLIPDGKRIIWDSRKGFIISNATYKEIGLLTCEATVNGHLYKTNYLTHRQTNTIIDVVLSPSHGIELSVGEKLVLNCTARTELNVGIDFNWEYPSSKHQHKKLVNRDLKTQSGSEMKKFLSTLTIDGVTRSDQGLYTCAASSGLMTKKNSTFVRVHEKEKTHTCPPCPPCGGGGGGGGGGGEVQLVESGGGGLVQPGGGSLRLSCAASGSSFSPYAMGWYRQAPGKQRELVSATSEGGFTLYADSVKGRFTISRDNSKNTVYLQMNSLRAEDTAVYYCYMHWRGSDPENYWGQGTQVTVSS |
| 26 | IAR044-034 Ang2_L1_Va_L2_ Vc | EVQLLESGGGGLVQPGGGSLRLSCAASGFALDYYAIGWFRQAPGKGLEGVSCISSGEGSTYYADSVKGRFTISRDNSKNTVYLQMNSLRAEDTAVYYCATDSRGDDVACEGLRRNEYDYWGQGTLVTVSSGGGGGGGGGGGSDTGRPFVEMYSEIPEIIHMTEGRELVIPCRVTSPNITVTLKKFPLDTLIPDGKRIIWDSRKGFIISNATYKEIGLLTCEATVNGHLYKTNYLTHRQTNTIIDVVLSPSHGIELSVGEKLVLNCTARTELNVGIDFNWEYPSSKHQHKKLVNRDLKTQSGSEMKKFLSTLTIDGVTRSDQGLYTCAASSGLMTKKNSTFVRVHEKDTTHTCPPCPPCGGGGGGGGGGGEVQLVESGGGGLVQPGGGSLRLSCAASGSSFSPYAMGWYRQAPGKQRELVSATSEGGFTLYADSVKGRFTISRDNSKNTVYLQMNSLRAEDTAVYYCYMHWRGSDPENYWGQGTQVTVSS |

(continued)

| SEQ ID NO: | Sequence Description | Sequence Information |
|---|---|---|
| | | Anti-VEGFC antibody sequence |
| 27 | IAR044-035 Ang2_L1_Va_L2_Vc | EVQLLESGGGLVQPGGSLRLSCAASGFALDYYAIGWFRQAPGKGLEGVSCISSGEGSTYYADSVKGRFTISRDNSKNTVYLQMNSLRAEDTAVYYCATDSRGDDVACEGLRRNEYDYWGQGTLVTVSSGGGGSGGGGSGGGGSDTGRPFVEMYSEIPEIIHMTEGRELVIPCRVTSPNITVTLKKFPLDTLIPDGKRIIWDSRKGFIISNATYKEIGLLTCEATVNGHLYKTNYLTHRQTNTIIDVVLSPSHGIELSVGEKLVLNCTARTELNVGIDFNWEYPSSKHQHKKLVNRDLKTQSGSEMKKFLSTLTIDGVTRSDQGLYTCAASSGLMTKKNSTFVRVHEKEPKSCDIPPPCPPCPPGGGGGGGGGEVQLVESGGGLVQPGGSLRLSCAASGSSFSPYAMGWYRQAPGKQRELVSATSEGGFTLYADSVKGRFTISRDNSKNTVYLQMNSLRAEDTAVYYCYMHWRGSDPENYWGQGTQVTVSS |
| 28 | IAR044-036 Ang2_L1_Va_L2_Vc | EVQLLESGGGLVQPGGSLRLSCAASGFALDYYAIGWFRQAPGKGLEGVSCISSGEGSTYYADSVKGRFTISRDNSKNTVYLQMNSLRAEDTAVYYCATDSRGDDVACEGLRRNEYDYWGQGTLVTVSSGGGGSGGGGSGGGGSDTGRPFVEMYSEIPEIIHMTEGRELVIPCRVTSPNITVTLKKFPLDTLIPDGKRIIWDSRKGFIISNATYKEIGLLTCEATVNGHLYKTNYLTHRQTNTIIDVVLSPSHGIELSVGEKLVLNCTARTELNVGIDFNWEYPSSKHQHKKLVNRDLKTQSGSEMKKFLSTLTIDGVTRSDQGLYTCAASSGLMTKKNSTFVRVHEKEPKSSDTPPPCPPCPPGGGGGGGGGGEVQLVESGGGLVQPGGSLRLSCAASGSSFSPYAMGWYRQAPGKQRELVSATSEGGFTLYADSVKGRFTISRDNSKNTVYLQMNSLRAEDTAVYYCYMHWRGSDPENYWGQGTQVTVSS |
| 29 | IAR044-038 Va_L2_Ang2_L1_Vc | SDTGRPFVEMYSEIPEIIHMTEGRELVIPCRVTSPNITVTLKKFPLDTLIPDGKRIIWDSRKGFIISNATYKEIGLLTCEATVNGHLYKTNYLTHRQTNTIIDVVLSPSHGIELSVGEKLVLNCTARTELNVGIDFNWEYPSSKHQHKKLVNRDLKTQSGSEMKKFLSTLTIDGVTRSDQGLYTCAASSGLMTKKNSTFVRVHEKEPKSSPPCPPCPPAPCGGGGGGGGSGGGGSCISSGEGSTYYADSVKGRFTISRDNSKNTVYLQCAASGFALDYYAIGWFRQAPGKGLEGVSCISSGEGSTYYADSVKGRFTISRDNSKNTVYLQMNSLRAEDTAVYYCATDSRGDDVACEGLRRNEYDYWGQGTLVTVSSGGGGSGGGGGGEVQLVESGGGLVQPGGSLRLSCAASGSSFSPYAMGWYRQAPGKQRELVSATSEGGFTLYADSVKGRFTISRDNSKNTVYLQMNSLRAEDTAVYYCYMHWRGSDPENYWGQGTQVTVSS |
| 30 | IAR044-039 Va_L2_Ang2_L1_Vc | SDTGRPFVEMYSEIPEIIHMTEGRELVIPCRVTSPNITVTLKKFPLDTLIPDGKRIIWDSRKGFIISNATYKEIGLLTCEATVNGHLYKTNYLTHRQTNTIIDVVLSPSHGIELSVGEKLVLNCTARTELNVGIDFNWEYPSSKHQHKKLVNRDLKTQSGSEMKKFLSTLTIDGVTRSDQGLYTCAASSGLMTKKNSTFVRVHEKESKYGPPCPPCPPCGGGGGGGGGGGGEVQLLESGGGLVQPGGSLRLSCAASGFALDYYAIGWFRQAPGKGLEGVSCISSGEGSTYYADSVKGRFTISRDNSKNTVYLQMNSLRAEDTAVYYCATDSRGDDVACEGLRRNEYDYWGQGTLVTVSSGGGGGGGGGEVQLVESGGGLVQPGGSLRLSCAASGSSFSPYAMGWYRQAPGKQRELVSATSEGGFTLYADSVKGRFTISRDNSKNTVYLQMNSLRAEDTAVYYCYMHWRGSDPENYWGQGTQVTVSS |

| SEQ ID NO: | Sequence Description | Sequence Information |
|---|---|---|
| | | Anti-VEGFC antibody sequence |
| 31 | IAR044-040 Va_L2_Ang2_L1_ Vc | SDTGRPFVEMYSEIPEIIHMTEGRELVIPCRVTSPNITVTLKKFPLDTLIPDGKRIIWDSRKGFII SNATYKEIGLLTCEATVNGHLYKTNYLTHRQTNTIIDVVLSPSHGIELSVGEKLVLNCTARTE LNVGIDFNWEYPSSKHQHKKLVNRDLKTQSGSEMKKFLSTLTIDGVTRSDQGLYTCAASSG LMTKKNSTFVRVHEKEKTHTCPPCPPCGGGGGGGGGGGEVQLLESGGGLVQPGGSLRLSCA ASGFALDYYAIGWFRQAPGKGLEGVSCISSGEGSTYYADSVKGRFTISRDNSKNTVYLQMN SLRAEDTAVYYCATDSRGDDVACEGLRRNEYDYWGQGTLVTVSSGGGGGGGGGGGEVQLV ESGGGLVQPGGSLRLSCAASGSSFSPYAMGWYRQAPGKQRELVSATSEGGFTLYADSVKG RFTISRDNSKNTVYLQMNSLRAEDTAVYYCYMHWRGSDPENYWGQGTQVTVSS |
| 32 | IAR044-022m or - 22b Va_L2_Ang2 _L1_Vc wherein 022m represents a monomeric polypeptide; 022b represents a dimeric polypeptide. | SDTGRPFVEMYSEIPEIIHMTEGRELVIPCRVTSPNITVTLKKFPLDTLIPDGKRIIWDSRKGFII SNATYKEIGLLTCEATVNGHLYKTNYLTHRQTNTIIDVVLSPSHGIELSVGEKLVLNCTARTE LNVGIDFNWEYPSSKHQHKKLVNRDLKTQSGSEMKKFLSTLTIDGVTRSDQGLYTCAASSG LMTKKNSTFVRVHEKDKTHTCPPCPAPELLGGRPGSGRPGSEVQLLESGGGLVQPGGSLRLS CAASGFALDYYAIGWFRQAPGKGLEGVSCISSGEGSTYYADSVKGRFTISRDNSKNTVYLQ MNSLRAEDTAVYYCATDSRGDDVACEGLRRNEYDYWGQGTLVTVSSGRPGSGRPGSEVQ LVESGGGLVQPGGSLRLSCAASGSSFSPYAMGWYRQAPGKQRELVSATSEGGFTLYADSVK GRFTISRDNSKNTVYLQMNSLRAEDTAVYYCYMHWRGSDPENYWGQGTQVTVSS |
| 33 | IAR044-029 Ang2_L1_Va_L1_ Vc-hinge | EVQLLESGGGLVQPGGSLRLSCAASGFALDYYAIGWFRQAPGKGLEGVSCISSGEGSTYYA DSVKGRFTISRDNSKNTVYLQMNSLRAEDTAVYYCATDSRGDDVACEGLRRNEYDYWGQ GTLVTVSSGGGGGGGGGGGSDTGRPFVEMYSEIPEIIHMTEGRELVIPCRVTSPNITVTLKKFP LDTLIPDGKRIIWDSRKGFIISNATYKEIGLLTCEATVNGHLYKTNYLTHRQTNTIIDVVLSPS HGIELSVGEKLVLNCTARTELNVGIDFNWEYPSSKHQHKKLVNRDLKTQSGSEMKKFLSTL TIDGVTRSDQGLYTCAASSGLMTKKNSTFVRVHEKGGGGGGGGGGEVQLVESGGGLVQPG GSLRLSCAASGSSFSPYAMGWYRQAPGKQRELVSATSEGGFTLYADSVKGRFTISRDNSKN TVYLQMNSLRAEDTAVYYCYMHWRGSDPENYWGQGTQVTVSSDKTHTCPPCPPC |
| 34 | IAR044-033 Ang2_L1_Va_L1_ Vc-hinge | EVQLLESGGGLVQPGGSLRLSCAASGFALDYYAIGWFRQAPGKGLEGVSCISSGEGSTYYAD SVKGRFTISRDNSKNTVYLQMNSLRAEDTAVYYCATDSRGDDVACEGLRRNEYDYWGQGT LVTVSSGRPGSGRPGSSDTGRPFVEMYSEIPEIIHMTEGRELVIPCRVTSPNITVTLKKFPLDTLI PDGKRIIWDSRKGFIISNATYKEIGLLTCEATVNGHLYKTNYLTHRQTNTIIDVVLSPSHGIELS VGEKLVLNCTARTELNVGIDFNWEYPSSKHQHKKLVNRDLKTQSGSEMKKFLSTLTIDGVTR SDQGLYTCAASSGLMTKKNSTFVRVHEKGRPGSGRPGSEVQLVESGGGLVQPGGSLRLSCA ASGSSFSPYAMGWYRQAPGKQRELVSATSEGGFTLYADSVKGRFTISRDNSKNTVYLQMNS LRAEDTAVYYCYMHWRGSDPENYWGQGTQVTVSSDKTHTCPPCPPC |
| | | Fc region sequence |

(continued)

| SEQ ID NO: | Sequence Description | Sequence Information |
|---|---|---|
| | | Anti-VEGFC antibody sequence |
| 35 | Fc region of human IgG1 | DKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG |
| | | Positive control VEGFA Trap molecule Aflibercept |
| 36 | Positive control Aflibercept | SDTGRPFVEMYSEIPEIIHMTEGRELVIPCRVTSPNITVTLKKFPLDTLIPDGKRIIWDSRKGFIISNATYKEIGLLTCEATVNGHLYKTNYLTHRQTNTIIDVVLSPSHGIELSVGEKLVLNCTARTELNVGIDFNWEYPSSKHQHKKLVNRDLKTQSGSEMKFLSTLTIDGVTRSDQGLYTCAASSGLMTKKNSTFVRVHEKDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG |
| | | Sequence of positive control anti-VEGFA/Ang2 antibody Faricimab |
| 37 | First heavy chain variable region VH1 of positive control antibody Faricimab | EVQLVESGGGLVQPGGSLRLSCAASGYDFTHYGMNWVRQAPGKGLEWVGWINTYTGEPTYAADFKRRFTFSLDTSKSTAYLQMNSLRAEDTAVYYCAKYPYYYGTSHWYFDVWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMASRTPEVTCVVVDVSHEDPEVKFNWYVDGVEVHNAKTKPREEQYNSTYRVVSVLTVLAQDWLNGKEYKCKVSNKALGAPIEKTISKAKGQPREPQVYTLPPCRDELTKNQVSLWCLVKGFYPSDIAVEWESNGQPENNYKTTPPVLDSDGSFFLYSKLTVDKSRWQQGNVFSCSVMHEALHNAYTQKSLSLSPGK |
| 38 | Second heavy chain variable region VH2 of positive control antibody Faricimab | QVQLVQSGAEVKKPGASVKVSCKASGYTFTGYYMHWVRQAPGQGLEWMGWINPNSGGTNYAQKFQGRVTMTRDTSISTAYMELSRLRSDDTAVYYCARSPNPYYYDSSGYYPGAFDIWGQGTMVTVSSASVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECDKTHTCPPCPAPEAAGGPSVFLFPPKPKDTLMASRTPEVTCVVVDVSHEDPEVKFNWYVDGVEVHNAKTKPREEQYNSTYRVVSVLTVLAQDWLNGKEYKCKVSNKALGAPIEKTISKAKGQPREPQVCTLPPSRDELTKNQVSLSCAVKGFYPSDIAVEWESNGQPENNYKTTPPVLDSDGSFFLVSKLTVDKSRWQQGNVFSCSVMHEALHNAYTQKSLSLSPGK |
| 39 | First light chain | DIQLTQSPSSLSASVGDRVTITCSASQDISNYLNWYQQKPGKAPKVLIYFTSSLHSGVPSRFSG |

38

(continued)

| SEQ ID NO: | Sequence Description | Sequence Information |
|---|---|---|
| | | Anti-VEGFC antibody sequence |
| | variable region VL1 of positive control antibody Faricimab | SGSGTDFTLTISSLQPEDFATYYCQQYSTVPWTFGQGTKVEIKRTVAAPSVFIFPPSDEQLKSG TASVVCLLNNFYPREAKVQWKVDNALQSGNSQESVTEQDSKDSTYSLSSTLTLSKADYEKH KVYACEVTHQGLSSPVTKSFN RGEC |
| 40 | Second light chain variable region VL2 of positive control anti-body Faricimab | SYVLTQPPSVSVAPGQTARITCGGNNIGSKSVHWYQQKPGQAPVLVVYDDSDRPSGIPERFS GSNSGNTATLTISRVEAGDEADYYCQVWDSSSDHWVFGGGTKLTVLSSASTKGPSVFPLAPS SKSTSGGTAALGCLVKDYFPEPVTVSWNSGALTSGVHTFPAVLQSSGLYSLSSVVTVPSSSLG TQTYICNVNHKPSNTKVDKKVEPKSC |
| | | Positive control VEGFC Trap molecule OPT-302 |
| 41 | Positive control OPT-302 | YSMTPPTLNITEESHVIDTGDSLSISCRGQHPLEWAWPGAQEAPATGDKDSEDTGVVRDCEG TDARPYCKVLLLHEVHAQDTGSYVCYYKYIKARIEGTTAASSYVFVRDFEQPFINKPDTLLV NRKDAMWVPCLVSIPGLNVTLRSQSSVLWPDGQEVVWDDRRGMLVSTPLLHDALYLQCET TWGDQDFLSNPFLVHITGNELYDIQLLPRKSLELLVGEKLVLNCTVWAEFNSGVTFDWDYPG KQAERGKWVPERRSQQTHTELSSILTIHNVSQHDLGSYVCKANNGIQRFRESTEVIVHEEPKS CDKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNYKTTPPVLDSDGSFF LYSKLTVDKSRWQQGNVFSCSVMHEALHNHYTQKSLSLSPGK |
| | | Positive control anti-VEGFA/Ang2 molecule, consisting of anti-VEGFA scFv + linker + anti-Ang2 VHH |
| 42 | Positive control IEX04-012 molecule | DIQLTQSPSSLSASVGDRVTITCSASQDISNYLNWYQQKPGKAPKVLIYFTSSLHSGVPSRFS GSGSGTDFTLTISSLQPEDFATYYCQQYSTVPWTFGCGTKVEIKGGGGSGGGGSGGGGSGG GGSEVQLVESGGGLVQPGGSLRLSCAASGYDFTHYGMNWVRQAPGKCLEWVGWINTYTG EPTYAADFKRRFTFSLDTSKSTAYLQMNSLRAEDTAVYYCAKYPYYYGTSHWYFDVWGQ GTLVTVSS*GGGGSGGGGSGGGGS*_EVQLLESGGGLVQPGGSLRLSCAASGFALDYYAIGWFRQA PGKGLEGVSCISSGEGSTYYADSVKGRFTISRDNSKNTVYLQMNSLRAEDTAVYYCATDSRGDDVA CEGLRRNEYDYWGQGTLVTVSS_ |
| | | Positive control anti-VEGFA/Ang2 molecule, consisting of VEGFA Trap + linker + anti-Ang2 VHH |

39

EP 4 628 512 A1

(continued)

| SEQ ID NO: | Sequence Description | Sequence Information |
|---|---|---|
| | | Anti-VEGFC antibody sequence |
| 43 | Positive control IEX04-010 molecule | SDTGRPFVEMYSEIPEIIHMTEGRELVIPCRVTSPNITVTLKKFPLDTLIPDGKRIIWDSRKGFIISNATYKEIGLLTCEATVNGHLYKTNYLTHRQTNTIIDVVLSPSHGIELSVGEKLVLNCTARTELNVGIDFNWEYPSSKHQHKKLVNRDLKTQSGSEMKKFLSTLTIDGVTRSDQGLYTCAASSGLMTKKNSTFVRVHEKDKTHTCPPCPAPELLGGPSVFLFPPKPKDTLMISRTPEVTCVVVDVSHEDPEVKFKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGGGGGSGGGGSGGGGS*EVQLLESGGGLVQPGGSLRLSCAASGFALDYYAIGWFRQAPGKGLEGVSCISSGEGSTYYADSVKGRFTISRDNSKNTVYLQMNSLRAEDTAVYYCAITDSRGDDVACEGLRRNEYDYWGQGTLVTVSS* |
| | | Positive control anti-VEGFA/VEGFC molecule, consisting of VEGFA Trap + Fc + linker + anti-VEGFC VHH |
| 44 | Positive control molecule IEX04-056 | SDTGRPFVEMYSEIPEIIHMTEGRELVIPCRVTSPNITVTLKKFPLDTLIPDGKRIIWDSRKGFIISNATYKEIGLLTCEATVNGHLYKTNYLTHRQTNTIIDVVLSPSHGIELSVGEKLVLNCTARTELNVGIDFNWEYPSSKHQHKKLVNRDLKTQSGSEMKKFLSTLTIDGVTRSDQGLYTCAASSGLMTKKNSTFVRVHEKDKTHTCPPCPAPELLGGPSVFLFPPKPKDTLMISRTPEVTCVVVDVSHEDPEVKFKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGGGGGGSGGGGSGGGGS*EVQLVESGGGLVQPGGSLRLSCAASGSFSPYAMGWYRQAPGKQRELVSATSSGGFTLYADSVKGRFTISRDNSKNTVYLQMNSLRAEDTAVYYCYTYREYDPEMYWGQGTQVTVSS* |
| | | Other hinge sequences of the present disclosure |
| 45 | Hinge_9 | EPKSSPPCPPCPAPCLLG |
| 46 | Hinge_10 | DKTHTCPPCPAPCLLG |
| | | CH3 region sequence |
| 47 | CH3 region of human IgG1 | GQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPG |
| | | Other trispecific molecules of the present disclosure |
| 48 | IAR044-026 | EVQLLESGGGLVQPGGSLRLSCAASGFALDYYAIGWFRQAPGKGLEGVSCISSGEGSTYYADSVKGRFTISRDNSKNTVYLQMNSLRAEDTAVYYCAITDSRGDDVACEGLRRNEYDYWGQGTLVTVSSGGGGSGGGGSGGGGSDTGRPFVEMYSEIPEIIHMTEGRELVIPCRVTSPNITVTLKKFPLDT |

40

| SEQ ID NO: | Sequence Description | Sequence Information |
|---|---|---|
| | | Anti-VEGFC antibody sequence |
| | Ang2_L1_Va_L2_ Vc | LIPDGKRIIWDSRKGFIISNATYKEIGLLTCEATVNGHLYKTNYLTHRQTNTIIDVVLSPSHGIE LSVGEKLVLNCTARTELNVGIDFNWEYPSSKHQHKKLVNRDLKTQSGSEMKKFLSTLTIDGV TRSDQGLYTCAASSGLMTKKNSTFVRVHEKEPKSSPPCPPCPAPCLLGGGGGGGGGGGGEVQ LVESGGGLVQPGGSLRLSCAASGSSFSPYAMGWYRQAPGKQRELVSATSEGGFTLYADSVKG RFTISRDNSKNTVYLQMNSLRAEDTAVYYCYMHWRGSDPENYWGQGTQVTVSS |
| 49 | IAR044-027 Ang2_L1_Va_L2_ CH3_Vc | EVQLLESGGGLVQPGGSLRLSCAASGFALDYYAIGWFRQAPGKGLEGVSCISSGEGSTYYAD SVKGRFTISRDNSKNTVYLQMNSLRAEDTAVYYCATDSRGDDVACEGLRRNEYDYWGQGT LVTVSSGGGGGGGGGGGSDTGRPFVEMYSEIPEIIHMTEGRELVIPCRVTSPNITVTLKKFPLDT LIPDGKRIIWDSRKGFIISNATYKEIGLLTCEATVNGHLYKTNYLTHRQTNTIIDVVLSPSHGIE LSVGEKLVLNCTARTELNVGIDFNWEYPSSKHQHKKLVNRDLKTQSGSEMKKFLSTLTIDGV TRSDQGLYTCAASSGLMTKKNSTFVRVHEKDKTHTCPPCPAPELLGGQPREPQVYTLPPSRD ELTKNQVSLTCLVKGFYPSDIAVEWESNGQPENNYKTTPPVLDSDGSFFLYSKLTVDKSRWQ QGNVFSCSVMHEALHNHYTQKSLSLSPGGGGGGGGGGGGGEVQLVESGGGLVQPGGSLRLSC AASGSSFSPYAMGWYRQAPGKQRELVSATSEGGFTLYADSVKGRFTISRDNSKNTVYLQMN SLRAEDTAVYYCYMHWRGSDPENYWGQGTQVTVSS |
| 50 | IAR044-031 Ang2_L1_Va_L2_ Vc | EVQLLESGGGLVQPGGSLRLSCAASGFALDYYAIGWFRQAPGKGLEGVSCISSGEGSTYYAD SVKGRFTISRDNSKNTVYLQMNSLRAEDTAVYYCATDSRGDDVACEGLRRNEYDYWGQGT LVTVSSGGGGGGGGGGGSDTGRPFVEMYSEIPEIIHMTEGRELVIPCRVTSPNITVTLKKFPLDT LIPDGKRIIWDSRKGFIISNATYKEIGLLTCEATVNGHLYKTNYLTHRQTNTIIDVVLSPSHGIE LSVGEKLVLNCTARTELNVGIDFNWEYPSSKHQHKKLVNRDLKTQSGSEMKKFLSTLTIDGV TRSDQGLYTCAASSGLMTKKNSTFVRVHEKDKTHTCPPCPAPCLLGGGGGGGGGGGGEVQL VESGGGLVQPGGSLRLSCAASGSSFSPYAMGWYRQAPGKQRELVSATSEGGFTLYADSVKG RFTISRDNSKNTVYLQMNSLRAEDTAVYYCYMHWRGSDPENYWGQGTQVTVSS |
| | | Other hinges |
| 51 | Hinge derived from human IgG1 | DKTHTCPPCPPCPPC |
| 52 | Hinge derived from human IgG2 | ERKCCVECPPC |
| 53 | Hinge derived from human IgG3 | EPKSCDTPPPCPRCP |
| 54 | Hinge derived from equine IgG6 | EPCCCPKCP |
| | | Other sequences |
| 55 | Linker Peptide | (G4S)n, wherein n is an integer equal to or greater than 1 |
| 56 | Linker Peptide | TS(G4S)n, wherein n is an integer equal to or greater than 1 |

| SEQ ID NO: | Sequence Description | Sequence Information |
|---|---|---|
| | | Anti-VEGFC antibody sequence |
| 57 | Linker Peptide | G(G4S)n, wherein n is an integer equal to or greater than 1 |
| 58 | Linker Peptide | (GGGGG)n, wherein n is an integer equal to or greater than 2 |
| 59 | Linker Peptide | (GRPGS)n, wherein n is an integer equal to or greater than 2 |
| 60 | Hinge sequence | CPPC |
| 61 | Hinge sequence | CPPCPPC |
| 62 | Linker Peptide | (G4S)2 |
| 63 | Linker Peptide | (G4S)3 |
| 64 | Linker Peptide | EPKSSPPCPPCPAPCLLG(GGGGG)2 |
| 65 | Linker peptide having the CH3 region at the C-terminus | DKTHTCPPCPAPELLGGQPREPQVYTLPPSRDELTKNQVSLTCLVKGFYPSDIAVEWESNGQP ENNYKTTPPVLDSDGSFFLYSKLTVDKSRWQQGNVFSCSVMHEALHNHYTQKSLSLSPG |
| 66 | Linker Peptide | EPKSSPPCPPCPAPC(GGGGG)2 |
| 67 | Linker Peptide | DKTHTCPPCPAPCLLG(GGGGG)2 |
| 68 | Linker Peptide | DTTHTCPPCPPC(GGGGG)2 |
| 69 | Linker Peptide | EPKSCDTPPPCPPCP(GGGGG)2 |
| 70 | Linker Peptide | EPKSSDTPPPCPPCPPC(GGGGG)2 |

**Claims**

1. A trispecific fusion protein, comprising:

   a polypeptide chain comprising (i) a VEGFC-binding domain; (ii) an Ang2-binding domain and (iii) a VEGFA-binding domain,
   wherein the VEGFC-binding domain and the Ang2-binding domain comprise or consist of a VHH domain that specifically binds to VEGFC or Ang2, respectively, and
   wherein the VEGFA-binding domain comprises or consists of a ligand capture domain that specifically binds to VEGFA, wherein the ligand capture domain comprises an extracellular domain from a VEGF receptor (VEGFR), preferably, the polypeptide also comprises (iv) a dimerization domain selected from a hinge region, a CH3 region, and an Fc region.

2. The fusion protein according to claim 1, wherein the polypeptide chain of the fusion protein comprises a hinge region, and wherein the hinge region comprises the CPPC (SEQ ID NO: 60) or CPPCPPC (SEQ ID NO: 61) amino acid sequence, preferably the hinge region comprising an amino acid sequence selected from SEQ ID NOs: 12-19 and SEQ ID NOs: 45-46, more preferably the amino acid sequence of SEQ ID NO: 12 or 13.

3. The fusion protein according to claims 1-2, wherein the VEGFC-binding domain comprises a CDR1-3 sequence contained in SEQ ID NO:4, preferably a CDR1 sequence comprising SEQ ID NO: 1, or consisting thereof; a CDR2 sequence comprising SEQ ID NO:2, or consisting thereof; and a CDR3 sequence comprising SEQ ID NO:3, or consisting thereof;

   further preferably, the VEGFC-binding domain comprising the amino acid sequence of SEQ ID NO: 4, or an amino acid sequence having at least 85%, 90%, 95% or 99% identity thereto, or an amino acid sequence having one or more (preferably 1-10, more preferably 1-5) amino acid additions, deletions and/or substitutions compared to SEQ ID NO: 4,
   most preferably, the VEGFC-binding domain comprising the amino acid sequence of SEQ ID NO: 4 or consisting of the amino acid sequence shown in SEQ ID NO: 4.

4. The fusion protein according to claims 1-3, wherein the Ang2-binding domain comprises a CDR1-3 sequence contained in SEQ ID NO: 8, preferably a CDR1 sequence comprising SEQ ID NO: 5, or consisting thereof; a CDR2 sequence comprising SEQ ID NO: 6, or consisting thereof; and a CDR3 sequence comprising SEQ ID NO: 7, or consisting thereof;

   further preferably, the Ang2-binding domain comprising SEQ ID NO: 8, or an amino acid sequence having at least 85%, 90%, 95% or 99% identity thereto, or an amino acid sequence having one or more (preferably 1-10, more preferably 1-5) amino acid additions, deletions and/or substitutions compared to SEQ ID NO: 8,
   most preferably, the Ang2-binding domain comprising the amino acid sequence of SEQ ID NO: 8 or consisting of the amino acid sequence shown in SEQ ID NO: 8.

5. The fusion protein according to claims 1-4, wherein the VEGFA-binding domain comprises immunoglobulin-like (Ig) domain 2 of VEGFR-1 and Ig-like domain 3 of VEGFR-2, and preferably comprises the amino acid sequence of SEQ ID NO: 9 or an amino acid sequence having at least 85%, 90%, 95% or 99% identity thereto or an amino acid sequence having one or more (preferably 1-10, more preferably 1-5) amino acid additions, deletions and/or substitutions compared to SEQ ID NO: 9,
   most preferably, the VEGFA-binding domain comprises the amino acid sequence shown in SEQ ID NO: 9, or is composed of the amino acid sequence shown in SEQ ID NO: 9.

6. The fusion protein according to claims 1-5, wherein the polypeptide chain of the fusion protein comprises, from the N-terminus to the C-terminus:

   (i) an Ang2-binding domain, a first linker peptide, a VEGFA-binding domain, a hinge region, a second linker peptide, and a VEGFC-binding domain,
   (ii) a VEGFC-binding domain, a first linker peptide, a VEGFA-binding domain, a hinge region, a second linker peptide, and an Ang2-binding domain,
   (iii) a VEGFA-binding domain, a hinge region, a first linker peptide, an Ang2-binding domain, a second linker peptide, and a VEGFC-binding domain,

(iv) a VEGFA-binding domain, a hinge region, a first linker peptide, a VEGFC-binding domain, a second linker peptide, and an Ang2-binding domain,

(v) an Ang2-binding domain, a first linker peptide, a VEGFC-binding domain, a second linker peptide, a VEGFA-binding domain, and a hinge region, or

(vi) a VEGFC-binding domain, a first linker peptide, an Ang2-binding domain, a second linker peptide, a VEGFA-binding domain, and a hinge region;

or, wherein the polypeptide chain of the fusion protein comprises, from the N-terminus to the C-terminus:

(i) an Ang2-binding domain, a first linker peptide, a VEGFA-binding domain, an Fc region or CH3 region, a second linker peptide, and a VEGFC-binding domain,

(ii) a VEGFC-binding domain, a first linker peptide, a VEGFA-binding domain, an Fc region or CH3 region, a second linker peptide, and an Ang2-binding domain,

(iii) a VEGFA-binding domain, an Fc region or a CH3 region, a first linker peptide, an Ang2-binding domain, a second linker peptide, and a VEGFC-binding domain,

(iv) a VEGFA-binding domain, an Fc region or a CH3 region, a first linker peptide, a VEGFC-binding domain, a second linker peptide, and an Ang2-binding domain,

(v) an Ang2-binding domain, a first linker peptide, a VEGFC-binding domain, a second linker peptide, a VEGFA-binding domain, and an Fc region or CH3 region, or

(vi) a VEGFC-binding domain, a first linker peptide, an Ang2-binding domain, a second linker peptide, a VEGFA-binding domain, and an Fc region or CH3 region,

and optionally, the polypeptide chain of the fusion protein further comprising a hinge region linked to the N-terminus of the Fc region or the CH3 region.

7. The fusion protein according to claim 6, wherein:

(i) the hinge region comprises an amino acid sequence selected from SEQ ID NOs: 12-19 and 45-46, more preferably the amino acid sequence of SEQ ID NO: 12 or 13;

(ii) the first and second linker peptides comprise a length of 5-12 amino acids, preferably comprising the amino acid sequence (GRPGS)n (SEQ ID NO: 59) or (GGGGG)n (SEQ ID NO: 58), wherein n = 2 or 3;

(iii) the Fc region is the Fc region from a human IgG, more preferably the Fc region comprising the amino acid sequence of SEQ ID NO: 35 or an amino acid sequence having at least 85%, 90%, 95% or 99% identity thereto;

(iv) the CH3 region is the Fc region from a human IgG, more preferably the CH3 region comprising the amino acid sequence of SEQ ID NO: 47 or an amino acid sequence having at least 85%, 90%, 95% or 99% identity thereto.

8. The fusion protein according to claims 1-7, wherein the polypeptide chain comprises:

(i) an amino acid sequence selected from SEQ ID NOs: 22-34 and 48-50; or

(ii) an amino acid sequence having at least 80%, 85%, 90%, 91%, 92%, 93%, 94%, 95%, 96%, 97%, 98%, 99% or higher identity to the amino acid sequence of (i); or

(iii) an amino acid sequence having at least 1-30, or 1-20, or 1-15, or 1-10, or 1-5 amino acid changes (e.g., substitutions, deletions and/or insertions, preferably substitutions, more preferably conservative substitutions) relative to the amino acid sequence of (i).

9. The fusion protein according to claims 1-8, wherein the polypeptide chain comprises the amino acid sequence of SEQ ID NO: 24 or 25, or consists thereof.

10. The fusion protein according to claims 1-9, which is a dimeric protein comprising two identical polypeptide chains.

11. A polynucleotide encoding the fusion protein according to claims 1-10.

12. An expression vector comprising the polynucleotide according to claim 11.

13. A host cell transfected with the vector according to claim 12.

14. A method of producing the fusion protein according to claims 1-10, comprising culturing the host cell according to claim 13, and recovering the produced fusion protein.

15. A pharmaceutical composition comprising the fusion protein according to claims 1-10 and a pharmaceutically acceptable carrier.

16. A method of treating a neovascularization-related disease, comprising administering to a subject the fusion protein according to claims 1-10 or a pharmaceutical composition thereof.

17. The method according to claim 16, wherein the disease is a solid tumor, wherein the administration of the fusion protein inhibits neovascularization within the tumor and/or growth of the tumor.

18. The method according to claim 16, wherein the disease is an ocular disease, such as choroidal neovascularization or retinal neovascularization.

19. A use of the fusion protein according to claims 1-10 as a medicine, as a medicine for treating a disease or as a diagnostic tool for diagnosing a disease, or for the preparation of a medicine for treating and/or preventing a disease in a subject and/or for preparing a diagnostic tool for diagnosing a disease, wherein the disease is preferably a neovascularization-related disease, e.g., a solid tumor and ocular disease.

20. A multi-specific fusion protein, comprising:
a polypeptide chain comprising a first VHH domain that specifically binds to a first antigen, a second VHH domain that specifically binds to a second antigen, and a ligand capture domain that specifically binds to a third antigen, respectively, wherein the polypeptide further comprises a dimerization domain selected from a hinge region, a CH3 region, or an Fc region, preferably, wherein the hinge region, CH3 region or Fc region are linked to the C-terminus of the ligand capture domain.

21. The fusion protein according to claim 20, wherein the polypeptide chain comprises:

(i) a first VHH domain, a first linker peptide, a ligand capture domain, a hinge region, a second linker peptide, and a second VHH domain,
(ii) a ligand capture domain, a hinge region, a first linker peptide, a first VHH domain, a second linker peptide, and a second VHH domain, or
(iii) a first VHH domain, a first linker peptide, a second VHH domain, a second linker peptide, a ligand capture domain, and a hinge region,

preferably, wherein the hinge region comprises an amino acid sequence selected from SEQ ID NOs: 12-19 and 45-46, more preferably the amino acid sequence of SEQ ID NO: 12 or 13,
preferably, the first and second linker peptides comprising the amino acid sequence of (GRPGS)n (SEQ ID NO: 59) or (GGGGG)n (SEQ ID NO: 58), wherein n = 2 or 3,
more preferably, the first and second antigens distinct from each other and independently selected from VEGFC and Ang2, respectively, and the third antigen being VEGFA.

22. A hinge region, comprising or consisting of an amino acid sequence selected from SEQ ID NOs: 12-19 and 45-46.

23. The hinge region according to claim 22, comprising or consisting of the amino acid sequence of SEQ ID NO: 12 or SEQ ID NO: 13.

Figure 1A

IAR044-034

IAR044-035

| ignal: | VWD1A,Wavelength=280 nm | | | | |
|---|---|---|---|---|---|
| RT [min] | Type | Width [min] | Area | Height | Area% |
| 2.929 | BV | 0.55 | 95.00 | 7.54 | 4.09 |
| 3.262 | VB | 1.88 | 2225.53 | 181.44 | 95.91 |

| gnal: | VWD1A,Wavelength=280 nm | | | | |
|---|---|---|---|---|---|
| RT [min] | Type | Width [min] | Area | Height | Area% |
| 2.930 | BV | 0.53 | 80.48 | 5.93 | 3.55 |
| 3.237 | VV | 0.63 | 2121.90 | 176.65 | 93.65 |

IAR044-036

| jnal: | VWD1A,Wavelength=280 nm | | | | |
|---|---|---|---|---|---|
| RT [min] | Type | Width [min] | Area | Height | Area% |
| 2.905 | BV | 0.52 | 93.55 | 7.63 | 4.06 |
| 3.226 | VB | 1.92 | 2210.53 | 177.72 | 95.94 |

Figure 1B

A

**HEK293T KDR reporter assay (VEGF A)**

- Eylea
- IAR044-022m
- IAR044-022b
- OPT302
- BLANK
- VEGFA(60ng/mL)

|      | Eylea | IAR044-022m | IAR044-022b | OPT302   |
|------|-------|-------------|-------------|----------|
| IC50 | 43.25 | 149.0       | 59.00       | ~ 0.000  |

B

**HEK293 KDR reporter assay (VEGF A)**

- Eylea
- IAR044-019
- OPT 302
- IgG
- VEGF A (60 ng/ml)
- Blank

C

**HEK293T KDR reporter assay (VEGF A)**

- Eylea
- IAR044-030
- IAR044-032
- IgG
- VEGF A
- Blank

|      | Eylea  | IAR044-030 | IAR044-032 |
|------|--------|------------|------------|
| IC50 | 0.5613 | 0.4821     | 0.5837     |

Figure 2

D

**HEK293T KDR reporter assay (VEGF A)**

| | IAR044-038 | IAR044-039 | IAR044-040 | Eylea | IgG |
|---|---|---|---|---|---|
| IC50 | 0.2673 | 0.3202 | 0.3219 | 0.3055 | 0.007810 |

E

**HEK293T KDR reporter assay (VEGF A)**

| | Eylea | IAR044-028 | IAR044-029 | IAR044-033 | IgG1 | vegfa | Blank |
|---|---|---|---|---|---|---|---|
| IC50 | 0.3501 | 0.5099 | 0.4916 | 0.3867 | ~ 0.000 | | |

Figure 2 (continued)

A

**HEK293T KDR reporter assay (VEGF C)**

Legend: Eylea, IAR044-022m, IAR044-022b, OPT302, VEGFC(100ng/mL), BLANK

|  | Eylea | IAR044-022m | IAR044-022b | OPT302 |
|---|---|---|---|---|
| IC50 | 18006 | 80.26 | 41.29 | 74.48 |

B

**HEK293T KDR reporter assay (VEGF C)**

Legend: Eylea, IAR044-19, OPT302, IgG, VEGF C (100ng/ml), Blank

C

**HEK293T KDR reporter assay (VEGF C)**

Legend: OPT-302, IAR044-030, IAR044-032, IgG, VEGF C, Blank

|  | OPT-302 | IAR044-030 | IAR044-032 |
|---|---|---|---|
| IC50 | 1.682 | 1.428 | 1.468 |

Figure 3

D

### HEK293T KDR reporter assay (VEGF C)

Legend:
- IAR044-038
- IAR044-039
- IAR044-040
- Opt302
- IgG
- VEGF C
- Blank

|  | IAR044-038 | IAR044-039 | IAR044-040 | Opt302 | IgG |
|---|---|---|---|---|---|
| IC50 | 0.7483 | 0.7548 | 0.7679 | 0.5458 | ~ 0.03569 |

E

### HEK293T KDR reporter assay (VEGF C)

Legend:
- Opt302
- IAR044-028
- IAR044-029
- IgG1
- Blank

|  | Opt302 | IAR044-028 | IAR044-029 | IgG1 | Blank |
|---|---|---|---|---|---|
| IC50 | 2.590 | ~ 1.504 | 1.573 | ~ 0.0004901 |  |

F

### HEK293T KDR reporter assay (VEGF C)

Legend:
- Opt302
- IAR044-033
- IgG1
- Blank
- VEGF A

|  | Opt302 | IAR044-033 | IgG1 | Blank | VEGF A |
|---|---|---|---|---|---|
| IC50 | 2.129 | ~ 3.069 | ~ 9.202 |  |  |

Figure 3 (continued)

51

## FLT4-Ba/F3 proliferation assay

Legend:
- IAR044-030
- IAR044-032
- OPT-302
- IEX04-056
- IgG1
- VEGF C(40ng/ml)
- Balnk

|  | IAR044-030 | IAR044-032 | OPT-302 | IEX04-056 |
|---|---|---|---|---|
| IC50 | 0.6069 | 0.6010 | 0.5996 | 0.3691 |

Figure 4

A

## Ang2 Elisa block

Legend:
- Faricimab
- IAR044-022m
- IAR044-022b
- IgG

|  | Faricimab | IAR044-022m | IAR044-022b | IgG |
|---|---|---|---|---|
| IC50 | 61.61 | 9.133 | 4.520 | ~ 0.000 |

B

## Ang2 block

Legend:
- Faricimab
- IAR044-019
- IgG

Figure 5

A

**293 Ang2 block assay**

|  | IgG | Facimab | IAR044-030 | IAR044-032 |
|---|---|---|---|---|
| IC50 | 0.7374 | 61.17 | 2.527 | 1.976 |

B

**293 Ang2 block assay**

|  | IAR044-038 | IAR044-039 | IAR044-040 | Faricimab |
|---|---|---|---|---|
| IC50 | 1.981 | 1.822 | 1.848 | 39.21 |

C

**293 Ang2 block assay**

|  | IgG | Faricimab | IAR044-028 | IAR044-029 | IAR044-033 |
|---|---|---|---|---|---|
| IC50 | 0.7374 | 61.17 | 2.762 | 2.572 | 2.856 |

Figure 6

A

**293 Phospho-Tie-2 assay**

| | IgG | IAR044-19 | Farcimab | IEX04-056 | IEX04-012 |
|---|---|---|---|---|---|
| IC50 | ~ | 1.708 | 57.34 | ~ 7.157 | 2.002 |

B

**293 Phospho-Tie-2 assay**

| | IgG | IAR044-030 | IAR044-032 | Faricimab |
|---|---|---|---|---|
| IC50 | ~ 0.3896 | 2.415 | 1.975 | 97.27 |

Figure 7

A

B

| | IgG1 | Aflibercept | OPT302 | IAR044-19 | Aflibercept+Opt302 | IEX04-056 | IEX04-012 |
|---|---|---|---|---|---|---|---|
| IC50 | ~ 0.03656 | 0.02776 | 1.804 | 0.4412 | 0.4019 | 0.4061 | ~ 0.01307 |

Figure 8

Figure 9

Figure 10

Figure 11

## DL-AAA induced RNV model

Figure 12

## Miles assay

Figure 13

VHH1

Ligand Capture Domain

Hinge Region

VHH2

A

VHH1

Ligand Capture Domain

VHH2

Hinge Region

B

Ligand Capture Domain

Hinge Region
VHH2

VHH1

C

Figure 14

## INTERNATIONAL SEARCH REPORT

| International application No. |
|---|
| **PCT/CN2023/135888** |

| A. | CLASSIFICATION OF SUBJECT MATTER |
|---|---|

C07K19/00(2006.01)i; C07K16/18(2006.01)i; C12N15/62(2006.01)i; C12N15/63(2006.01)i; A61K39/395(2006.01)i; A61P35/00(2006.01)i

According to International Patent Classification (IPC) or to both national classification and IPC

| B. | FIELDS SEARCHED |
|---|---|

Minimum documentation searched (classification system followed by classification symbols)

IPC: C07K, C12N, A61K, A61P

Documentation searched other than minimum documentation to the extent that such documents are included in the fields searched

Electronic data base consulted during the international search (name of data base and, where practicable, search terms used)

CNABS, DWPI, WPABS, CNTXT, ENTXT, ENTXTC, WPABSC, VEN, CNKI, 万方, WANFANG, Pubmed, ISI Web of Science, 百度学术, Baidu Scholar and search terms: 融合蛋白, 三特异性, 多特异性, 血管内皮生长因子, VEGF A, VEGF C, 人血管生成素, Ang2, angiopoietin-2, ANGPT2, 单域抗体, 血管内皮生长因子受体, fusion protein, trispecific, multispecific, VHH, sdAb, VEGFR, etc.; GenBank, EMBL, 中国专利生物序列检索系统, STN和检索的序列: SEQ ID NOs: 1-70, GenBank, EMBL, China Patent Biological Sequence Search System, STN and searched sequences: SEQ ID NOs: 1-70.

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 114206907 A (REGENERON PHARMACEUTICALS, INC.) 18 March 2022 (2022-03-18) see SEQ ID NO: 40, SEQ ID NO: 41, and SEQ ID NO: 60 | 22, 23 |
| Y | CN 114206907 A (REGENERON PHARMACEUTICALS, INC.) 18 March 2022 (2022-03-18) see SEQ ID NO: 40, SEQ ID NO: 41, and SEQ ID NO: 60 | 7 |
| X | US 2020283495 A1 (ST PHI THERAPEUTICS; YIZUN BIOTECH SHANGHAI CO., LTD.; YIZUN BIOPHARM SHANGHAI CO., LTD.;) 10 September 2020 (2020-09-10) see SEQ ID NO: 3 | 22-23 |
| Y | US 2020283495 A1 (ST PHI THERAPEUTICS; YIZUN BIOTECH SHANGHAI CO., LTD.; YIZUN BIOPHARM SHANGHAI CO., LTD.;) 10 September 2020 (2020-09-10) see SEQ ID NO: 3 | 7 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **31 January 2024** | **11 February 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**EP 4 628 512 A1**

## INTERNATIONAL SEARCH REPORT

<table>
<tr><td colspan="2">International application No.</td></tr>
<tr><td colspan="2"><b>PCT/CN2023/135888</b></td></tr>
</table>

**C.    DOCUMENTS CONSIDERED TO BE RELEVANT**

| Category* | Citation of document, with indication, where appropriate, of the relevant passages | Relevant to claim No. |
|---|---|---|
| Y | WO 2022199603 A1 (NANJING GENSCRIPT BIOTECH CO., LTD.) 29 September 2022 (2022-09-29)<br>    see embodiment 1, and claims 1-61 | 1-2, 5-7, 10-15, 19-21 |
| Y | CN 105820243 A (BOEHRINGER INGELHEIM INTERNATIONAL GMBH) 03 August 2016 (2016-08-03)<br>    see embodiments 1-12, and claims 1-40 | 1-2, 5-7, 10-15, 19-21 |
| A | CN 109069638 A (GENSUN BIOPHARMA INC.) 21 December 2018 (2018-12-21)<br>    see claims 1-20, and embodiments 1-9 | 1-15, 19-23 |
| A | WO 2022143801 A1 (WUXI BIOLOGICS SHANGHAI CO., LTD.; WUXI BIOLOGICS IRELAND LTD.;) 07 July 2022 (2022-07-07)<br>    see claims 1-117, and embodiments 1-10 | 1-15, 19-23 |

Form PCT/ISA/210 (second sheet) (July 2022)

## INTERNATIONAL SEARCH REPORT

| International application No. |
| --- |
| **PCT/CN2023/135888** |

**Box No. I      Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

    a.   ☑   forming part of the international application as filed.

    b.   ☐   furnished subsequent to the international filing date for the purposes of international search (Rule 13*ter*.1(a)),

         ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2023/135888** |

**Box No. II        Observations where certain claims were found unsearchable (Continuation of item 2 of first sheet)**

This international search report has not been established in respect of certain claims under Article 17(2)(a) for the following reasons:

1. ☑  Claims Nos.: **16-18 and "the use of the fusion protein of claims 1-10 as a drug, and the use of the fusion protein as a drug for treating diseases" of claim 19.**
   because they relate to subject matter not required to be searched by this Authority, namely:

   PCT Rule 39.1(iv) – methods for treatment of the human or animal body by surgery or therapy.

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2022)

International application No.

**PCT/CN2023/135888**

| Patent document cited in search report | | | Publication date (day/month/year) | Patent family member(s) | | | Publication date (day/month/year) |
|---|---|---|---|---|---|---|---|
| CN | 114206907 | A | 18 March 2022 | SG | 11202110958 | QA | 28 October 2021 |
| | | | | KR | 20220038348 | A | 28 March 2022 |
| | | | | KR | 102519234 | B1 | 10 April 2023 |
| | | | | ZA | 202107647 | B | 30 March 2022 |
| | | | | AU | 2020396490 | A1 | 28 October 2021 |
| | | | | AU | 2020396490 | B2 | 15 December 2022 |
| | | | | AU | 2020396490 | C1 | 27 April 2023 |
| | | | | IL | 288352 | A | 01 January 2022 |
| | | | | IL | 288352 | B | 01 November 2022 |
| | | | | IL | 288352 | B2 | 01 March 2023 |
| | | | | SG | 11202110968 | VA | 28 October 2021 |
| | | | | TW | 202128744 | A | 01 August 2021 |
| | | | | PE | 20221261 | A1 | 16 August 2022 |
| | | | | US | 2023068199 | A1 | 02 March 2023 |
| | | | | US | 11753459 | B2 | 12 September 2023 |
| | | | | JP | 2022547652 | A | 15 November 2022 |
| | | | | IL | 288360 | A | 01 January 2022 |
| | | | | IL | 288360 | B1 | 01 May 2023 |
| | | | | IL | 288360 | B2 | 01 September 2023 |
| | | | | PE | 20221791 | A1 | 25 November 2022 |
| | | | | IL | 302538 | A | 01 July 2023 |
| | | | | MY | 190623 | A | 27 April 2022 |
| | | | | EP | 3931204 | A1 | 05 January 2022 |
| | | | | BR | 112021025438 | A2 | 21 June 2022 |
| | | | | NZ | 781136 | A | 26 May 2023 |
| | | | | SG | 11202110953 | UA | 28 October 2021 |
| | | | | SG | 11202110955 | VA | 28 October 2021 |
| | | | | JP | 2022548818 | A | 22 November 2022 |
| | | | | US | 2022227835 | A1 | 21 July 2022 |
| | | | | US | 11485770 | B2 | 01 November 2022 |
| | | | | JP | 2023179531 | A | 19 December 2023 |
| | | | | US | 2023331812 | A1 | 19 October 2023 |
| | | | | MY | 193355 | A | 06 October 2022 |
| | | | | KR | 20230152810 | A | 03 November 2023 |
| | | | | IL | 288355 | A | 01 January 2022 |
| | | | | IL | 288355 | B | 01 November 2022 |
| | | | | IL | 288355 | B2 | 01 March 2023 |
| | | | | WO | 2021112928 | A1 | 10 June 2021 |
| | | | | MX | 2021014862 | A | 27 September 2022 |
| | | | | CO | 2021018181 | A2 | 17 January 2022 |
| | | | | JP | 2022547651 | A | 15 November 2022 |
| | | | | US | 2022009998 | A1 | 13 January 2022 |
| | | | | US | 11306135 | B2 | 19 April 2022 |
| | | | | US | 2022162289 | A1 | 26 May 2022 |
| | | | | US | 11459374 | B2 | 04 October 2022 |
| | | | | US | 2022009999 | A1 | 13 January 2022 |
| | | | | US | 11299532 | B2 | 12 April 2022 |
| | | | | MY | 193351 | A | 06 October 2022 |
| | | | | IL | 296512 | A | 01 November 2022 |
| | | | | IL | 296512 | B1 | 01 August 2023 |

Form PCT/ISA/210 (patent family annex) (July 2022)

## INTERNATIONAL SEARCH REPORT
### Information on patent family members

| International application No. |
|---|
| **PCT/CN2023/135888** |

| Patent document cited in search report | Publication date (day/month/year) | Patent family member(s) | | | Publication date (day/month/year) |
|---|---|---|---|---|---|
| | | IL | 296512 | B2 | 01 December 2023 |
| | | AU | 2020397803 | A1 | 28 October 2021 |
| | | AU | 2020397803 | B2 | 15 December 2022 |
| | | AU | 2020397803 | C1 | 11 May 2023 |
| | | MX | 2022006759 | A | 30 January 2023 |
| | | US | 2023041349 | A1 | 09 February 2023 |
| | | WO | 2021112924 | A1 | 10 June 2021 |
| | | EP | 3906302 | A1 | 10 November 2021 |
| | | AU | 2020398830 | A1 | 28 October 2021 |
| | | AU | 2020398830 | B2 | 15 December 2022 |
| | | AU | 2020398830 | C1 | 27 April 2023 |
| | | AU | 2023201590 | A1 | 13 April 2023 |
| | | US | 2021214430 | A1 | 15 July 2021 |
| | | US | 11098112 | B2 | 24 August 2021 |
| | | IL | 296864 | A | 01 November 2022 |
| | | IL | 296864 | B1 | 01 June 2023 |
| | | IL | 296864 | B2 | 01 October 2023 |
| | | AU | 2023201588 | A1 | 13 April 2023 |
| | | KR | 20230051296 | A | 17 April 2023 |
| | | NZ | 781138 | A | 26 May 2023 |
| | | US | 2021214733 | A1 | 15 July 2021 |
| | | US | 11098311 | B2 | 24 August 2021 |
| | | WO | 2021112927 | A1 | 10 June 2021 |
| | | IL | 296893 | A | 01 December 2022 |
| | | IL | 296893 | B1 | 01 January 2024 |
| | | AU | 2023201586 | A1 | 13 April 2023 |
| | | MX | 2022011973 | A | 09 November 2022 |
| | | TW | 202128745 | A | 01 August 2021 |
| | | WO | 2021112929 | A1 | 10 June 2021 |
| | | IL | 304390 | A | 01 September 2023 |
| | | BR | 112021025158 | A2 | 21 June 2022 |
| | | EP | 3931303 | A1 | 05 January 2022 |
| | | TW | 202122417 | A | 16 June 2021 |
| | | IL | 301888 | A | 01 June 2023 |
| | | KR | 20220035393 | A | 22 March 2022 |
| | | ZA | 202107644 | B | 30 March 2022 |
| | | MX | 2022006788 | A | 11 July 2022 |
| | | NZ | 781143 | A | 26 May 2023 |
| | | MY | 190686 | A | 10 May 2022 |
| | | CO | 2021018214 | A2 | 17 January 2022 |
| | | BR | 112021025769 | A2 | 12 April 2022 |
| | | PE | 20221789 | A1 | 25 November 2022 |
| | | KR | 20220038062 | A | 25 March 2022 |
| | | ZA | 202107646 | B | 30 March 2022 |
| | | US | 2022127331 | A1 | 28 April 2022 |
| | | US | 11472861 | B2 | 18 October 2022 |
| | | AU | 2023201748 | A1 | 01 June 2023 |
| | | TW | 202128743 | A | 01 August 2021 |
| | | MX | 2022012042 | A | 27 October 2022 |
| | | AU | 2020398547 | A1 | 28 October 2021 |

Form PCT/ISA/210 (patent family annex) (July 2022)

## INTERNATIONAL SEARCH REPORT
### Information on patent family members

| International application No. |
| --- |
| **PCT/CN2023/135888** |

| Patent document cited in search report | Publication date (day/month/year) | Patent family member(s) | | | Publication date (day/month/year) |
| --- | --- | --- | --- | --- | --- |
| | | AU | 2020398547 | B2 | 22 December 2022 |
| | | AU | 2020398547 | C1 | 11 May 2023 |
| | | KR | 20220038347 | A | 28 March 2022 |
| | | PE | 20221790 | A1 | 25 November 2022 |
| | | AU | 2020398125 | A1 | 28 October 2021 |
| | | AU | 2020398125 | B2 | 15 December 2022 |
| | | AU | 2020398125 | C1 | 11 May 2023 |
| | | MY | 190625 | A | 27 April 2022 |
| | | SG | 11202110964 | QA | 28 October 2021 |
| | | SG | 11202110972 | UA | 28 October 2021 |
| | | US | 2023357357 | A1 | 09 November 2023 |
| | | JP | 2022550930 | A | 06 December 2022 |
| | | JP | 2022552052 | A | 15 December 2022 |
| | | US | 2021206832 | A1 | 08 July 2021 |
| | | US | 11104715 | B2 | 31 August 2021 |
| | | IL | 288351 | A | 01 January 2022 |
| | | IL | 288351 | B1 | 01 January 2024 |
| | | US | 2022396608 | A1 | 15 December 2022 |
| | | US | 11542317 | B1 | 03 January 2023 |
| | | US | 2021171570 | A1 | 10 June 2021 |
| | | US | 11053280 | B2 | 06 July 2021 |
| | | NZ | 781145 | A | 26 May 2023 |
| | | MX | 2021014863 | A | 28 September 2022 |
| | | US | 2023119662 | A1 | 20 April 2023 |
| | | US | 11732025 | B2 | 22 August 2023 |
| | | US | 2022098280 | A1 | 31 March 2022 |
| | | US | 2022009997 | A1 | 13 January 2022 |
| | | US | 11440950 | B2 | 13 September 2022 |
| | | NZ | 781137 | A | 26 May 2023 |
| | | MY | 193354 | A | 06 October 2022 |
| | | MX | 2022006758 | A | 30 January 2023 |
| | | MY | 190626 | A | 27 April 2022 |
| | | BR | 112022001016 | A2 | 16 August 2022 |
| | | IL | 288367 | A | 01 January 2022 |
| | | IL | 288367 | B | 01 October 2022 |
| | | IL | 288367 | B2 | 01 February 2023 |
| | | CO | 2021018203 | A2 | 17 January 2022 |
| | | CA | 3129193 | A1 | 10 June 2021 |
| | | CA | 3129193 | C | 04 October 2022 |
| | | BR | 112021025359 | A2 | 21 June 2022 |
| | | US | 2022235116 | A1 | 28 July 2022 |
| | | US | 11505594 | B2 | 22 November 2022 |
| | | US | 2023235019 | A1 | 27 July 2023 |
| | | WO | 2021112926 | A1 | 10 June 2021 |
| | | AU | 2020397865 | A1 | 28 October 2021 |
| | | AU | 2020397865 | B2 | 15 December 2022 |
| | | AU | 2020397865 | C1 | 27 April 2023 |
| | | MY | 190627 | A | 27 April 2022 |
| | | MX | 2022006760 | A | 30 January 2023 |
| | | US | 2022033471 | A1 | 03 February 2022 |

Form PCT/ISA/210 (patent family annex) (July 2022)

**INTERNATIONAL SEARCH REPORT**
Information on patent family members

| International application No. |
| --- |
| **PCT/CN2023/135888** |

| Patent document cited in search report | Publication date (day/month/year) | Patent family member(s) | | | Publication date (day/month/year) |
| --- | --- | --- | --- | --- | --- |
| | | US | 11535663 | B2 | 27 December 2022 |
| | | US | 2021206833 | A1 | 08 July 2021 |
| | | US | 11180540 | B2 | 23 November 2021 |
| | | US | 2023272044 | A1 | 31 August 2023 |
| | | CA | 3172625 | A1 | 10 June 2021 |
| | | EP | 3906249 | A1 | 10 November 2021 |
| | | AU | 2023201589 | A1 | 06 April 2023 |
| | | CO | 2022000660 | A2 | 28 January 2022 |
| | | MY | 193349 | A | 06 October 2022 |
| | | NZ | 781142 | A | 26 May 2023 |
| | | US | 2022033472 | A1 | 03 February 2022 |
| | | US | 11407813 | B2 | 09 August 2022 |
| | | US | 2021171605 | A1 | 10 June 2021 |
| | | US | 11186625 | B2 | 30 November 2021 |
| | | US | 2022048975 | A1 | 17 February 2022 |
| | | US | 11459373 | B2 | 04 October 2022 |
| | | US | 2022389081 | A1 | 08 December 2022 |
| | | US | 11548932 | B2 | 10 January 2023 |
| | | CA | 3159586 | A1 | 10 June 2021 |
| | | MY | 193350 | A | 06 October 2022 |
| | | MY | 190624 | A | 27 April 2022 |
| | | EP | 3906250 | A1 | 10 November 2021 |
| | | WO | 2021112925 | A1 | 10 June 2021 |
| | | AU | 2023201585 | A1 | 13 April 2023 |
| | | TW | 202128991 | A | 01 August 2021 |
| | | IL | 288357 | A | 01 January 2022 |
| | | IL | 288357 | B | 01 November 2022 |
| | | IL | 288357 | B2 | 01 March 2023 |
| | | EP | 3906303 | A1 | 10 November 2021 |
| | | KR | 20220038349 | A | 28 March 2022 |
| | | KR | 20230051297 | A | 17 April 2023 |
| | | PE | 20221770 | A1 | 11 November 2022 |
| | | WO | 2021112923 | A1 | 10 June 2021 |
| | | US | 2022275054 | A1 | 01 September 2022 |
| | | US | 11649273 | B2 | 16 May 2023 |
| | | US | 2021371501 | A1 | 02 December 2021 |
| | | US | 11286290 | B2 | 29 March 2022 |
| | | IL | 296863 | A | 01 November 2022 |
| | | IL | 296863 | B1 | 01 June 2023 |
| | | IL | 296863 | B2 | 01 October 2023 |
| | | TW | 202134256 | A | 16 September 2021 |
| | | PE | 20221788 | A1 | 25 November 2022 |
| | | CA | 3172631 | A1 | 10 June 2021 |
| | | CA | 3172631 | C | 02 January 2024 |
| | | CO | 2021018234 | A2 | 17 January 2022 |
| | | MY | 193353 | A | 06 October 2022 |
| | | IL | 302921 | A | 01 July 2023 |
| | | BR | 112021025432 | A2 | 21 June 2022 |
| | | CO | 2021018192 | A2 | 17 January 2022 |
| | | KR | 20220041083 | A | 31 March 2022 |

Form PCT/ISA/210 (patent family annex) (July 2022)

**INTERNATIONAL SEARCH REPORT**
Information on patent family members

| International application No. |
| --- |
| **PCT/CN2023/135888** |

| Patent document cited in search report | | | Publication date (day/month/year) | Patent family member(s) | | | Publication date (day/month/year) |
| --- | --- | --- | --- | --- | --- | --- | --- |
| | | | | KR | 102519236 | B1 | 10 April 2023 |
| | | | | JP | 2022548197 | A | 17 November 2022 |
| | | | | JP | 2023171771 | A | 05 December 2023 |
| | | | | US | 2021284684 | A1 | 16 September 2021 |
| | | | | US | 11174283 | B2 | 16 November 2021 |
| | | | | US | 2022227836 | A1 | 21 July 2022 |
| | | | | US | 11505593 | B2 | 22 November 2022 |
| US | 2020283495 | A1 | 10 September 2020 | None | | | |
| WO | 2022199603 | A1 | 29 September 2022 | None | | | |
| CN | 105820243 | A | 03 August 2016 | CY | 1118339 | T1 | 28 June 2017 |
| | | | | MX | 343440 | B | 07 November 2016 |
| | | | | SG | 193561 | A1 | 29 November 2013 |
| | | | | AU | 2017200237 | A1 | 02 February 2017 |
| | | | | AU | 2017200237 | B2 | 17 May 2018 |
| | | | | HK | 1190412 | A1 | 04 July 2014 |
| | | | | ECSP | 13013001 | A | 31 December 2013 |
| | | | | HUE | 030148 | T2 | 28 April 2017 |
| | | | | PT | 2694546 | T | 28 December 2016 |
| | | | | AP | 201307085 | D0 | 31 August 2013 |
| | | | | CA | 2827817 | A1 | 04 October 2012 |
| | | | | CA | 2827817 | C | 15 December 2020 |
| | | | | PE | 20140448 | A1 | 13 April 2014 |
| | | | | RS | 55361 | B1 | 31 March 2017 |
| | | | | EA | 201301108 | A1 | 31 March 2014 |
| | | | | EA | 025148 | B1 | 30 November 2016 |
| | | | | JP | 2014515602 | A | 03 July 2014 |
| | | | | JP | 6023786 | B2 | 09 November 2016 |
| | | | | HK | 1225400 | A1 | 08 September 2017 |
| | | | | ES | 2606302 | T3 | 23 March 2017 |
| | | | | SI | 2694546 | T1 | 31 January 2017 |
| | | | | WO | 2012131078 | A1 | 04 October 2012 |
| | | | | JP | 2017023152 | A | 02 February 2017 |
| | | | | JP | 6297657 | B2 | 20 March 2018 |
| | | | | AP | 201307085 | A0 | 31 August 2013 |
| | | | | MY | 171007 | A | 23 September 2019 |
| | | | | BR | 112013025304 | A2 | 06 June 2017 |
| | | | | BR | 112013025304 | B1 | 11 October 2022 |
| | | | | EP | 2694546 | A1 | 12 February 2014 |
| | | | | EP | 2694546 | B1 | 21 September 2016 |
| | | | | MA | 34979 | B1 | 01 March 2014 |
| | | | | TN | 2013000390 | A1 | 20 January 2015 |
| | | | | AU | 2012237234 | A1 | 05 September 2013 |
| | | | | AU | 2012237234 | B2 | 20 October 2016 |
| | | | | EP | 3144322 | A2 | 22 March 2017 |
| | | | | EP | 3144322 | A3 | 31 May 2017 |
| | | | | AR | 085984 | A1 | 13 November 2013 |
| | | | | HRP | 20161689 | T1 | 24 February 2017 |
| | | | | US | 2022017642 | A1 | 20 January 2022 |
| | | | | EA | 201600338 | A1 | 30 September 2016 |
| | | | | EA | 036746 | B1 | 16 December 2020 |

Form PCT/ISA/210 (patent family annex) (July 2022)

## INTERNATIONAL SEARCH REPORT
### Information on patent family members

International application No.

**PCT/CN2023/135888**

| Patent document cited in search report | | | Publication date (day/month/year) | Patent family member(s) | | | Publication date (day/month/year) |
|---|---|---|---|---|---|---|---|
| | | | | CL | 2013002623 | A1 | 14 February 2014 |
| | | | | NZ | 614249 | A | 29 May 2015 |
| | | | | US | 2020010569 | A1 | 09 January 2020 |
| | | | | US | 11161916 | B2 | 02 November 2021 |
| | | | | KR | 20140018317 | A | 12 February 2014 |
| | | | | KR | 101907572 | B1 | 15 October 2018 |
| | | | | DK | 2694546 | T3 | 05 December 2016 |
| | | | | PL | 2694546 | T3 | 31 March 2017 |
| | | | | MX | 2013010949 | A | 06 June 2014 |
| | | | | MX | 337543 | B | 10 March 2016 |
| | | | | SG | 10201602373 | TA | 30 May 2016 |
| | | | | IL | 227936 | A0 | 30 September 2013 |
| | | | | IL | 227936 | B | 28 June 2018 |
| | | | | UY | 33998 | A | 28 September 2012 |
| | | | | UA | 114707 | C2 | 25 July 2017 |
| | | | | US | 2013078248 | A1 | 28 March 2013 |
| | | | | US | 9527925 | B2 | 27 December 2016 |
| | | | | CO | 6801639 | A2 | 29 November 2013 |
| | | | | LT | 2694546 | T | 10 November 2016 |
| | | | | US | 2017247475 | A1 | 31 August 2017 |
| | | | | US | 10414828 | B2 | 17 September 2019 |
| CN | 109069638 | A | 21 December 2018 | US | 2017275353 | A1 | 28 September 2017 |
| | | | | US | 10894823 | B2 | 19 January 2021 |
| | | | | US | 2021115123 | A1 | 22 April 2021 |
| | | | | EP | 3432927 | A1 | 30 January 2019 |
| | | | | EP | 3432927 | A4 | 20 November 2019 |
| | | | | WO | 2017165681 | A1 | 28 September 2017 |
| WO | 2022143801 | A1 | 07 July 2022 | KR | 20230126718 | A | 30 August 2023 |
| | | | | EP | 4271715 | A1 | 08 November 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7531173 B

**Non-patent literature cited in the description**

- **GESTUR VIDARSSON et al.** *IgG subclasses and allotypes: from structure to effector functions*, 20 October 2014
- **KABAT et al.** Sequences of Proteins of Immunological Interest. National Institutes of Health, 1987
- **NORTH et al.** A New Clustering of Antibody CDR Loop Conformations. *Journal of Molecular Biology*, 2011, vol. 406, 228-256
- **KABAT et al.** Sequences of Proteins of Immunological Interest. 1991
- **YU, D. et al.** *Mol. Ther.*, 2012, vol. 20 (3), 938-947
- **HOLASH, J et al.** *PNAS*, 2002, vol. 99 (17)
- **YAZAKI** ; **WU**. *Methods in Molecular Biology*, 2003, vol. 248, 255-268